# EUROPEAN PATENT SPECIFICATION

(11) **EP 2 035 417 B1**
(45) Date of publication and mention of the grant of the patent: **02.03.2011**
(21) Application number: 07733082.7
(22) Date of filing: 06.06.2007
(51) Int. Cl.: C07D 413/04, C07D 413/14, A61K 31/4245, A61P 3/10

(54) **BENZIMIDAZOLES AND THEIR USE FOR THE TREATEMNT OF DIABETES**
BENZIMIDAZOLE UND IHRE VERWENDUNG ZUR BEHANDLUNG VON DIABETES
BENZIMIDAZOLES ET LEUR APPLICATION AU TRAITEMENT DU DIABÈTE

(30) Priority: 08.06.2006 US 811895 P
(43) Date of publication of application: 18.03.2009
(73) Proprietor: AstraZeneca AB, 151 85 Södertälje (SE)
(72) Inventor: BIRCH, Alan Martin, Macclesfield Cheshire SK10 4TG (GB); BUTLIN, Roger John, Macclesfield Cheshire SK10 4TG (GB); PLOWRIGHT, Alleyn, 431 83 Mölndal (SE)
(74) Representative: Berry, Ian Gordon
(86) International application number: PCT/GB2007/002070
(87) International publication number: WO 2007/141517

(56) References cited:
- WO-A-2004/047755
- WO-A-2004/100881
- WO-A-2006/064189
- WO-A-2006/134317
- US-A1- 2003 072 757
- US-A1- 2005 070 545
- S. N. SAWHNEY ET. AL.: "Synthesis of some 2-(5-substituted 1,3,4-oxadiazol-2-yl)-, 2-(5-substituted 1,3,4-thiadiazol-2-yl)- and 2-(3-mercapto-4-substituted- 4H-1,2,4-triazol-5-yl)- benzimidazoles as potential antiinflammatory agents." INDIAN JOURNAL OF CHEMISTRY SECTION B, vol. 30B, 1991, pages 407-412, XP009089706

## Description

The present invention relates to compounds which inhibit acetyl CoA(acetyl coenzyme A):diacylglycerol acyltransferase (DGAT1) activity, processes for their preparation, pharmaceutical compositions containing them as the active ingredient, methods for the treatment of disease states associated with DGAT1 activity, to their use as medicaments and to their use in the manufacture of medicaments for use in the inhibition of DGAT1 in warm-blooded animals such as humans. In particular this invention relates to compounds useful for the treatment of type II diabetes, insulin resistance, impaired glucose tolerance and obesity in warm-blooded animals such as humans, more particularly to the use of these compounds in the manufacture of medicaments for use in the treatment of type II diabetes, insulin resistance, impaired glucose tolerance and obesity in warm-blooded animals such as humans.

Acyl CoA:diacylglycerol acyltransferase (DGAT) is found in the microsomal fraction of cells. It catalyzes the final reaction in the glycerol phosphate pathway, considered to be the main pathway of triglyceride synthesis in cells by facilitating the joining of a diacylglycerol with a fatty acyl CoA, resulting in the formation of triglyceride. Although it is unclear whether DGAT is rate-limiting for triglyceride synthesis, it catalyzes the only step in the pathway that is committed to producing this type of molecule [Lehner & Kuksis (1996) Biosynthesis oftriacylglycerols. Prog. Lipid Res. 35: 169-201].

Two DGAT genes have been cloned and characterised. Both of the encoded proteins catalyse the same reaction although they share no sequence homology. The DGAT1 gene was identified from sequence database searches because of its similarity to acyl CoA:cholesterol acyltransferase (ACAT) genes. [Cases et al (1998) Identification of a gene encoding an acyl CoA:diacylglycerol acyltransferase, a key enzyme in triacylglycerol synthesis. Proc. Natl. Acad. Sci. USA 95: 13018-13023]. DGAT1 activity has been found in many mammalian tissues, including adipocytes.

Because of the previous lack of molecular probes, little is known about the regulation of DGAT1. DGAT1 is known to be significantly up-regulated during adipocyte differentiation.

Studies in gene knockout mice has indicated that modulators of the activity of DGAT1 would be of value in the treatment of type II diabetes and obesity. DGAT1 knockout (*Dgat1*^{*-*/*-*}) mice, are viable and capable of synthesizing triglycerides, as evidenced by normal fasting serum triglyceride levels and normal adipose tissue composition. *Dgat1^{-l-}* mice have less adipose tissue than wild-type mice at baseline and are resistant to diet-induced obesity. Metabolic rate is ∼20% higher in *Dgat1*^{*-*/}*⁻* mice than in wild-type mice on both regular and high-fat diets [Smith et al (2000) Obesity resistance and multiple mechanisms of triglyceride synthesis in mice lacking DGAT. Nature Genetics 25: 87-90]. Increased physical activity in *Dgat1*^{*-*/}*⁻* mice partially accounts for their increased energy expenditure. The *Dgat1*^{*-*/}*⁻* mice also exhibit increased insulin sensitivity and a 20% increase in glucose disposal rate. Leptin levels are 50% decreased in the *Dgat1^{-l-}* mice in line with the 50% decrease in fat mass.

When *Dgat1^{-l-}* mice are crossed with *ob*/*ob* mice, these mice exhibit the *ob*/*ob* phenotype [Chen et al (2002) Increased insulin and leptin sensitivity in mice lacking acyl CoA:diacylglycerol acyltransferase J. Clin. Invest. 109:1049-1055] indicating that the *Dgat1*^{*-*/*-*} phenotype requires an intact leptin pathway. When *Dgat1^{-l-}* mice are crossed with *Agouti* mice a decrease in body weight is seen with normal glucose levels and 70% reduced insulin levels compared to wild type, *agouti* or *ob*/*ob*/ *Dgat1^{-l-}* mice.

Transplantation of adipose tissue from *Dgat1^{-l-}* mice to wild type mice confers resistance to diet-induced obesity and improved glucose metabolism in these mice [Chen et al (2003) Obesity resistance and enhanced glucose metabolism in mice transplanted with white adipose tissue lacking acyl CoA:diacylglycerol acyltransferase J. Clin. Invest. 111: 1715-1722].

International Patent Applications W02004/047755 (Tularik and Japan Tobacco) and W02005/013907 (Japan Tobacco and Amgen) describe fused bicyclic nitrogen-containing heterocycles which are inhibitors of DGAT-1. JP2004-67635 (Otsuka Pharmaceuticals) describes thiazoleamido substituted phenyl compounds which are further substituted with alkylphosphonates and which inhibit DGAT-1. WO2004/100881 (Bayer) describes biphenylamino compounds substituted with imidazole, oxazole or thiazole which inhibit DGAT-1. Our co-pending International Application PCT/GB2005/004726 describes oxadiazole compounds which inhibit DGAT-1. Sawhney and Gupta (Indian Journal of Chemistry 1991, 30B, 407-412, describes the synthesis of some substituted 4H-1,2,4-triazolo-5-yl-benzimidazoles.

Accordingly, the present invention provides a compound of formula (I) 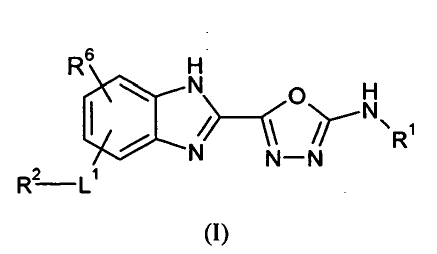 or a salt thereof, wherein:
R¹ is selected from phenyl, cyclopentyl, cyclohexyl, and HET-1, wherein R¹ is optionally substituted with either:
   i) a substituent selected from group a) and optionally a substituent selected from either group b) or group c); or
   ii) 1 or 2 substituents independently selected from group b) and optionally a substituent selected from group c); or
   iii) up to 4 substituents independently selected from group c);
wherein groups a) to c) are as follows:
group a) nitro, -C(O)ₙR²⁰, a carboxylic acid mimic or bioisostere thereof, -NR²¹R²², -C(O)NR²¹R²², -OC(O)NR²¹R²², -NR²¹C(O)ₙR²⁰, -NR²⁰CONR²¹R²², -S(O)₂NR²¹R²² or -NR²¹S(O)₂R²² where R²⁰, R²¹ and R²² are independently selected from hydrogen and (1-6C)alkyl, or R²¹ and R²² together with the nitrogen atom to which they are attached form an optionally substituted ring having 3 to 10 atoms, which optionally contains further heteroatoms such as S(O)ₘ, oxygen and nitrogen;
group b) R¹ is optionally substituted by 1 or 2 substituents independently selected from halo(1-6C)alkyl, cyano, (1-6C)alkyl, hydroxy, (1-6C)alkoxy, benzyloxy, -SOₘ(1-6C)alkyl and -OSO₂(1-6C)alkyl;
group c) halo;
and when R¹ is substituted by two (1-6C)alkoxy groups, these may be joined together to form a 5- or 6-membered ring fused to R¹;
n is (independently at each occurrence) 1 or 2;
m is (independently at each occurrence) 0, 1 or 2;
L¹ is a direct bond or is a linker selected from -0-, -OCH₂-, -CH₂O-, -S(O)ₘ-, -S(O)ₘCH₂-, -CH₂S(O)m- and -(CR⁷R⁸)₁₋₂-;
R² is selected from (3-6C)cycloalkyl, (5-12C)bicycloalkyl, phenyl, HET-2 and (2-6C)alkyl; wherein R² is substituted by -L²-R³;
L² is a direct bond or is a linker selected from -(CR⁴R⁵)₁₋₂-, -O-(CR⁴R⁵)₁₋₂- and - CH₂(CR⁴R⁵)₁₋₂- (wherein for each value of L², the CR⁴R⁵ group is directly attached to R³);
each R⁴ is independently selected from hydrogen, hydroxy, (1-3C)alkoxy, (1-4C)alkyl, hydroxy(1-3C)alkyl and (1-2C)alkoxy(1-2C)alkyl; provided that when L² is -O-(CR⁴R⁵)₁₋₂- then the R⁴ on the carbon atom directly attached to the oxygen atom is not hydroxy or (1-3C)alkoxy;
each R⁵ is independently selected from hydrogen and methyl;
R³ is selected from hydroxy, carboxy, (1-6C)alkoxycarbonyl, and a carboxylic acid mimic or bioisostere;
R⁶ is selected from hydrogen, fluoro, chloro, hydroxy, methoxy, halo(1-2C)alkyl, methyl, ethyl, cyano and methylsulfonyl;
each R⁷ is independently selected from hydrogen, (1-4C)alkyl and (1-4C)alkoxy;
each R⁸ is independently selected from hydrogen and methyl;
HET-1 is a 5- or 6-membered heteroaryl ring containing 1 or 2 ring heteroatoms independently selected from O, N and S (provided there are no O-O, S-S or O-S bonds within the ring);
HET-2 is a 4, 5- or 6-membered saturated, partially or fully unsaturated heterocyclyl ring containing 1, 2 or 3 ring heteroatoms independently selected from O, N and S (provided there are no O-O, S-S or O-S bonds within the ring), wherein a ring carbon atom may be oxidised to C(O) and/or a ring sulfur atom may be oxidised to S(O) or S(O)₂.

In another embodiment is provided a compound of formula (I), or a salt thereof, as described immediately above wherein R² is optionally substituted by -L²-R³ as described immediately above and herein.

It should be understood that when L¹ is not a direct bond, the right hand side of the linking group as written is attached to R².

It will be understood that the definition of R¹ substituted by two (1-6C)alkoxy groups, joined together to form a 5- or 6-membered ring fused to R¹ is intended to define structures such as those shown below (wherein, in these examples, R¹ is phenyl):

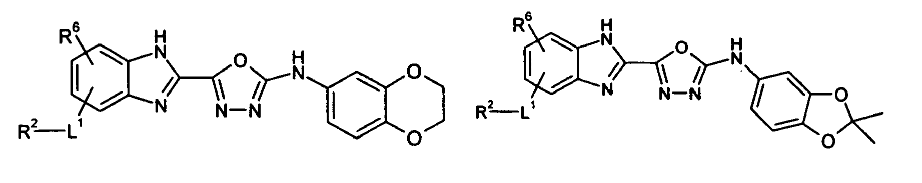

In this specification the term "alkyl" includes both straight and branched chain alkyl groups but references to individual alkyl groups such as "propyl" are specific for the straight chain version only. An analogous convention applies to other generic terms. Unless otherwise stated the term "alkyl" advantageously refers to chains with 1-10 carbon atoms, suitably from 1- 6 carbon atoms, preferably 1-4 carbon atoms.

In this specification the term "alkoxy" means an alkyl group as defined hereinbefore linked to an oxygen atom.

It is to be understood that optional substituents on any group may be attached to any available atom as appropriate unless otherwise specified, including heteroatoms provided that they are not thereby quaternised.

In this specification the term "heteroatom" refers to non-carbon atoms such as oxygen, nitrogen or sulphur atoms.

Unless specified otherwise, the expression "haloalkyl" refers to alkyl groups which carry at least one halo substitutent. This includes perhalo groups where all hydrogen atoms are replaced by halo such as fluoro.

It is to be understood that optional substituents on any group may be attached to any available atom as appropriate unless otherwise specified, including heteroatoms provided that they are not thereby quatemised.

Within this specification composite terms are used to describe groups comprising more than one functionality such as (1-6C)alkoxy(1-6C)alkyl. Such terms are to be interpreted in accordance with the meaning which is understood by a person skilled in the art for each component part.

Where optional substituents are chosen from "0, 1, 2 or 3" groups it is to be understood that this definition includes all substituents being chosen from one of the specified groups or the substituents being chosen from two or more of the specified groups. An analogous convention applies to substituents chosen from "0, 1 or 2" groups and "1 or 2" and any other analogous groups.

Substituents may be present at any suitable position on, for example, an alkyl group. Therefore, hydroxy substituted (1-6C)alkyl includes hydroxymethyl, 1-hydroxyethyl, 2-hydroxyethyl and 3-hydroxypropyl.

Examples of **(1-4C)alkyl** include methyl, ethyl, propyl and isopropyl; examples of **(1-6C)alkyl** include methyl, ethyl, propyl, isopropyl, t-butyl, pentyl, iso-pentyl, 1-2-dimethylpropyl and hexyl; examples of **(2-6C)alkyl** include ethyl, propyl, isopropyl, t-butyl, pentyl, iso-pentyl, 1-2-dimethylpropyl and hexyl; examples of **(1-3C)alkoxy** include methoxy, ethoxy, propoxy and isopropoxy; examples of **(1-4C)alkoxy** include methoxy, ethoxy, propoxy, isopropoxy and tert-butoxy; examples of **(1-6C)alkoxy** include methoxy, ethoxy, propoxy, isopropoxy, tert-butoxy and pentoxy; examples of **(1-2C)alkoxy(1-2C)alkyl** include methoxymethyl, ethoxymethyl and methoxyethyl; examples of **(3-6C)cycloalkyl** cyclopropyl, cyclobutyl, cyclopentyl, cyclohexyl; examples of **(5-12C)bicycloalkyl** include norbornyl, decalinyl (bicyclo[4,4,0]decyl (cis and trans), bicyclo[5,3,0]decyl and hydrindanyl (bicyclo[4,3,0]nonyl); examples of **halo** are chloro, bromo, iodo and fluoro; examples of **halo(1-6C)alkyl** include **halo(1-4C)alkyl** such as chloromethyl, fluoroethyl, fluoromethyl, fluoropropyl, fluorobutyl, dichloromethyl, difluoromethyl, 1,2-difluoroethyl and 1,1-difluoroethyl as well as perhalo(1-6C)alkyl (including perhalo(1-4C)alkyl) such as trifluoromethyl, pentafluoroethyl, and heptafluoropropyl; examples of **halo(1-2C)alkyl** include fluoromethyl, difluoromethyl, trifluoromethyl, fluoroethyl and pentafluoroethyl; examples of **hydroxy(1-6C)alkyl** include hydroxy(1-3C)alkyl such as hydroxy methyl, 1-hydroxyethyl, 2-hydroxyethyl and 3-hydroxypropyl; examples of **(1-6C)alkoxycarbonyl** (N-(1-6C)alkylcarbamoyl) include (1-4C)alkoxycarbonyl such as methoxycarbonyl, ethoxycarbonyl, propoxycarbonyl, iso-propoxycarbonyl and tert-butoxycarbonyl; examples of **-S(O)ₘ(1-6C)alkyl** include methylthio, ethylthio, propylthio, isopropylthio and butylthio, methylsulfinyl, ethylsulfinyl, propylsulfinyl, isopropylsulfinyl and butylsulfinyl, methylsulfonyl, ethylsulfonyl, propylsulfonyl, isopropylsulfonyl and butylsulfonyl; examples of **-OS(O)₂(1-6C)alkyl** include methylsufonyloxy, ethylsulfonyloxy, propylsulfonyloxy, isopropylsulfonyloxy and tertbutylsulfonyloxy;

As used herein, the reference to carboxylic acid mimic or bioisostere includes groups as defined in The Practice of Medicinal Chemistry, Wermuth C.G. Ed.: Academic Press: New York, 1996, p203. Particular examples of such groups include -SO₃H, S(O)₂NHR¹³, -S(O)₂NHC(O)R¹³, -CH₂S(O)₂R¹³, -C(O)NHS(O)₂R¹³, -C(O)NHOH, -C(O)NHCN, -CH(CF₃)OH, C(CF₃)₂OH, -P(O)(OH)₂ and groups of sub-formula (a)-(i') below 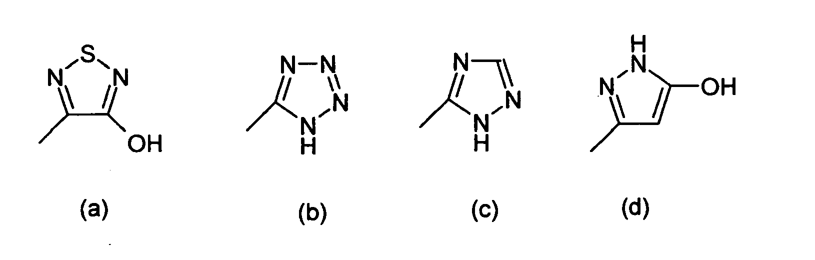 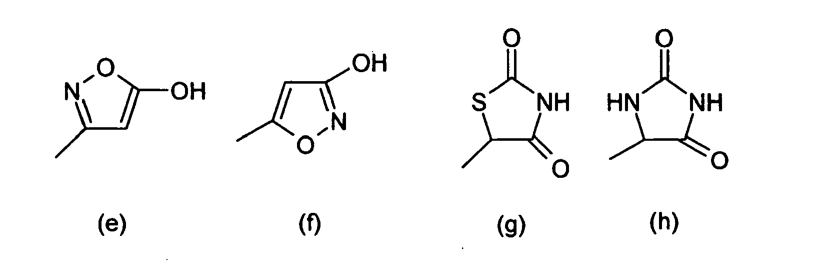 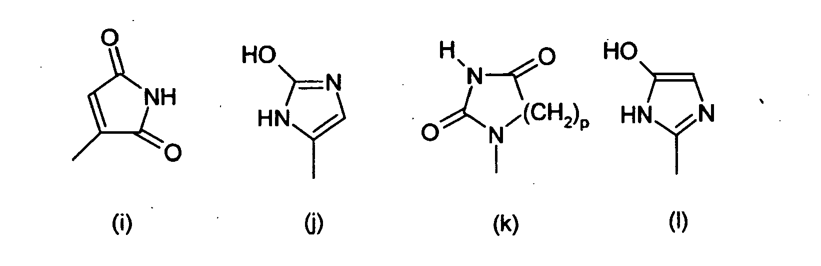 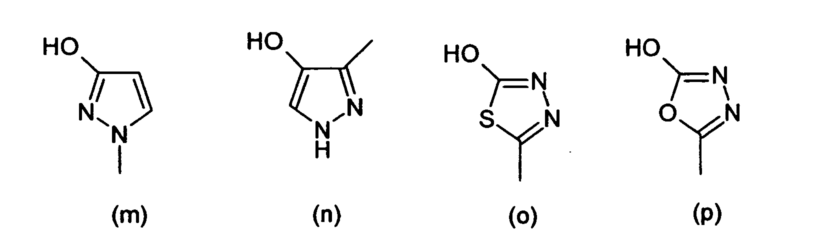 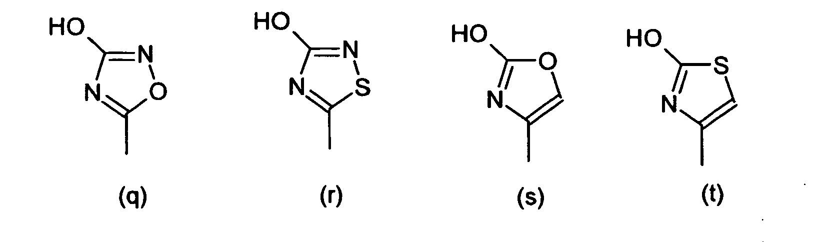 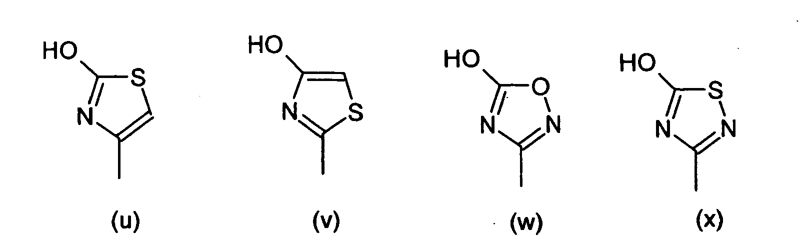 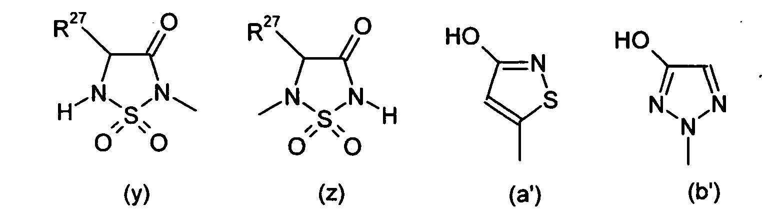 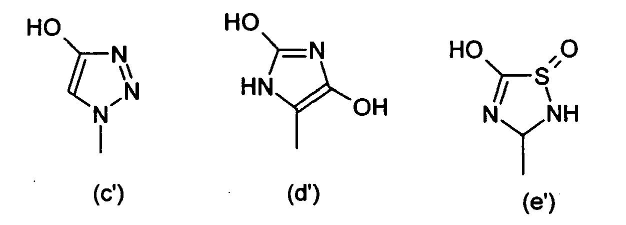 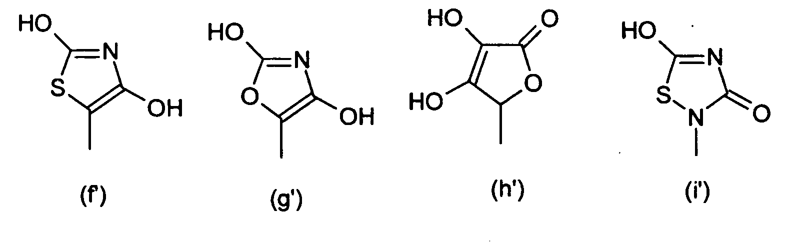 wherein R¹³ is (1-6C)alkyl, aryl or heteroaryl; and R²⁷ is hydrogen or (1-4C)alkyl. It will be understood that in the above sub-formulae (a) to (i'), keto-enol tautomerism may be possible and that the sub-formulae (a) to (i') should be taken to encompass all tautomers thereof.

In a further aspect of the invention, there is provided a compound of formula (IA), or a salt thereof, 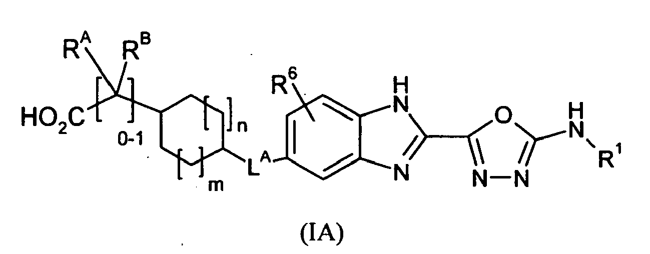 wherein R¹ is selected from phenyl, optionally substituted with 1 or 2 substituents independently selected from halo, halo(1-6C)alkyl, cyano, (1-6C)alkyl, hydroxy, (1-6C)alkoxy, -SOₘ(1-6C)alkyl and -OSO₂(1-6C)alkyl; or R¹ is optionally substituted with 1, 2, 3, or 4 fluoro;
R^{A} and R^{B} are each independently hydrogen or methyl;
R⁶ is hydrogen, fluoro, chloro or methyl;
L^{A} is a direct bond, -CH₂- or -O-;
m is 0, 1 or 2;
n is 0 or 1;
provided that m + n is 0, 1 or 2.

For the avoidance of doubt it is to be understood that where in this specification a group is qualified by 'hereinbefore defined' or 'defined hereinbefore' the said group encompasses the first occurring and broadest definition as well as each and all of the particular definitions for that group.

It is to be understood that where substituents contain two substituents on an alkyl chain, in which both are linked by a heteroatom (for example two alkoxy substituents), then these two substituents are not substituents on the same carbon atom of the alkyl chain.

Examples of HET-1 include oxazolyl, oxadiazolyl, pyridyl, pyrimidinyl, imidazolyl, pyrazinyl, pyridazinyl, pyrrolyl, thienyl, furyl, thiazolyl, isoxazolyl, pyrazolyl and isothiazolyl.

Examples of HET-2 include oxazolyl, oxadiazolyl, pyridyl, pyrimidinyl, imidazolyl, pyrazinyl, pyridazinyl, pyrrolyl, thienyl, furyl, thiazolyl, isoxazolyl, pyrazolyl, isothiazolyl, oxadiazolyl, thiadiazolyl, morpholinyl, thiomorpholinyl, piperazinyl, piperidinyl, 2-oxopiperidinyl, pyrrolidinyl, 2-oxopyrrolidinyl, tetrahydropyranyl, dihydropyranyl, tetrahydrothienyl, azetidinyl, homomorpholinyl, diazepinyl and azepinyl.

Examples of rings formed by NR²¹R²² include pyrrolidinyl, piperidinyl, piperazinyl, morpholino and thiomorpholino (and versions thereof wherein the sulfur has been oxidised to S(O) or (SO)₂).

If not stated elsewhere, suitable optional substituents for a particular group are those as stated for similar groups herein.

A compound of formula (I) may form stable acid or basic salts, and in such cases administration of a compound as a salt may be appropriate, and pharmaceutically acceptable salts may be made by conventional methods such as those described following.

Suitable pharmaceutically-acceptable salts include acid addition salts such as methanesulfonate, tosylate, α-glycerophosphate, fumarate, hydrochloride, citrate, maleate, tartrate and (less preferably) hydrobromide. Also suitable are salts formed with phosphoric and sulfuric acid. In another aspect suitable salts are base salts such as a group (I) (alkali metal) salt, a group (II) (alkaline earth metal) salt, an organic amine salt for example triethylamine, morpholine, *N*-methylpiperidine, *N*-ethylpiperidine, procaine, dibenzylamine, *N,N*-dibenzylethylamine, tris-(2-hydroxyethyl)amine, *N*-methyl d-glucamine and amino acids such as lysine. There may be more than one cation or anion depending on the number of charged functions and the valency of the cations or anions.

However, to facilitate isolation of the salt during preparation, salts which are less soluble in the chosen solvent may be preferred whether pharmaceutically-acceptable or not.

Within the present invention it is to be understood that a compound of the formula (I) or a salt thereof may exhibit the phenomenon of tautomerism and that the formulae drawings within this specification can represent only one of the possible tautomeric forms. It is to be understood that the invention encompasses any tautomeric form which inhibits DGAT1 activity and is not to be limited merely to any one tautomeric form utilised within the formulae drawings.

It will be appreciated by those skilled in the art that certain compounds of formula (I) contain asymmetrically substituted carbon and/or sulfur atoms, and accordingly may exist in, and be isolated in, optically-active and racemic forms. Some compounds may exhibit polymorphism. It is to be understood that the present invention encompasses any racemic, optically-active, polymorphic or stereoisomeric form, or mixtures thereof, which form possesses properties useful in the inhibition of DGAT1 activity, it being well known in the art how to prepare optically-active forms (for example, by resolution of the racemic form by recrystallization techniques, by synthesis from optically-active starting materials, by chiral synthesis, by enzymatic resolution, by biotransformation, or by chromatographic separation using a chiral stationary phase) and how to determine efficacy for the inhibition of DGAT1 activity by the standard tests described hereinafter.

It is also to be understood that certain compounds of the formula (I) and salts thereof can exist in solvated as well as unsolvated forms such as, for example, hydrated forms. It is to be understood that the invention encompasses all such solvated forms which inhibit DGAT1 activity.

As stated before, we have discovered a range of compounds that have good DGAT1 inhibitory activity. They have good physical and/or pharmacokinetic properties in general.

Particular aspects of the invention comprise a compound of formula (I), or a salt thereof, wherein the substituents R¹ to R⁷ and other substituents mentioned above have values defined hereinbefore, or any of the following values (which may be used where appropriate with any of the definitions and embodiments disclosed hereinbefore or hereinafter):

In one embodiment of the invention there are provided compounds of formula (I), in an alternative embodiment there are provided salts, particularly pharmaceutically-acceptable salts, of compounds of formula (I). In a further embodiment, there are provided salts, particularly pharmaceutically-acceptable salts of pro-drugs of compounds of formula (I). Reference herein to a compound of formula (I) should in general be taken to apply also to compounds of formula (IA).

Particular values of variable groups in compounds of formulae (I) are as follows. Such values may be used where appropriate with any of the other values, definitions, aspects, claims or embodiments defined hereinbefore or hereinafter.
1) R¹ is phenyl
2) R¹ is cyclopentyl or cyclohexyl
3) R¹ is HET-1
4) R¹ is substituted with 1 substituent
5) R¹ is substituted with 1 substituent selected from cyano, (1-6C)alkoxy, benzyloxy, - SO₂Me and chloro
6) R¹ is substituted with 2 alkoxy substituents joined together to form a ring fused to R¹
7) R¹ is substituted with 1, 2 or 3 fluoro
8) L¹ is a direct bond
9) L¹ is -O-
10) L¹ is -OCH₂- or -CH₂O-
11) L¹ is -S(O)ₘ-, -S(O)ₘCH₂- or -CH₂S(O)ₘ-
12) L¹ is -(CR⁷R⁸)₁₋₂-, such as -CH₂-
13) L¹ is a direct bond or -O-
14) R² is (3-6C)cycloalkyl, for example cyclohexyl
15) R² is (5-12C)bicycloalkyl
16) R² is phenyl
17) R² is HET-2
18) R² is (2-6C)alkyl
19) L² is a direct bond
20) L² is-(CR⁴R⁵)₁₋₂-
21) L² is -O-(CR⁴R⁵)₁₋₂-
22) L² is -CH₂(CR⁴R⁵)₁₋₂-
23) CR⁴R⁵ is CH₂
24) CR⁴R⁵ is CHMe
25) CR⁴R⁵ is CMe₂
26) CR⁴R⁵ is CH₂CH(OH)
27) CR⁴R⁵ is CH₂CH(CH₂OH)
28) CR⁴R⁵ is CH₂CH(CH₂OMe)
29) CR⁴R⁵ is CH(OH)
30) CR⁴R⁵ is CH(OMe)
31) L² is a direct bond or -CH₂-
32) R³ is hydroxy
33) R³ is carboxy
34) R³ is (1-6C)alkoxycarbonyl
35) R³ is a carboxylic acid mimic or bioisostere
36) R³ is carboxy or (1-6C)alkoxycarbonyl
37) R⁶ is hydrogen or fluoro
38) R⁶ is hydrogen

In one aspect of the invention, there is provided a compound of formula (I) or a salt thereof, wherein
R¹ is phenyl substituted with 1 substituent selected from cyano, (1-6C)alkoxy, benzyloxy, - SO₂Me and chloro, or substituted with 1, 2 or 3 fluoro;
L¹ is a direct bond or -0-;
R² is (3-6C)cycloalkyl, for example cyclohexyl;
L² is a direct bond or -CH₂-;
R³ is carboxy or (1-6C)alkoxycarbonyl; and
R⁶ is hydrogen.

In another aspect of the invention, there is provided a compound of formula (I) or a salt thereof, as described immediately above but wherein R⁶ is fluoro.

In other aspects of the invention, there are provided a compound of formula (I) or a salt thereof, as described in either of the two aspects immediately above wherein R¹ is phenyl substituted with 1 substituent selected from cyano, benzyloxy and chloro, or substituted with 1, 2 or 3 fluoro.

Further particular compounds of the invention are each of the Examples, each of which provides a further independent aspect of the invention. In further aspects, the present invention also comprises any two or more compounds of the Examples.

Particular compounds of the invention are any one or more of the following, or salts thereof:
trans-2-[4-[2-[5-[(2,4,5-trifluorophenyl)amino]-1,3,4-oxadiazol-2-yl]-1H-benzoimidazol-5-yl]cyclohexyl]acetic acid;
trans-2-[4-[2-[5-[(3,4-difluorophenyl)amino]-1,3,4-oxadiazol-2-yl]-1H-benzoimidazol-5-yl]cyclohexyl]acetic acid;
{trans-4-[2-(5-{[3-(benzyloxy)phenyl]amino}-1,3,4-oxadiazol-2-yl)-1H-benzimidazol-5-yl]cyclohexyl}acetic acid;
[trans-4-(2-{5-[(4-cyanophenyl)amino]-1,3,4-oxadiazol-2-yl}-1H-benzimidazol-5-yl)cyclohexyl]acetic acid;
trans-2-[4-[2-[5-[(2-methoxyphenyl)amino]-1,3,4-oxadiazol-2-yl]-1H-benzoimidazol-5-yl]cyclohexyl]acetic acid;
trans-2-[4-[2-[5-(7,10-dioxabicyclo[4.4.0]deca-1,3,5-trien-3-ylamino)-1,3,4-oxadiazol-2-yl]-1H-benzoimidazol-5-yl]cyclohexyl]acetic acid;
trans-2-[4-[2-[5-[(4-chlorophenyl)amino]-1,3,4-oxadiazol-2-yl]-1H-benzoimidazol-5-yl]cyclohexyl]acetic acid;
trans-2-[4-[2-[5-[(3-chlorophenyl)amino]-1,3,4-oxadiazol-2-yl]-1H-benzoimidazol-5-yl]cyclohexyl]acetic acid;
trans-2-[4-[2-[5-[(4-methylsulfonylphenyl)amino]-1,3,4-oxadiazol-2-yl]-1H-benzoimidazol-5-yl]cyclohexyl]acetic acid;
trans-2-[4-[2-[5-[(4-fluorophenyl)amino]-1,3,4-oxadiazol-2-yl]-1H-benzoimidazol-5-yl]cyclohexyl]acetic acid;
trans-2-[4-[2-[5-[(3,4,5-trifluorophenyl)amino]-1,3,4-oxadiazol-2-yl]-1H-benzoimidazol-5-yl]cyclohexyl]acetic acid;
cis-4-[[2-[5-[(2,4,5-trifluorophenyl)amino]-1,3,4-oxadiazol-2-yl]-3H-benzoimidazol-5-yl]oxy]cyclohexane-1-carboxylic acid;
cis-4-[[2-[5-[(4-fluorophenyl)amino]-1,3,4-oxadiazol-2-yl]-3H-benzoimidazol-5-yl]oxy]cyclohexane-1-carboxylic acid;
trans-4-[[2-[5-[(2,4,5-trifluorophenyl)amino]-1,3,4-oxadiazol-2-yl]-3H-benzoimidazol-5-yl]oxy]cyclohexane-1-carboxylic acid;
*cis*-4-{4-Fluoro-2-[5-(4-fluorophenylamino)-1,3,4-oxadiazol-2-yl]-1H-benzimidazol-5-yloxy}cyclohexanecarboxylic acid;
*trans*-4-{4-Fluoro-2-[5-(4-fluorophenylamino)-1,3,4-oxadiazol-2-yl]-1H-benzimidazol-5-yloxy}cyclohexanecarboxylic acid;
*cis-*4-[[4-fluoro-2-[5-[(2,4,5-trifluorophenyl)amino]-1,3,4-oxadiazol-2-yl]-3H-benzoimidazol-5-yl]oxy]cyclohexane-1-carboxylic acid;
*trans*-4-[[4-fluoro-2-[5-[(2,4,5-trifluorophenyl)amino]-1,3,4-oxadiazol-2-yl]-3H-benzoimidazol-5-yl]oxy]cyclohexane-1-carboxylic acid;
*trans*-3-[4-[2-[5-[(4-fluorophenyl)amino]-1,3,4-oxadiazol-2-yl]-1H-benzoimidazol-5-yl]cyclohexyl]oxypropanoic acid; and
*trans*-2-[4-[2-[5-[(4-fluorophenyl)amino]-1,3,4-oxadiazol-2-yl]-1H-benzoimidazol-5-yl]cyclohexyl]oxyacetic acid.

Intermediates 1, 2, 3, 4, 5, 6, 7, 8, 9, 10, 11, 30, 31, 32 and other intermediates described herein are also within the scope of the present invention and are thereby each provided as separate independent aspects of the invention.

### Process

A compound of formula (I) and its salts may be prepared by any process known to be applicable to the preparation of chemically related compounds. Such processes, when used to prepare a compound of the formula (I), or a salt thereof, are provided as a further feature of the invention.

In a further aspect the present invention also provides that the compounds of the formula (I) and salts thereof, can be prepared by a process a) to d) as follows (wherein all variables are as hereinbefore defined for a compound of formula (I) unless otherwise stated):
a) reaction of a compound of formula (I) to form another compound of formula (I);
b) cyclisation of a compound of formula (2); 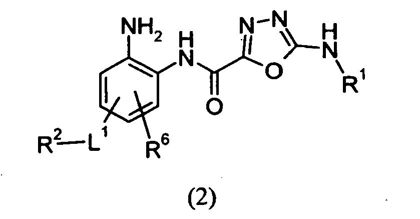
c) when L¹ is -0- or -O-CH₂-, by reaction of a compound of formula (3), or the equivalent hydroxy-methyl compound, with a compound of formula R²-L¹-X¹, wherein X¹ is a suitable leaving group; 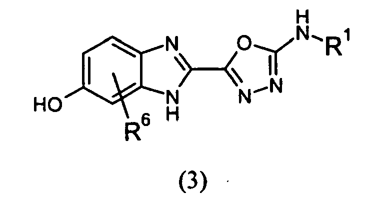
d) by reaction of a compound of formula (4) with a compound of formula R²-L¹-X², wherein X² is for example a boronic acid, stannane or a sulfide, L¹ is a direct bond and X is suitably halo; 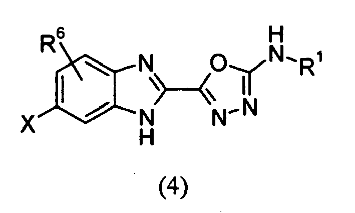
and thereafter if necessary or desirable:
i) removing any protecting groups; and/or
ii) forming a salt thereof.

### Process a)

Examples of conversions of a compound of formula (I) into another compound of Formula (I), well known to those skilled in the art, include functional group interconversions such as hydrolysis (in particular ester hydrolysis), oxidation or reduction (such as the reduction of an acid to an alcohol), and/or further functionalisation by standard reactions such as amide or metal-catalysed coupling, or nucleophilic displacement reactions.

### Process b)

Cyclisation of the compound of formula (2) may be carried out by treatment with acid, for example acetic acid.

The compound of formula (2) may be made by reduction of a compound of formula (5) for example using palladium/carbon or platinum/carbon as hydrogenation catalysts.

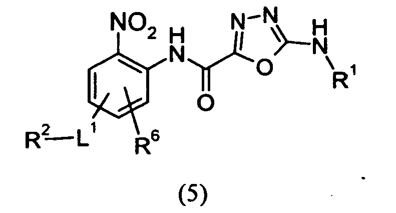

Under some conditions, reduction of the nitro group to give the amino group and cyclisation to give the benzimidazole ring may happen in one step, as illustrated in Scheme 1 below:

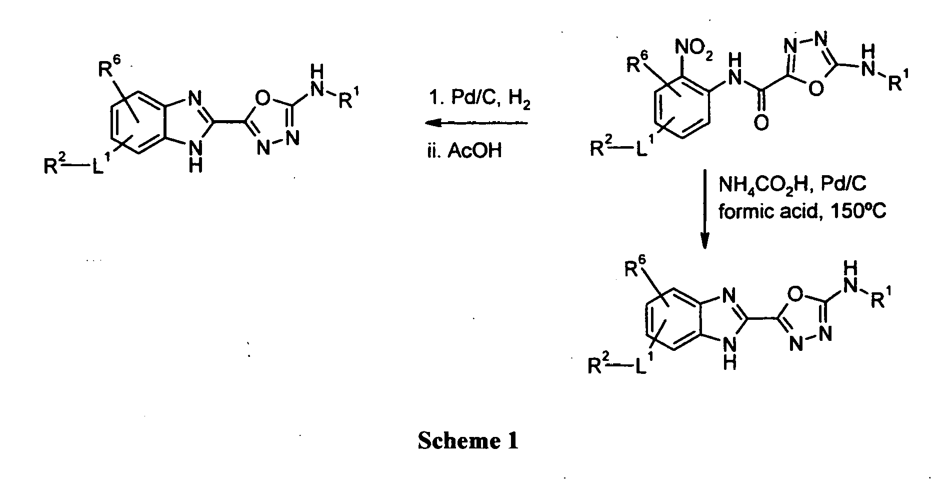

A compound of formula (5) may be made by cyclisation of a hydrazine derivative as illustrated in Scheme 2 below:

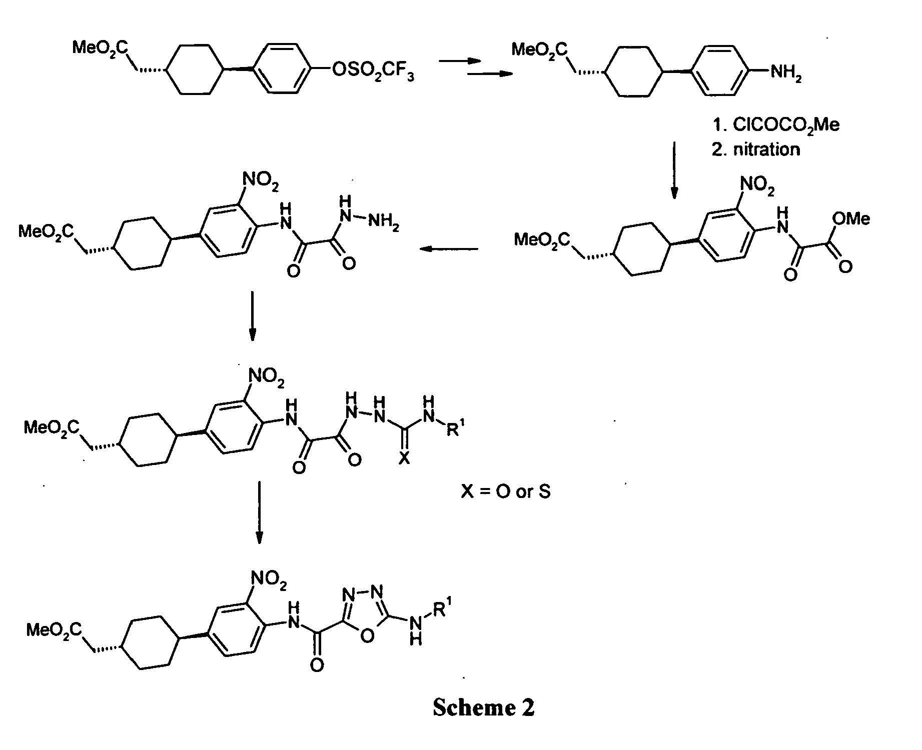

For values of -L¹-R² other than that shown in Scheme 2, analogous amine intermediates may be made as illustrated in Schemes 3 to 5 (where P is a suitable protecting group). 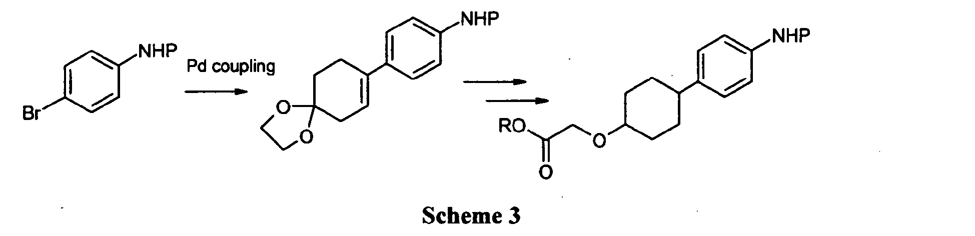 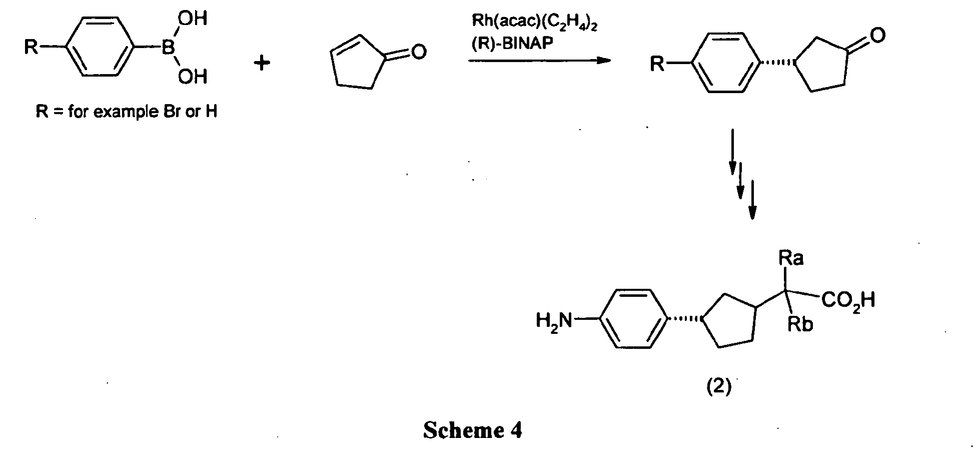 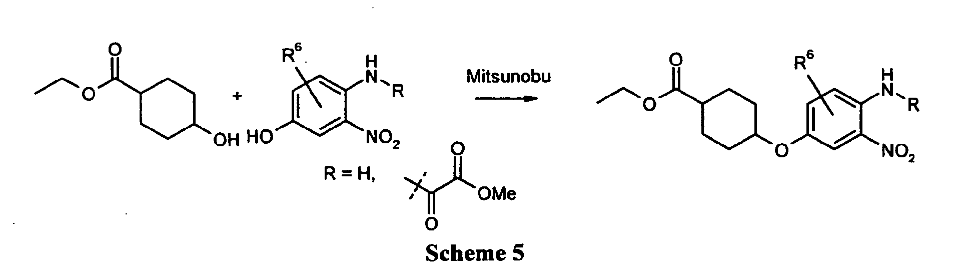 [acac = acetylacetone]

In one particular embodiment of Scheme 5 R⁶ is fluoro.

Compounds of formula (5) may also be made by coupling a compound of formula (6) with a compound of formula (7), for example using a palladium catalyst such as *tris*(dibenzylideneacetone)dipalladium(0) and the appropriate ligand such as xantphos (Buchwald reaction). 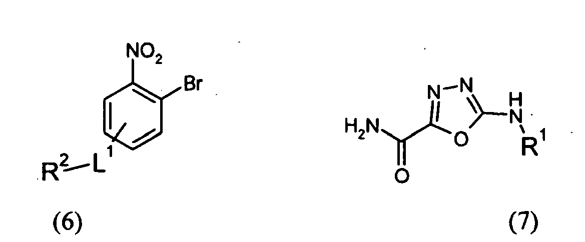 Compounds of formula (5) may also be made by coupling a compound of formula (8) with a salt of formula (9) (wherein M is a suitable metal counterion, for example sodium). For example using an appropriate coupling reaction, such as a carbodiimide coupling reaction performed with EDAC, optionally in the presence of DMAP, in a suitable solvent such as DCM, chloroform or DMF at room temperature.

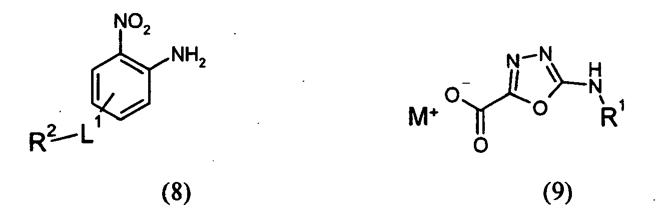

Compounds of formula (6) may be made, for example, from the compounds below using metal catalysed coupling reactions for example using palladium or Mitsunobu or alkylation reactions with the phenol. Both compounds can be synthesised as described in J. Am. Chem. Soc. 1952, 74, 1574.

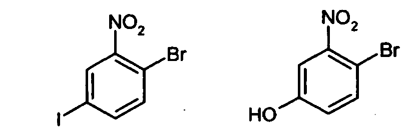

Compounds of formula (8) may be made, for example, from the compounds below using metal catalysed coupling reactions for example using palladium catalysis or Mitsunobu conditions (as shown in Scheme 5) or alkylation reactions with the phenol.

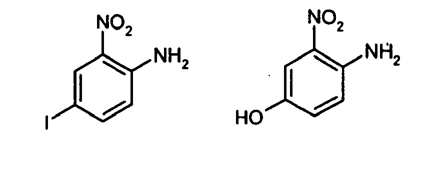

Compounds of formula (7) and (9) may be made by aminolysis or alkaline hydrolysis of ester (10) as prepared using a published procedure (J. Het. Chem. 1977, 14, 1385-1388). Ester (10) may be made by cyclisation of a compound of formula (11) (where X is O or S), which itself may be made by reaction of an iso(thiocyanate) R¹- NCX (where X is O or S) with a suitable hydrazide intermediate.

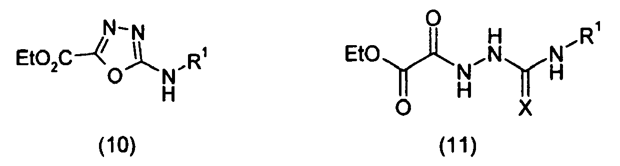

Iso(thio)cyanates R¹- NCX (where X is O or S) are commercially available or may be made by reaction of the aniline R¹-NH₂ with for example (thio)phosgene or a (thio)phosgene equivalent followed by a suitable base (such as triethylamine).

An alternative method for making compounds of formula (8a) is illustrated below:

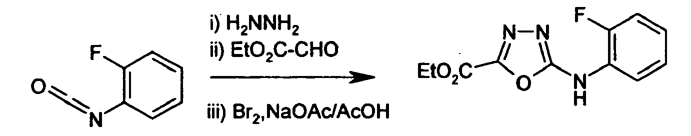

### Process c)

Compounds of formula (3) may be reacted with compounds of formula R²-L¹-X¹ under a variety of conditions depending on the nature of X¹. For example if X¹ is Hand L¹ is -O- and R² is not aromatic then well-known conditions suitable for the Mitsunobu reaction may be used, for example,

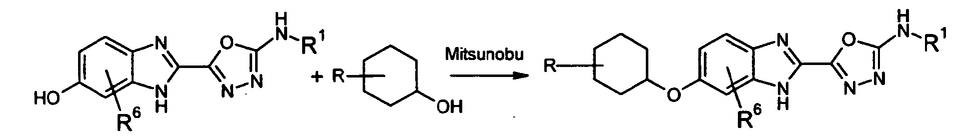

Alternatively, if X¹ is a leaving group such as halo and at least either R² is not aromatic (for example R² is alkyl) or L¹ is -OCH₂-, then an alkylation reaction can be used.

Compounds of formula (3) may be made by an analogous method to that shown in Scheme 6 below, with a protected oxygen substituent (for example benzyloxy) instead of bromine in the starting material.

### Process d)

Process d) may be carried out for example by palladium catalysed cross coupling reactions, for example, when X² is suitably a boronic acid using tetrakis(triphenylphosphine) palladium(0) and a base such as potassium carbonate (Suzuki reaction) or a stannane using *tris*(dibenzylideneacetone)dipalladium(0) and triphenylarsine (Stille reaction) or a suitable group such as sulfide using metal catalysed conditions such as the palladium catalysed conditions described in J. Am. Chem. Soc. 2006, 128, 2180.

Compounds of formula (4) may be made as illustrated in Scheme 6: 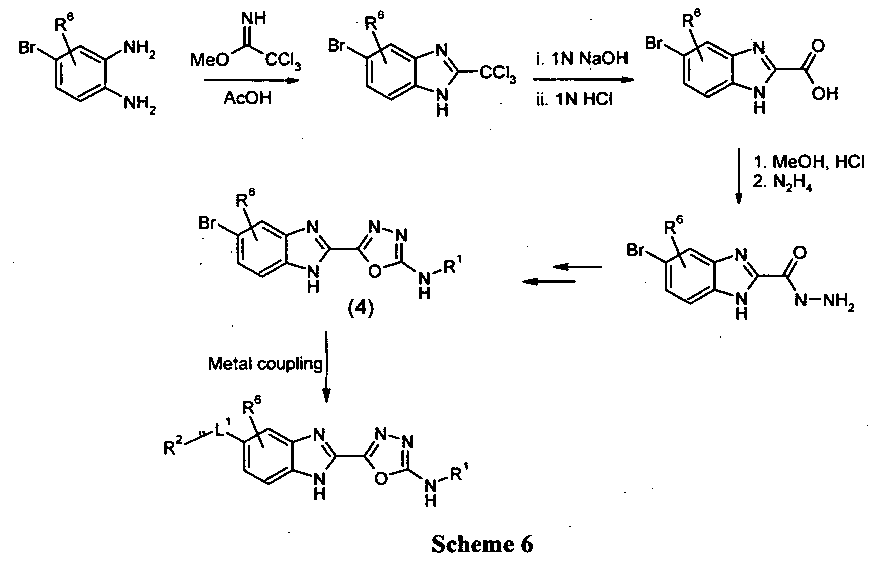 Alternatively compounds of this type can be made as shown in Scheme 7:

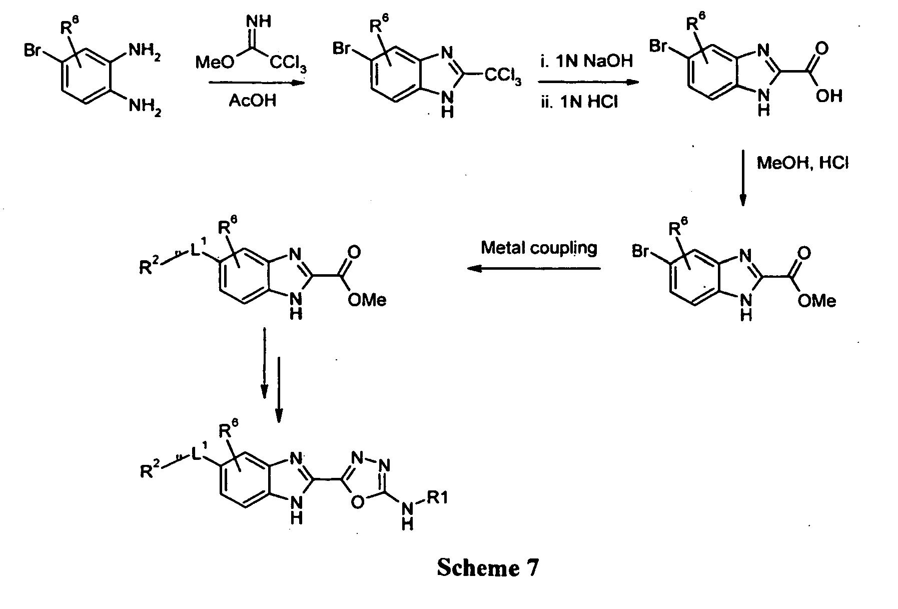

It will be appreciated that during the above reactions, certain groups may need protecting, for example the NH of the benzimidazole ring, if present, or the NH linked to the oxadiazole ring, or precursor thereof.

It will be appreciated that certain of the various ring substituents in the compounds of the present invention, for example R⁶, may be introduced by standard aromatic substitution reactions or generated by conventional functional group modifications either prior to or immediately following the processes mentioned above, and as such are included in the process aspect of the invention. Such reactions may convert one compound of the formula (I) into another compound of the formula (I). Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogen group. Particular examples of modifications include the reduction of a nitro group to an amino group by for example, catalytic hydrogenation with a nickel catalyst or treatment with iron in the presence of hydrochloric acid with heating; oxidation of alkylthio to alkanesulfinyl or alkanesulfonyl.

If not commercially available, the necessary starting materials for the procedures such as those described above may be made by procedures which are selected from standard organic chemical techniques, techniques which are analogous to the synthesis of known, structurally similar compounds, techniques which are described or illustrated in the references given above, or techniques which are analogous to the above described procedure or the procedures described in the examples. The reader is further referred to Advanced Organic Chemistry, 5th Edition, by Jerry March and Michael Smith, published by John Wiley & Sons 2001, for general guidance on reaction conditions and reagents.

It will be appreciated that some intermediates to compounds of the formula (I) are also novel and these are provided as separate independent aspects of the invention. In particular, certain compounds of formulae (2), (3), (4) and/or (5) are each provided as independent aspects of the invention.

It will also be appreciated that in some of the reactions mentioned herein it may be necessary/desirable to protect any sensitive groups in compounds. The instances where protection is necessary or desirable are known to those skilled in the art, as are suitable methods for such protection. Conventional protecting groups may be used in accordance with standard practice (for illustration see T.W. Greene, Protective Groups in Organic Synthesis, John Wiley and Sons, 1991).

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Thus, if reactants include, for example, groups such as amino, carboxy or hydroxy it may be desirable to protect the group in some of the reactions mentioned herein.

Examples of a suitable protecting group for a hydroxy group is, for example, an acyl group, for example an alkanoyl group such as acetyl, an aroyl group, for example benzoyl, a silyl group such as trimethylsilyl or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively a silyl group such as trimethylsilyl or SEM may be removed, for example, by fluoride or by aqueous acid; or an arylmethyl group such as a benzyl group may be removed, for example, by hydrogenation in the presence of a catalyst such as palladium-on-carbon.

A suitable protecting group for an amino group is, for example, an acyl group, for example an alkanoyl group such as acetyl, an alkoxycarbonyl group, for example a methoxycarbonyl, ethoxycarbonyl or tert-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a t-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine or 2-hydroxyethylamine, or with hydrazine.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon.

Resins may also be used as a protecting group.

The protecting groups may be removed at any convenient stage in the synthesis using conventional techniques well known in the chemical art, or they may be removed during a later reaction step or work-up.

The skilled organic chemist will be able to use and adapt the information contained and referenced within the above references, and accompanying Examples therein and also the examples herein, to obtain necessary starting materials, and products.

The removal of any protecting groups and the formation of a (pharmaceutically-acceptable) salt are within the skill of an ordinary organic chemist using standard techniques. Furthermore; details on the these steps has been provided hereinbefore.

When an optically active form of a compound of the invention is required, it may be obtained by carrying out one of the above procedures using an optically active starting material (formed, for example, by asymmetric induction of a suitable reaction step), or by resolution of a racemic form of the compound or intermediate using a standard procedure, or by chromatographic separation of diastereoisomers (when produced). Enzymatic techniques may also be useful for the preparation of optically active compounds and/or intermediates.

Similarly, when a pure regioisomer of a compound of the invention is required, it may be obtained by carrying out one of the above procedures using a pure regioisomer as a starting material, or by resolution of a mixture of the regioisomers or intermediates using a standard procedure.

According to a further aspect of the invention there is provided a pharmaceutical composition which comprises a compound of formula (I) and (IZA) as defined hereinbefore or a pharmaceutically-acceptable salt thereof, in association with a pharmaceutically-acceptable excipient or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as com starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial. Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

According to a further aspect of the present invention there is provided a compound of formula (I) or a pharmaceutically acceptable salt thereof as defined hereinbefore for use in a method of treatment of the human or animal body by therapy.

Reference herein to a compound of formula (I) should be understood to refer equally to compounds of formula (I) and (IZA).

We have found that compounds of the present invention inhibit DGAT1 activity and are therefore of interest for their blood glucose-lowering effects.

A further feature of the present invention is a compound of formula (I) or a pharmaceutically-acceptable salt thereof for use as a medicament.

Conveniently this is a compound of formula (I), or a pharmaceutically-acceptable salt thereof, for use as a medicament for producing an inhibition of DGAT1 activity in a warm-blooded animal such as a human being.

Particularly this is a compound of formula (I), or a pharmaceutically-acceptable salt thereof, for use as a medicament for treating diabetes mellitus and/or obesity in a warm-blooded animal such as a human being.

Thus according to a further aspect of the invention there is provided the use of a compound of formula (I), or a pharmaceutically-acceptable salt thereof in the manufacture of a medicament for use in the production of an inhibition of DGAT1 activity in a warm-blooded animal such as a human being.

Thus according to a further aspect of the invention there is provided the use of a compound of formula (I), or a pharmaceutically-acceptable salt thereof in the manufacture of a medicament for use in the treatment of diabetes mellitus and/or obesity in a warm-blooded animal such as a human being.

According to a further aspect of the invention there is provided a pharmaceutical composition which comprises a compound of formula (I) as defined hereinbefore or a pharmaceutically-acceptable salt thereof, in association with a pharmaceutically-acceptable excipient or carrier for use in producing an inhibition of DGAT1 activity in an warm-blooded animal, such as a human being.

According to a further aspect of the invention there is provided a pharmaceutical composition which comprises a compound of formula (I) as defined hereinbefore or a pharmaceutically-acceptable salt thereof, in association with a pharmaceutically-acceptable excipient or carrier for use in the treatment of diabetes mellitus and/or obesity in an warm-blooded animal, such as a human being.

According to a further feature of the invention there is provided a method for producing an inhibition of DGAT1 activity in a warm-blooded animal, such as a human being, in need of such treatment which comprises administering to said animal an effective amount of a compound of formula (I) or a pharmaceutically-acceptable salt thereof as defined hereinbefore.

According to a further feature of the invention there is provided a method of treating diabetes mellitus and/or obesity in a warm-blooded animal, such as a human being, in need of such treatment which comprises administering to said animal an effective amount of a compound of formula (I) or a pharmaceutically-acceptable salt thereof as defined hereinbefore.

As stated above the size of the dose required for the therapeutic or prophylactic treatment of a particular disease state will necessarily be varied depending on the host treated, the route of administration and the severity of the illness being treated. Preferably a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

As stated above compounds defined in the present invention are of interest for their ability to inhibit the activity of DGAT1. A compound of the invention may therefore be useful for the prevention, delay or treatment of a range of disease states including diabetes mellitus, more specifically type 2 diabetes mellitus (T2DM) and complications arising there from (for example retinopathy, neuropathy and nephropathy), impaired glucose tolerance (IGT), conditions of impaired fasting glucose, metabolic acidosis, ketosis, dysmetabolic syndrome, arthritis, osteoporosis, obesity and obesity related disorders, (which include peripheral vascular disease, (including intermittent claudication), cardiac failure and certain cardiac myopathies, myocardial ischaemia, cerebral ischaemia and reperfusion, hyperlipidaemias, atherosclerosis, infertility and polycystic ovary syndrome); the compounds of the invention may also be useful for muscle weakness, diseases of the skin such as acne, Alzheimer's disease, various immunomodulatory diseases (such as psoriasis), HIV infection, inflammatory bowel syndrome and inflammatory bowel disease such as Crohn's disease and ulcerative colitis.

In particular, the compounds of the present invention are of interest for the prevention, delay or treatment of diabetes mellitus and/or obesity and/or obesity related disorders. In one aspect, the compounds of the invention are used for prevention, delay or treatment of diabetes mellitus. In another aspect, the compounds of the invention are used for prevention, delay or treatment of obesity. In a further aspect, the compounds of the invention are used for prevention, delay or treatment of obesity related disorders.

The inhibition of DGAT1 activity described herein may be applied as a sole therapy or in combination with one or more other substances and/or treatments for the indication being treated. Such conjoint treatment may be achieved by way of the simultaneous, sequential or separate administration of the individual components of the treatment. Simultaneous treatment may be in a single tablet or in separate tablets. For example such conjoint treatment may be beneficial in the treatment of metabolic syndrome [defined as abdominal obesity (as measured by waist circumference against ethnic and gender specific cut-points) plus any two of the following: hypertriglyceridemia (> 150 mg/dl; 1.7mmol/l); low HDLc (<40 mg/dl or <1.03mmol/l for men and <50 mg/dl or 1.29 mmol/l for women) or on treatment for low HDL (high density lipoprotein); hypertension (SBP ≥ 130 mmHg DBP ≥ 85 mmHg) or on treatment for hypertension; and hyperglycemia (fasting plasma glucose ≥ 100 mg/dl or 5.6 mmol/l or impaired glucose tolerance or pre-existing diabetes mellitus) - International Diabetes Federation & input from IAS/NCEP].

Such conjoint treatments may include the following main categories:
1) Anti-obesity therapies such as those that cause weight loss by effects on food intake, nutrient absorption or energy expenditure, such as orlistat, sibutramine and the like.
2) Insulin secretagogues including sulphonylureas (for example glibenclamide, glipizide), prandial glucose regulators (for example repaglinide, nateglinide);
3) Agents that improve incretin action (for example dipeptidyl peptidase IV inhibitors, and GLP-1 agonists);
4) Insulin sensitising agents including PPARgamma agonists (for example pioglitazone and rosiglitazone), and agents with combined PPARalpha and gamma activity;
5) Agents that modulate hepatic glucose balance (for example metformin, fructose 1, 6 bisphosphatase inhibitors, glycogen phopsphorylase inhibitors, glycogen synthase kinase inhibitors, glucokinase activators);
6) Agents designed to reduce the absorption of glucose from the intestine (for example acarbose);
7) Agents that prevent the reabsorption of glucose by the kidney (SGLT inhibitors);
8) Agents designed to treat the complications of prolonged hyperglycaemia (for example aldose reductase inhibitors);
9) Anti- dyslipidaemia agents such as, HMG-CoA reductase inhibitors (eg statins); PPARα-agonists (fibrates, eg gemfibrozil); bile acid sequestrants (cholestyramine); cholesterol absorption inhibitors (plant stanols, synthetic inhibitors); bile acid absorption inhibitors (IBATi) and nicotinic acid and analogues (niacin and slow release formulations);
10) Antihypertensive agents such as, β-blockers (eg atenolol, inderal); ACE inhibitors (eg lisinopril); Calcium antagonists (eg. nifedipine); Angiotensin receptor antagonists (eg candesartan), α antagonists and diuretic agents (eg. furosemide, benzthiazide);
11) Haemostasis modulators such as, antithrombotics, activators of fibrinolysis and antiplatelet agents; thrombin antagonists; factor Xa inhibitors; factor VIIa inhibitors); antiplatelet agents (eg. aspirin, clopidogreI); anticoagulants (heparin and Low molecular weight analogues, hirudin) and warfarin;
12) Agents which antagonise the actions of glucagon; and
13) Anti-inflammatory agents, such as non-steroidal anti-inflammatory drugs (eg. aspirin) and steroidal anti-inflammatory agents (eg. cortisone).

In addition to their use in therapeutic medicine, compounds of formula (I) and their pharmaceutically-acceptable salts are also useful as pharmacological tools in the development and standardisation of *in vitro* and *in vivo* test systems for the evaluation of the effects of inhibitors of DGAT1 activity in laboratory animals such as cats, dogs, rabbits, monkeys, rats and mice, as part of the search for new therapeutic agents.

As indicated above, all of the compounds, and their corresponding salts, are useful in inhibiting DGAT1. The ability of the compounds of formula (I), and their corresponding acid addition salts, to inhibit DGAT1 may be demonstrated employing the following enzyme assay:

### Human Enzyme Assay

The *in vitro* assay to identify DGAT1 inhibitors uses human DGAT1 expressed in insect cell membranes as the enzyme source (Proc. Natl. Acad. Sci. 1998, 95, 13018-13023). Briefly, sf9 cells were infected with recombinant baculovirus containing human DGAT1 coding sequences and harvested after 48 h. Cells were lysed by sonication and membranes isolated by centrifuging at 28000 rpm for 1 h at 4 °C on a 41% sucrose gradient. The membrane fraction at the interphase was collected, washed, and stored in liquid nitrogen.

DGAT1 activity was assayed by a modification of the method described by Coleman (Methods in Enzymology 1992, 209, 98-102). Compound at 1-10 µM was incubated with 0.4 µg membrane protein, 5 mM MgCl₂, and 100µM 1,2 dioleoyl-sn-glycerol in a total assay volume of 200 µl in plastic tubes. The reaction was started by adding ¹⁴C oleoyl coenzyme A (30µM final concentration) and incubated at room temperature for 30 minutes. The reaction was stopped by adding 1.5 mL 2-propanol:heptane:water (80:20:2). Radioactive triolein product was separated into the organic phase by adding 1mL heptane and 0.5 mL 0.1 M carbonate buffer pH 9.5. DGAT1 activity was quantified by counting aliquots of the upper heptane layer by liquid scintillography.

Using this assay the compounds generally show activity with IC₅₀ <10µM, particularly < 1 µM. Example 10 showed an IC₅₀ = 0.01 µM and Examples 1 to 4 the following respectively; 0.011 µM; 0.01 µM; 0.006 µM and 0.019 µM. Examples 6 to 8 showed the following respectively; 0.054 µM; 0.002 µM and 0.008 µM. Example 11 showed an IC₅₀ = 0.003 µM and Examples 14 to 18 the following respectively; 0.043 µM; 0.019 µM; 0.023 µM; 0.013 µM and 0.016 µM.

The ability of the compounds of formula (I), and their corresponding pharmaceutically-acceptable acid salts, to inhibit DGAT1 may further be demonstrated employing the following whole cell assays 1) and 2):

### 1) Measurement of Triglyceride Synthesis in 3T3 Cells

Mouse adipocyte 3T3 cells were cultured to confluency in 6 well plates in new born calf serum containing media. Differentiation of the cells was induced by incubating in medium containing 10% foetal calf serum, 1 µg/mL insulin, 0.25 µM dexamethasone and 0.5 mM isobutylmethyl xanthine. After 48 h the cells were maintained in medium containing 10% foetal calf serum and 1 µg/mL insulin for a further 4-6 days. For the experiment, the medium was changed to serum-free medium and the cells pre-incubated with compound solubilised in DMSO (final concentration 0.1%) for 30 minutes. De novo lipogenesis was measured by the addition of 0.25 mM sodium acetate plus 1 µCi/mL ¹⁴C-sodium acetate to each well for a further 2 h (J. Biol. Chem., 1976, 251, 6462-6464). The cells were washed in phosphate buffered saline and solubilised in 1% sodium dodecyl sulfate. An aliquot was removed for protein determination using a protein estimation kit (Perbio) based on the method of Lowry (J. Biol. Chem., 1951, 193, 265-275). The lipids were extracted into the organic phase using a heptane:propan-2-ol:water (80:20:2) mixture followed by aliquots of water and heptane according to the method of Coleman (Methods in Enzymology, 1992, 209, 98-104). The organic phase was collected and the solvent evaporated under a stream of nitrogen. The extracts solubilised in iso-hexane:acetic acid (99:1) and lipids separated via normal phase high performance liquid chromatography (HPLC) using a Lichrospher diol-5,4 × 250 mm column and a gradient solvent system of iso-hexane:acetic acid (99:1) and iso-hexane:propan-2-ol:acetic acid (85:15:1), flow rate of 1 mL/minute according to the method of Silversand and Haux (1997). Incorporation of radiolabel into the triglyceride fraction was analysed using a Radiomatic Flo-one Detector (Packard) connected to the HPLC machine.

### 2) Measurement of Triglyceride Synthesis in MCF7 Cells

Human mammary epithelial (MCF7) cells were cultured to confluency in 6 well plates in foetal calf serum containing media. For the experiment, the medium was changed to serum-free medium and the cells pre-incubated with compound solubilised in DMSO (final concentration 0.1%) for 30 minutes. De novo lipogenesis was measured by the addition of 50 µM sodium acetate plus 3 µCi/mL ¹⁴C-sodium acetate to each well for a further 3 h (J. Biol. Chem., 1976,251,6462-6464). The cells were washed in phosphate buffered saline and solubilised in 1% sodium dodecyl sulfate. An aliquot was removed for protein determination using a protein estimation kit (Perbio) based on the method of Lowry (J. Biol. Chem., 1951, 193, 265-275). The lipids were extracted into the organic phase using a heptane:propan-2-ol:water (80:20:2) mixture followed by aliquots of water and heptane according to the method of Coleman (Methods in Enzymology, 1992, 209, 98-104). The organic phase was collected and the solvent evaporated under a stream of nitrogen. The extracts solubilised in iso-hexane:acetic acid (99:1) and lipids separated via normal phase high performance liquid chromatography (HPLC) using a Lichrospher diol-5, 4 × 250 mm column and a gradient solvent system of iso-hexane:acetic acid (99: 1) and iso-hexane:propan-2-ol:acetic acid (85:15:1), flow rate of 1 mL/minute according to the method of Silversand and Haux (J. Chromat. B, 1997, 703, 7-14). Incorporation of radiolabel into the triglyceride fraction was analysed using a Radiomatic Flo-one Detector (Packard) connected to the HPLC machine.

In the above other pharmaceutical composition, process, method, use and medicament manufacture features, the alternative and preferred embodiments of the compounds of the invention described herein also apply.

### Examples

The invention will now be illustrated by the following Examples in which, unless stated otherwise:
(i) temperatures are given in degrees Celsius (°C); operations were carried out at room or ambient temperature, that is, at a temperature in the range of 18-25 °C and under an atmosphere of an inert gas such as argon;
(ii) organic solutions were dried over anhydrous magnesium sulfate; evaporation of solvent was carried out using a rotary evaporator under reduced pressure (600-4000 Pa; 4.5-30 mmHg) with a bath temperature of up to 60 °C;
(iii) chromatography means flash chromatography on silica gel; where a Biotage cartridge is referred to this means a cartridge containing KP-SIL^{™} silica, 60Å, particle size 32-63 mM, supplied by Biotage, a division of Dyax Corp., 1500 Avon Street Extended, Charlottesville, VA 22902, USA;
(iv) in general, the course of reactions was followed by TLC and reaction times are given for illustration only;
(v) yields are given for illustration only and are not necessarily those which can be obtained by diligent process development; preparations were repeated if more material was required;
(vi) where given, NMR data (¹H) is in the form of delta values for major diagnostic protons, given in parts per million (ppm) relative to tetramethylsilane (TMS), determined at 300 or 400 MHz (unless otherwise stated) using perdeuterio dimethyl sulfoxide (DMSO-d₆) as solvent, unless otherwise stated; peak multiplicities are shown thus: s, singlet; d, doublet; dd, doublet of doublets; dt, doublet of triplets; dm, doublet of multiplets; t, triplet, q, quartet; m, multiplet; br, broad;
(vii) chemical symbols have their usual meanings; SI units and symbols are used;
(viii) solvent ratios are given in volume : volume (v/v) terms;
(ix) mass spectra (MS) (loop) were recorded on a Micromass Platform LC equipped with HP 1100 detector; unless otherwise stated the mass ion quoted is (MH⁺);
(x) LCMS (liquid chromatography-mass spectrometry) were recorded on a system comprising Waters 2790 LC equipped with a Waters 996 Photodiode array detector and Micromass ZMD MS, using a Phenomenex® Gemini 5u C18 110A 50x2 mm column and eluting with a flow rate of 1.1 ml/min with 5% (Water/Acetonitrile (1:1) + 1% formic acid) and a gradient increasing from 0-95% of acetonitrile over the first 4 minutes, the balance (95-0%) being water and where HPLC Retention Times are reported these are in minutes in this system unless otherwise stated; unless otherwise stated the mass ion quoted is (MH⁺);
(xi) where phase separation cartridges are stated then ISOLUTE Phase Separator 70ml columns, supplied by Argonaut Technologies, New Road, Hengoed, Mid Glamorgan, CF82 8AU, United Kingdom, were used;
(xii) where a SiliCycle cartridge is referred to this means a cartridge containing Ultra Pure Silica Gel particle size 230-400 mesh, 40 -63 um pore size, supplied by SiliCycle Chemical Division, 1200 Ave St-Jean-Baptiste, Suite 114, Quebec City, Quebec, G2E 5E8, CANADA;
(xiii) where an Isco Companion is referred to then a Combiflash companion chromatography instrument, supplied by ISOC Inc. Address Teledyne ISOC Inc, 4700 Superior Street, Lincoln, NE 68504, USA, was used;
(xiv) where a microwave is referred to this means a Biotage Initiator sixty or Smith Creator microwave, supplied by Biotage, a division of Dyax Corp., 1500 Avon Street Extended, Charlottesville, VA 22902, USA;
(xv) where GCMS is referred to then a Gas Chromatography -Mass Spectrometry analysis was carried out on a QP-2010 GC-MS system fitted with an AOC 20i autosampler and controlled by 'GCMS solutions' software, version 2.0, supplied by Shimadzu, Milton Keynes, MK12 5RE, UK; the GC column was a DB-5MS of length 25 m, 0.32 mm i.d. with a film thickness of 0.52 µm supplied by J & W Scientific, Folsom, CA, USA;
(xvi) where a centrifuge is referred to this means a Genevac EZ-2plus, supplied by Genevac Limited, The Soveriegn Centre, Farthing Road, Ipswich, IP1 5AP, UK;
(xvii) where chiral chromatography is referred to this is carried generally out using a 20µm Merck 50mm Chiralpak AD column, (Chiral Stationary Phase supplied by Chiral Technologies Europe, Parc d'Innovation, Bd. Gonthier d'Andernach, 67404 Illkirch Cedex, France), using MeCN/2-propanol/AcOH (90/10/0.1) as eluent, flow rate 80 mL/min, wavelength 300nm, using a Gilson prep HPLC instrument (200ml heads);
(xviii) melting points were determined using a Buchi 530 apparatus and are uncorrected;
(xix) the large scale hydrogenation at 2 bar for Intermediate 1ii was carried out using a Buchi hydrogenator Model 280 (Buchi AG, Gschwaderstrasse 12, CH 8610, Uster / Switzerland);
(xx) The following abbreviations may be used below or in the process section hereinbefore:
   - Et₂O or ether: diethyl ether
   - DMF: dimethylformamide
   - DCM: dichloromethane
   - DME: 1,2-dimethoxyethane
   - MeOH: methanol
   - EtOH: ethanol
   - H₂O: water
   - TFA: trifluoroacetic acid
   - THF: tetrahydrofuran
   - DMSO: dimethylsulfoxide
   - HOBt: 1-hydroxybenzotriazole
   - EDCI (EDAC): 1-ethyl-3-(3-dimethylaminopropyl)carbodi-imide hydrochloride
   - DIPEA: diisopropylethylamine
   - DEAD: diethyl azodicarboxylate
   - EtOAc: ethyl acetate
   - NaHCO₃: sodium bicarbonate / sodium hydrogencarbonate
   - K₃PO₄: potassium phosphate
   - PS: polymer supported
   - BINAP: 2,2'-bis(diphenylphosphino)-1,1'binaphthyl
   - Dppf: 1,1'-bis(diphenylphosphino)ferrocene
   - dba: dibenzylidineacetone
   - PS-CDI: polymer supported carbonyldiimidazole
   - CH₃CN or MeCN: acetonitrile
   - h: hour
   - min: minute
   - HATU: O-(7-Azabenzotriazol-1-yl)-N,N,N',N'-tetramethyluronium hexofluorophosphate
   - NaOH: sodium hydroxide
   - AcOH: acetic acid
   - DMA: dimethyl acetamide
   - nBuLi: n-butyl lithium
   - MgSO₄: magnesium sulfate
   - Na₂SO₄: sodium sulfate
   - CDCl₃: deutero chloroform
   - CD₃OD: per-deuterated methanol
   - Boc: tert-butoxycarbonyl

All final compound names were derived using ACD NAME computer package.

The preparation of intermediates used in each Example is described at the end of the final compound Examples section.

### Example 1 : trans-2-[4-[2-[5-[(2,4,5-Trifluorophenyl)aminol]-1,3,4-oxadiazol-2-yl]-1H-benzoimidazol-5-yl]cyclohexyl]acetic acid

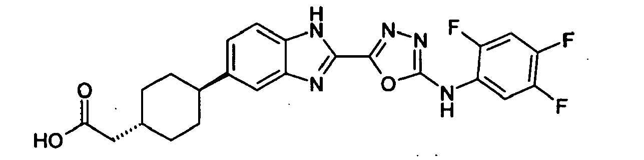

A solution of *trans*-2-[4-[2-[5-[(2,4,5-trifluorophenyl)amino]-1,3,4-oxadiazol-2-yl]-1H-benzoimidazol-5-yl]cyclohexyl]acetic acid methyl ester (Intermediate 1, 530 mg, 1.09 mmol) and lithium hydroxide monohydrate (459 mg, 11 mmol) in a mixture of THF (15 mL), water (15 mL) and MeOH (30 mL) was stirred at 35°C for 4 h. The reaction mixture was concentrated *in vacuo* to one quarter of its original volume and then a 1N aqueous solution of citric acid (80 mL) was added. The resulting precipitate was filtered, washed with water (40 mL) and then recrystallised from MeOH to give the title compound as a white solid (400 mg, 78%).
¹H NMR δ 1.11-1.20 (2H, m), 1.48-1.52 (2H, m), 1.76-1.87 (5H, m), 2.15-2.17 (2H, d), 2.53 - 2.61 (1H, m), 7.19 (0.5H, d), 7.25 (0.5H, d), 7.36 (0.5H, s), 7.46 (0.5H, d), 7.57 (0.5H, s), 7.64 (0.5H, d), 7.70-7.77 (1H, m), 8.19 - 8.27 (1H, m), 11.06 (1H, br s), 12.06 (1H, br s), 13.57 (1H, s); MS m/e MH⁺ 472.

### Example 2 : trans-2-[4-[2-[5-[(3,4-Difluorophenyl)amino]-1,3,4-oxadiazol-2-yl]-1H-benzoimidazol-5-yl]cyclohexyl]acetic acid

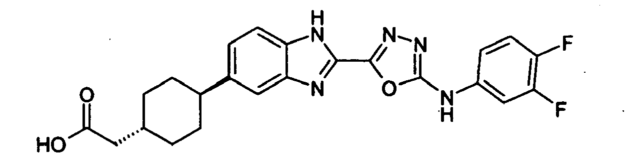

A solution of *trans*-methyl 2-[4-[2-[5-[(3,4-difluorophenyl)amino]-1,3,4-oxadiazol-2-yl]-1H-benzoimidazol-5-yl]cyclohexyl]acetate (Intermediate 2, 238mg, 0.51mmol) and lithium hydroxide monohydrate (214mg, 5.09mmol) in a mixture of THF (8mL), water (8mL) and MeOH (16mL) was stirred at 35°C for 3 h. The reaction mixture was concentrated *in vacuo* to one quarter of its original volume and then a 1N aqueous solution of citric acid (80 mL) was added. The resulting precipitate was filtered, washed with water (40 mL) and recrystallised from MeOH to give the title compound as a bright yellow solid (95 mg, 41%).
¹H NMR δ 1.12-1.21 (2H, m), 1.49 - 1.58 (2H, m), 1.75-1.88 (5H, m), 2.17 (2H, d), 2.60 (1H, m), 7.22 - 7.65 (5H, m), 7.71 - 7.77 (1H, m), 11.17-11.30 (1H, m), 11.80 - 12.20 (1H, s), 13.50 (1H, s); MS m/e MH⁺ 454.

### Example 3: {trans-4-[2-(5-{[3-(Benzyloxy)phenyl]amino}-1,3,4-oxadiazol-2-yl)-1H-benzimidazol-5-yl]cyclohexyl}acetic acid

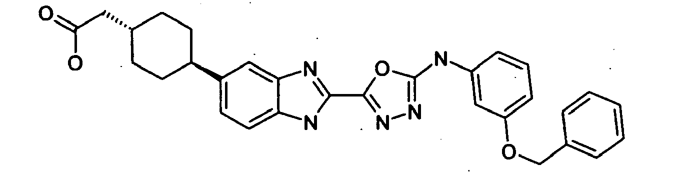

A solution of lithium hydroxide in water (10%, 2 mL) was added in one portion to a stirred suspension of methyl {*trans*-4-[2-(5-{[3-(benzyloxy)phenyl]amino}-1,3,4-oxadiazol-2-yl)-1*H*-benzimidazol-5-yl]cyclohexyl}acetate (Intermediate 3, 200 mg, 0.372 mmol) in a 1:1 mixture of THF and MeOH (10 mL) and the reaction mixture was stirred at ambient temperature for 20 h. A 1M aqueous solution of citric acid (10 mL) was added and then the mixture was filtered to leave a solid, which was washed with water (2x10 mL). The crude solid was washed with methanol to give the title compound as a pale yellow powder (30mg,15%).
¹H NMR δ 1.09-1.24 (2H, m), 1.45-1.61 (2H, m), 1.7-1.91 (5H, m), 2.17 (2H, d), 2.5-2.7 (1H, m), 5.12 (2H, d), 6.75 (1H, d), 7.16-7.68 (11H, m), 10.96 (1H, d), 12.1 (1H, s), 13.5 (1H, s); MS m/e MH⁺ 524.

### Example 4 : [trans-4-(2-{5-[(4-Cyanophenyl)amino]-1,3,4-oxadiazol-2-yl}-1H-benzimidazol-5-yl)cyclohexyl]acetic acid

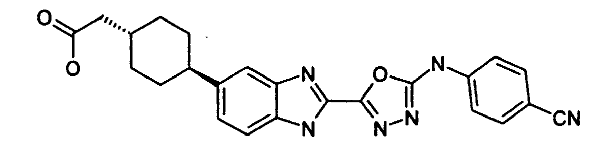

Methyl [*trans*-4-(2-{5-[(4-cyanophenyl)amino]-1,3,4-oxadiazol-2-yl}-1*H*-benzimidazol-5-yl)cyclohexyl]acetate (Intermediate 4, 220 mg, 0.48 mmol) was added in one portion to a solution of potassium trimethylsilanolate (619 mg, 4.82 mmol) in THF (10 mL) and the reaction mixture was heated in a microwave at 80°C for 10 mins. The mixture was concentrated *in vacuo* and then a 1M aqueous solution of citric acid was added. The mixture was stirred for 20 mins and then filtered to leave a solid, which was washed with water (2x10 mL) to give the title compound as a pale yellow solid (200 mg, 94%). ¹H NMR δ1.09-1.24 (2H, m), 1.45-1.61 (2H, m), 1.7-1.91 (5H, m), 2.17 (2H, d), 2.52-2.7 (1H, m), 7.06-7.64 (3H, m), 7.79 (2H, d), 7.86 (2H, d), 11.5 (1H, s), 12.0 (1H, s), 13.55 (1H, s); MS m/e MH⁺ 443.

### Examples 5-11

The following Examples were prepared in an analogous manner to Example 3 using Intermediates 5-11 as starting materials:
**Example 5: (4-{2-[5-(2-Methoxy-phenylamino)[1,3,4]oxadiazol-2-yl]-1*H-*benzimidazol-5-yl}-cyclohexyl)acetic acid.**
**Example 6: (4-{2-[5-(2,3-Dihydrobenzo[1,4]dioxin-6-ylamino)[1,3,4]oxadiazol-2-yl]-1*H*-benzimidazol-5-yl}cyclohexyl)acetic acid.**
**Example 7: (4-{2-[5-(4-Chlorophenylamino)[1,3,4]oxadiazol-2-yl]-1*H*-benzimidazol-5-yl}-cyclohexyl)acetic acid.**
**Example 8: (4-{2-[5-(3-Chlorophenylamino)[1,3,4]oxadiazol-2-yl]-1*H*-benzimidazol-5-yl}-cyclohexyl)acetic acid.**
**Example 9: (4-{2-[5-(4-Methylsulfonylphenylamino)[1,3,4]oxadiazol-2-yl]-1*H-*benzimidazol-5-yl}cyclohexyl)acetic acid.**
**Example 10: (4-{2-[5-(4-Fluorophenylamino)[1,3,4]oxadiazol-2-yl]-1*H*-benzimidazol-5-yl}cyclohexyl)acetic acid.**
**Example 11: (4-{2-[5-(3,4,5-Trifluorophenylamino)[1,3,4]oxadiazol-2-yl]-1*H-*benzimidazol-5-yl}cyclohexyl)acetic acid.**

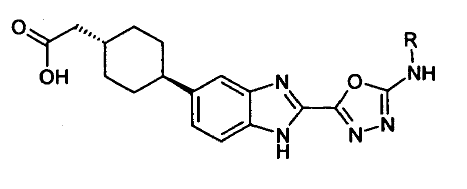

| **Ex** | **R** | **¹H NMR** | **MS m/e MH⁺** |
|---|---|---|---|
| **5** | 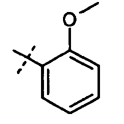 | δ 1.09-1.24 (2H, m), 1.45-1.61 (2H, m), 1.7-1.91 (5H, m), 2.17 (2H, d), 2.52-2.7 (1H, m), 3.87 (3H, s), 7.0-7.65 (6H, m), 8.0 (1H, d), 10.05 (1H, d), 12.1 (1H, s), 13.5 (1H, s). | 448 |
| **6** | 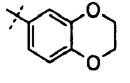 | δ 1.09-1.24 (2H, m), 1.45-1.61 (2H, m), 1.7-1.91 (5H, m), 2.17 (2H, d), 2.5-2.68 (1H, m), 4.18-4.32 (4H. m), 6.9 (1H, d), 7.03 (1H, dd), 7.15-7.68 (3H, m), 7.26 (1H, d), 10.7 (1H, s), 12.1 (1H, s), 13.5 (1H, s). | 476 |
| **7** | 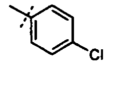 | δ1.09-1.24 (2H, m), 1.45-1.61 (2H, m), 1.7-1.91 (5H, m), 2.17 (2H, d), 2.52-2.7 (1H, m), 7.15-7.7 (3H, m), 7.47 (2H, d), 7.67 (2H, d), 11.1 (1H, d), 12.08 (1H, s), 13.55 (1H, s). | 452 |
| **8** | 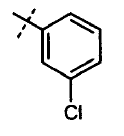 | δ1.09-1.24 (2H, m), 1.45-1.61 (2H, m), 1.7-1.91 (5H, m), 2.17 (2H, d), 2.52-2.7 (1H, m), 7.1-7.83 (7H, m), 11.2 (1H, d), 12.08 (1H, s), 13.6 (1H, s). | 452 |
| **9** | 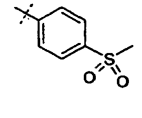 | δ1.09-1.24 (2H, m), 1.45-1.61 (2H, m), 1.7-1.91 (5H, m), 2.17 (2H, d), 2.52-2.7 (1H, m), 3.24 (3H, s), 7.2-7.75 (3H, m), 7.9 (2H, d), 8.03 (2H, d), 11.6 (1H, d), 12.2 (1H, s), 13.7 (1H, s). | 497 |
| **10** | 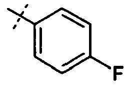 | δ1.09-1.24 (2H, m), 1.45-1.61 (2H, m), 1.7-1.91 (5H, m), 2.17 (2H, d), 2.52-2.7 (1H, m), 7.15-7.7 (7H, m), 11.0 (1H, d), 12.1 (1H, s), 13.55 (1H, s). | 436 |
| **11** | 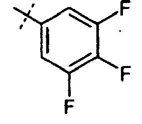 | δ1.09-1.24 (2H, m), 1.45-1.61 (2H, m), 1.7-1.91 (5H, m), 2.17 (2H, d), 2.52-2.7 (1H, m), 7.18-7.78 (5H, m), 11.6 (1H, s), 12.1 (1H, s), 13.65 (1H, s). | 473 |

### Example 12 : cis-4-[[2-(5-[(2,4,5-Trifluorophenyl)amino]-1,3,4-oxadiazol-2-yl]-3H-benzoimidazol-5-yl]oxy]cyclohexane-1-carboxylic acid

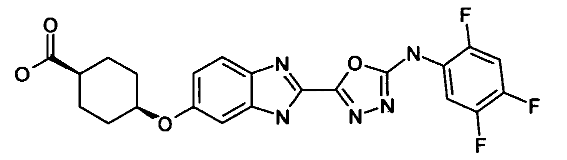

Lithium hydroxide monohydrate (83 mg, 2.0mmol) was added to a stirred solution of *cis-*ethyl 4-[[2-[5-[(2,4,5-trifluorophenyl)amino]-1,3,4-oxadiazol-2-yl]-3H-benzoimidazol-5-yl]oxy]cyclohexane-1-carboxylate (Intermediate 30, 98 mg, 0.2 mmol) in a mixture of THF (4 mL), MeOH (8 mL) and water (4 mL) and the reaction mixture was warmed to 40°C and stirred at this temperature for 3 h. The reaction mixture was concentrated *in vacuo* to one quarter of its original volume and then a 1N aqueous solution of citric acid (80 mL) was added. The resulting precipitate was filtered, washed with water (40 mL) and then recrystallised from EtOH to give the title compound as a grey solid (39 mg, 75%). ¹H NMR δ 1.66-1.87 (8H, m), 2.37 - 2.41 (1H, m), 4.57 (1H, s), 6.93 - 6.96 (0.66H, m), 7.01 (1H, m), 7.26 (0.33H, s), 7.43 (0.63H, d), 7.63 (1H, d), 7.67 - 7.74 (1H, m), 8.18-8.25 (1H, m), 10.98 (0.63H, s), 12.07 (0.69H, s), 13.40 (0.44H, s), 13.50 (0.24H, s); MS m/e MH⁺ 474.

### Example 13 : cis-4-[[2-[5-[(4-Fluorophenyl)amino]-1,3,4-oxadiazol-2-yl]-3H-benzoimidazol-5-yl]oxy]cyclohexane-1-carboxylic acid

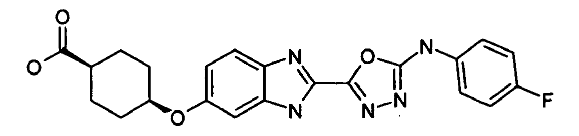

Lithium hydroxide monohydrate (44 mg, 1.0mmol) was added in one portion to a stirred solution of *cis-*ethyl 4-[[2-[5-[(4-fluorophenyl)amino]-1,3,4-oxadiazol-2-yl]-3H-benzoimidazol-5-yl]oxy]cyclohexane-1-carboxylate (Intermediate 31, 80 mg, 0.17 mmol) in a mixture of THF (2 mL), MeOH (4 mL) and water (2 mL) and the reaction mixture was stirred at 40 °C for 2 h. The reaction mixture was concentrated *in vacuo* to one quarter of its original volume and then a 1N aqueous solution of citric acid (80 mL) was added. The resulting precipitate was filtered, washed with water (40 mL) and then recrystallised from EtOH to give the title compound as a pink solid (49 mg, 66%).
¹H NMR δ 1.67 1.73 (4H, m), 1.78-1.89 (4H, m), 2.37 - 2.46 (1H, m), 4.56 (1H, s), 6.96 - 7.01 (1.7H, m), 7.22 - 7.28 (2.3H, m), 7.42 (0.2H, br s), 7.61 - 7.68 (2.6H, m), 10.90 (1H, s), 12.07 (1H, s), 13.38 (1H, s); MS m/e MH⁺ 438.

### Example 14 : trans-4-[[2-[5-[(2,4,5-Trifluorophenyl)amino]-1,3,4-oxadiazol-2-yl]-3H-benzoimidazol-5-yl]oxy]cyclohexane-1-carboxylic acid

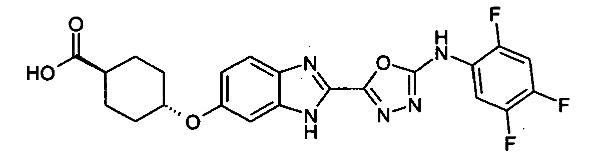

Lithium hydroxide monohydrate (63 mg, 1.5mmol) was added in one portion to a stirred solution of *trans*-ethyl 4-[[2-[5-[(2,4,5-trifluorophenyl)amino]-1,3,4-oxadiazol-2-yl]-3H-benzoimidazol-5-yl]oxy]cyclohexane-1-carboxylate (Intermediate 32, 75mg, 0.15mmol) in a mixture of THF (2mL), MeOH (4mL) and water (2mL) and the reaction mixture was stirred at 40°C for 3 h. The reaction mixture was concentrated *in vacuo* to one quarter of its original volume and then a 1N aqueous solution of citric acid (80 mL) was added. The resulting precipitate was filtered, washed with water (40 mL) and then recrystallised from EtOH to give the title compound as a white solid (64mg, 90%).
¹H NMR δ 1.41-1.56 (4H, m), 1.95-1.99 (2H, m), 2.09 - 2.13 (2H, m), 2.29 (1H, m), 4.34 (1H, m), 6.91 - 6.94 (0.67H, m), 6.96 - 7.03 (1H, m), 7.30 (0.35H, m), 7.43 (0.36H, d), 7.62 (0.63H, d), 7.67 - 7.74 (1H, m), 8.21 (1H, m), 11.0 (1H, br s), 12.10 (1H, br s), 13.45 (1H, d); MS m/e MH⁺ 474.

### Example 15 : cis-4-{4-Fluoro-2-[5-(4-fluorophenylamino)-1,3,4-oxadiazol-2-yl]-1H-benzimidazol-5-yloxy}cyclohexanecarboxylic acid

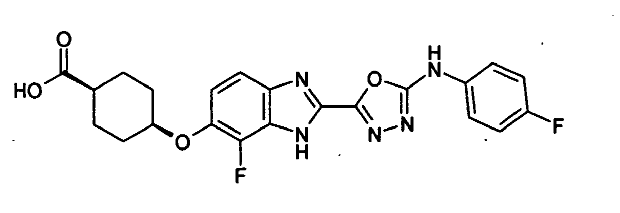

*cis-*Ethyl 4-[[4-fluoro-2-[5-[(4-fluorophenyl)amino]-1,3,4-oxadiazol-2-yl]-3H-benzoimidazol-5-yl]oxy]cyclohexane-1-carboxylate (Intermediate 33; 300 mg, 0.62 mmol) was dissolved in 1:1 THF:MeOH (10 mL), 2M NaOH (4 mL, 8.0 mmol) added and the mixture stirred at ambient temperature for 7 hr. 1M Citric acid (10 mL) was added and the resulting precipitate filtered, washed with water (20 mL) and dried in vacuo at 50 °C. The crude product was recrystallised from glacial acetic acid to give the title compound (220 mg, 78%) as a white powder.
¹H NMR: δ 1.58-1.73 (4H, m), 1.78-1.94 (4H, m), 2.3-2.42 (1H, m), 4.38-4.55 (1H, m), 7.16-7.4 (4H, m), 7.6-7.7 (2H, m), 11.0 (1H, s), 12.16 (1H, s), 13.9 (1H, s); MS m/e MH⁺ 456.

The following Examples 16-18 were prepared from intermediates 34-36 respectively in an analogous manner to Example 15.

### Example 16: trans-4-{4-Fluoro-2-[5-(4-fluorophenylamino)-1,3,4-oxadiazol-2-yl]-1H-benzimidazol-5-yloxy}cyclohexanecarboxylic acid.

### Example 17: cis-4-[[4-fluoro-2-[5-[(2,4,5-trifluorophenyl)amino]-1,3,4-oxadiazol-2-yl]-3H-benzoimidazol-5-yl]oxy]cyclohexane-1-carboxylic acid.

### Example 18: trans-4-[[4-fluoro-2-[5-[(2,4,5-trifluorophenyl)amino]-1,3,4-oxadiazol-2-yl]-3H-benzoimidazol-5-yl]oxy]cyclohexane-1-carboxylic acid.

| Example | | ¹H NMR | MS m/e MH⁺ |
|---|---|---|---|
| **16** | 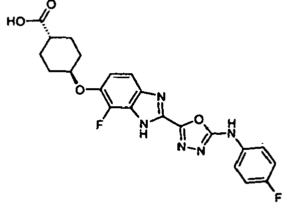 | 1.38-1.55 (4H, m), 1.86-2.12 (4H, m), 2.21-2.32 (1H, m), 4.12-4.25 (1H, m), 7.18-7.38 (4H, m), 7.6-7.7 (2H, m), 11.05 (1H, s), 12.4 (2H, s). | 456 |
| **17** | 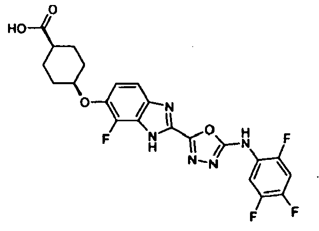 | 1.6-1.73 (4H, m), 1.78-1.9 (4H, m), 2.31-2.42 (1H, m), 4.43-4.52 (1H, m), 7.2-7.4 (2H, m), 7.68-7.79 (1H, m), 8.17-8.29 (1H, m), 11.2 (1H, s), 12.1 (1H, s), 13.8 (1H, s). | 492 |
| **18** | 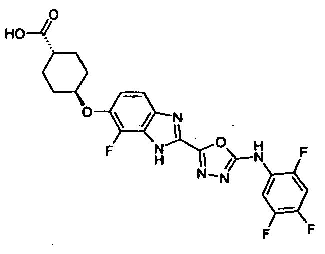 | 1.42-1.6 (4H, m), 1.94-2.18 (4H, m), 2.29-2.4 (1H, m), 4.2-4.31 (1H, m), 7.26-7.44 (2H, m), 7.73-7.84. (1H, m), 8.22-8.35 (1H, m), 11.3 (1H, s), 12.2 (2H, s). | 492 |

### Example 19 : trans-3-[4-[2-[5-[(4-fluorophenyl)amino]-1,3,4-oxadiazol-2-yl]-1H-benzoimidazol-5-yl]cyclohexyl]oxyprovanoic acid

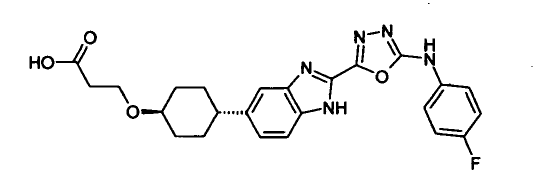

Methyl *trans*-3-[4-[2-[5-[(4-fluorophenyl)amino]-1,3,4-oxadiazol-2-yl]-1H-benzoimidazol-5-yl]cyclohexyl]oxypropanoate (Intermediate 37, 240 mg, 0.50 mmol) was stirred in a mixture of MeOH (4 mL) and a 2M aqueous solution of NaOH (2 mL) at ambient temperature for 6 h. The reaction mixture was cooled in an ice bath and acidified by the addition of 2M HCl and the resulting precipitate was filtered, washed with water (10 mL) followed by ether (10 mL) and dried under high vacuum to leave a solid. The solid was recrystallised from refluxing glacial acetic acid to give the title compound as a cream coloured solid (53 mg, 23%).
¹H NMR (500.133 MHz) δ1.29 - 1.40 (m, 2H), 1.51 - 1.62 (m, 2H), 1.89 - 1.96 (m, 2H), 2.07 - 2.14 (m, 2H), 2.44 (t, 2H), 2.62-2.69 (m, 1H), 3.32 - 3.41 (m, 1H), 3.70 (t, 2H), 7.15 - 7.22 (m, 3H), 7.46 (s, 1H), 7.55 (d, 1H), 7.60 - 7.66 (m, 2H); MS m/e MH⁺ 466.

### Example 20 : trans-2-[4-[2-[5-[(4-fluorophenyl)amino]-1,3,4-oxadiazol-2-yl]-1H-benzoimidazol-5-yl]cyclohexyl]oxyacetic acid

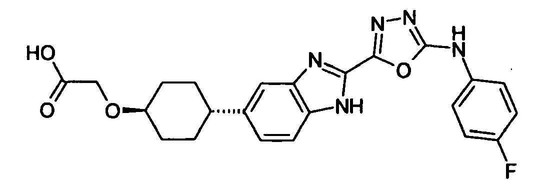

Prepared from intermediate 38 in an analogous manner to Example 19.
¹H NMR (499.803 MHz) δ1.32-1.45 (m, 2H), 1.48 - 1.62 (m, 2H), 1.88 - 1.98 (m, 2H), 2.08 - 2.19 (m, 2H), 2.60 - 2.71 (m, 1H), 3.39 - 3.51 (m, 1H), 4.05 (s, 2H), 7.14 - 7.24 (m, 3H), 7.55 (d, 1H), 7.59 - 7.68 (m, 2H), 10.51 (s, 1H); MS m/e MH⁺ 452.

### Preparation of intermediates

### Intermediate 1 : trans-2-[4-[2-[5-[(2,4,5-Trifluorophenyl)amino]-1,3,4-oxadiazol-2-yl]-1H-benzoimidazol-5-yl]cyclohexyl]acetic acid methyl ester

### i) Methyl 2-[4-(4-hydroxyphenyl)cyclohexylidene]acetate

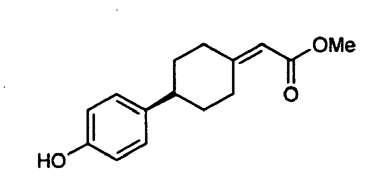

Trimethyl phosphonoacetate (170 mL, 1.05 mol) was added dropwise to a stirred suspension of sodium hydride (60 % in mineral oil, 27.5 g, 1.14 mol) in THF (3.5 L) cooled to 12°C. After completion of addition, the reaction mixture was allowed to warm to ambient temperature and stirred for 1 h. In a separate vessel, N,N-tetramethyl guanidine (144 mL, 1.14 mol) was added to a suspension of 4-(4-hydroxyphenyl)cyclohexan-1-one (235 g, 0.95 mol) in THF (1.2 L) and the reaction mixture was stirred for 1 h at ambient temperature. The phosphonoacetate mixture was cooled to 10°C and the guanidine solution added slowly, controlling the temperature between 8 and 12°C until no residual exotherm was observed. The temperature was allowed to rise to ambient temperature and the reaction mixture was stirred for 16 h. The mixture was partitioned between a dilute aqueous solution of ammonium chloride (2.4 L) and ethyl acetate (2.4 L). The aqueous phase was separated and extracted with ethyl acetate (1.2 L). The organic phases were combined and washed with brine (2.4 L), dried (MgSO₄) and concentrated *in vacuo* to leave an off white solid. The solid was slurried in a mixture of ether and hexane (2:1; 470 mL), filtered and washed with a mixture of ether and isohexane (2:1; 240 mL) to give the product as a white solid (285 g, 94%). M.Pt. 151-152°C.

### ii) trans-Methyl 2-[4-(4-hydroxyphenyl)cyclohexyl]acetate

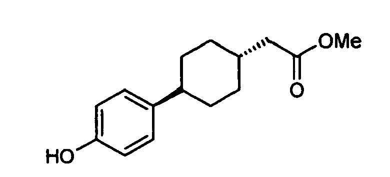

10% Palladium on carbon (50% water wet, 6.9 mmol) was added to methyl 2-[4-(4-hydroxyphenyl)cyclohexylidene]acetate (100 g, 0.41 mol) in dry THF (400 mL). The reaction mixture was heated at 30°C under a hydrogen atmosphere (2 bar). The mixture was filtered over Celite to leave a solid, which was washed with THF (50 mL). The THF solution was concentrated *in vacuo* to leave a residue, which was washed with ethyl acetate. The crude mixture was dissolved in hot ethyl acetate (100 mL) and then cooled to ambient temperature. After chilling with ice water, the precipitate was filtered and washed with ethyl acetate (50 mL) to give the title compound as a solid (42 g, 42%). M.Pt. 116.6 - 117.0°C

### iii) trans-Methyl 2-[4-(4-aminophenyl)cyclohexyl]acetate

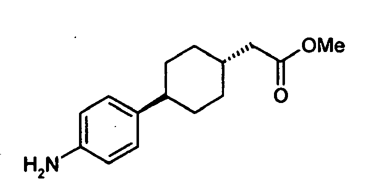

A solution of *trans-*methyl 2-[4-(4-hydroxyphenyl)cyclohexyl]acetate (2.82 g, 11.4 mmol) and diisopropylethylamine (2.32 mL, 13.3 mmol) in DCM (40 mL) was cooled to 4 °C and trifluoromethanesulfonyl chloride (1.42 mL, 13.3 mmol) was added over 30 mins, maintaining the temperature below 6°C. The reaction mixture was stirred at 4°C for 45 mins and then warmed to 15°C. Stirring was stopped and the reaction mixture was left for 16 h. The mixture was poured into ice water (18 mL), the layers separated and the aqueous layer extracted with DCM (7 mL). The combined organic phases were washed with a 2N aqueous solution of sodium hydroxide (2 mL) and then brine (9 mL), dried (MgSO₄) and concentrated *in vacuo* to leave the intermediate triflate as a yellow solid (4.59 g, 106%), which was used with no further purification.
The intermediate triflate (12 g, 32 mmol) was added to a mixture of cesium carbonate (14.4 g, 44 mmol), palladium acetate (0.43 g, 1.9 mmol), BINAP (1.2 g, 1.9 mmol), and benzophenone imine (7.9 mL, 47 mmol) in THF (200 mL). Stirring was started and the vessel was evacuated and purged with nitrogen 5 times. The stirred mixture was heated to reflux for 16 h. The reaction mixture was cooled to ambient temperature and concentrated *in vacuo* to leave a residue. The residue was partitioned between ether (360 mL) and water (210 mL) and the layers were separated. The aqueous layer was extracted with ether (3 x 360 mL) and the combined organic layers were dried (MgSO₄) and concentrated *in vacuo* to leave a crude yellow oil which was used with no further purification.
The crude imine (21 g, 51 mmol) was dissolved in methanol (300 mL) and the solution cooled to 4°C. AIM solution of hydrochloric acid (100 mL) was added slowly, maintaining the temperature below 7°C. The suspension was warmed to ambient temperature over 16 h. The methanol was removed *in vacuo* and the resulting mixture diluted with water (100 mL). The aqueous mixture was washed with ether (2 x 30 mL) and the combined organic layer washed with a 1 M solution of hydrochloric acid (2 x 30 mL). The combined aqueous layers were basified to pH9 with a 10% aqueous solution of sodium carbonate to give a precipitate. Ethyl acetate (3 x 200 mL) was added and the layers were separated. The combined organic layers were dried (MgSO₄) and concentrated *in vacuo* until a precipitate formed. The mixture was cooled, filtered and washed with hexane (20 mL) to give the product as a pale yellow solid. The filtrates were concentrated *in vacuo* to give additional product, which were combined, concentrated *in vacuo* and purified by column chromatography, using a gradient of 10 → 50 % EtOAc and isohexane as eluent to give the product as a yellow solid (5.1 g, combined yield 65% over 2 steps). M.Pt. 117 - 118°C

### iv) Methyl ({4-[trans-4-(2-methoxy-2-oxoethyl)cyclohexyl]phenyl}aminol(oxo)-acetate

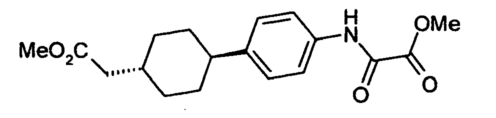

Methyl chloro(oxo)acetate (0.842 mL) was added to a stirred solution of *trans-*methyl 2-[4-(4-aminophenyl)cyclohexyl]acetate (1740 mg) and pyridine (0.689 mL) in DCM (50 mL) at 0°C. After the addition was complete the mixture was allowed to warm to ambient temperature and stirred for 64 hours. The solution was diluted with DCM (100 mL), washed with water (50 mL) and brine (50 mL), then dried and concentrated *in vacuo* to give the title compound (2267 mg) as a solid.
¹H NMR δ 7.60 (2H, d), 7.18 (2H, d), 3.83 (3H, s), 3.58 (3H, s), 2.58-35 (1H+DMSO, m), 2.21 (2H, d), 1.75 (5H, m), 1.43 (2H, m), 1.12 (2H, m); MS m/e (M-H)⁻ 332.

### v) trans-Methyl 2-[4-[4-1(methoxycarbonylformyl)amino]-3-nitro-phenyl]cyclohexyl]-acetate

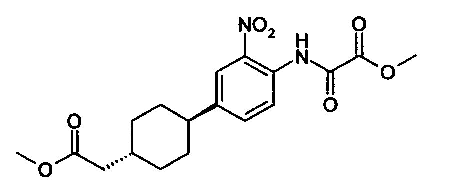

Trifluoroacetic anhydride (84 µL, 0.6 mmol) was added in one portion to a stirred suspension of ammonium nitrate (49 mg, 0.6 mmol) in DCM (3 mL) at 0°C under an argon atmosphere. The reaction mixture was stirred at 0°C for 1 min and then a solution of *trans*-methyl 2-[4-[4-[(methoxycarbonylformyl)amino]phenyl]cydo-hexyl]acetate (200 mg, 0.6 mmol) in DCM (1 mL) was added. The reaction mixture was stirred at 0°C for 30 mins and then warmed to ambient temperature and stirred for 3 h. A second portion of trifluoroacetic anhydride (28 µL, 0.2 mmol) was added and the reaction mixture was stirred for 16 h at ambient temperature. The reaction mixture was concentrated *in vacuo* to half its original volume and then a saturated aqueous solution of sodium hydrogen carbonate (30 mL) and EtOAc (20 mL) were added. The layers were separated and the organic layer was washed with brine (10 mL), dried (MgSO₄) and concentrated *in vacuo* to leave a yellow oil. The oil was purified by column chromatography, eluting with a gradient of 30 to 60% EtOAc in isohexane, to give the title compound as a yellow oil which crystallised on standing (92 mg, 41 %).
¹H NMR δ 1.13 - 1.22 (2H, m), 1.51 (2H, q), 1.92 - 1.95 (5H, m), 2.27 (2H, d), 2.54 - 2.60 (1H, m), 3.69 (3H, s), 4.02 (3H, s), 7.56 - 7.58 (1H, m), 8.11 (1H, d), 8.71 (1H, d), 11.80 (1H, s); MS m/e (M-H)⁻ 377.

### vi) Methyl [trans-4-(4-{[hydrazino(oxo)acetyl]amino}-3-nitrophenyl)cvclohexyl] acetate

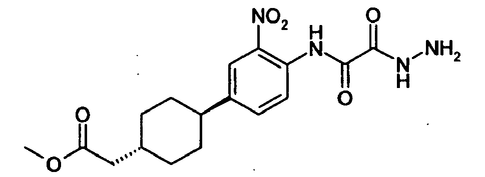

Hydrazine monohydrate (13 µL, 0.26 mmol) was added in one portion to a stirred solution of *trans-*methyl 2-[4-[4-[(methoxycarbonylformyl)amino]-3-nitro-phenyl]cyclohexyl]-acetate (81 mg, 0.2 mmol) in EtOH (4 mL) and the reaction mixture was stirred at ambient temperature for 2 h. The reaction mixture was concentrated *in vacuo* to leave a residue that was triturated with ether (15 mL) and then filtered to leave the title compound as a yellow solid (76 mg, 94%).
¹H NMR δ 1.09 - 1.18 (2H, m), 1.46 - 1.54 (2H, m), 1.75 - 1.83 (5H, m), 2.25 - 2.26 (2H, d), 2.57 - 2.63 (1H, m), 3.60 (3H, s), 4.72 (2H, s), 7.69 - 7.72 (1H, m), 7.97 (1H, d), 8.13 (1H, d), 10.52 (1H, s), 11.41 (1H, s); MS m/e (M-H)⁻ 377.

### vii) trans-Methyl 2-[4-[3-nitro-4-[5-[(2,4,5-trifluorophenyl)amino]1,3,4-oxadiazole-2-carbonyl]amino]phenyl]cyclohexyl]acetate

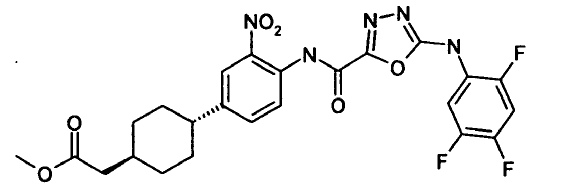

2,4,5-Trifluorophenylisothiocyanate (515 mg, 2.7 mmol) was added in one portion to a stirred solution of methyl [*trans-*4-(4-{[hydrazino(oxo)acetyl]amino}-3-nitrophenyl)cyclohexyl]-acetate (1.0 g, 2.7 mmol) in DMA (50 mL). The reaction mixture was stirred at ambient temperature for 30 mins and then EDAC (522 mg, 2.7 mmol) was added in one portion. The reaction mixture was warmed to 80°C and stirred for 30 mins. The mixture was concentrated *in vacuo* to leave a bright yellow solid, which was triturated with hot acetonitrile. The mixture was cooled to ambient temperature and then filtered to leave the title compound as a bright yellow solid (1.18 g, 81%).
¹H NMR δ 1.10 - 1.18 (2H, m), 1.46 - 1.56 (2H, m), 1.76 - 1.85 (5H, m), 2.25 - 2.27 (2H, d), 2.58 - 2.65 (1H, m), 3.61 (3 H, s), 7.69 - 7.77 (2H, m), 7.90 (1H, d), 7.94 - 7.95 (1H, m), 8.14 - 8.21 (1H, m), 11.19 (1H, s), 11.46 (1H, s); MS m/e MH⁺ 534.

### viii) trans-Methyl 2-[4-[3-amino-4-[[5-[(2,4,5-trifluorophenyl)amino]1,3,4-oxadiazole-2-carbonyl]amino]phenyl]cyclohexyl]acetate

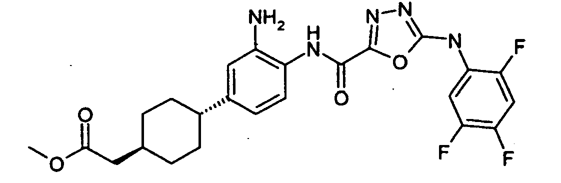

A stirred suspension of *trans-*methyl 2-[4-[3-nitro-4-[[5-[(2,4,5-trifluorophenyl)-amino]1,3,4-oxadiazole-2-carbonyl]amino]phenyl]cyclohexyl]acetate (1.14 g, 2.14 mmol) and 10% palladium on carbon (100 mg) in a mixture of THF (30 mL), MeOH (30 mL) and dioxane (30 mL) was stirred under a hydrogen atmosphere for 2 h. The mixture was filtered and the solvent was concentrated *in vacuo* to give the title compound as a yellow solid (1.13 g, 100%).
¹H NMR δ 1.05 - 1.20 (2H, m), 1.35 - 1.50 (2H, m), 1.70 - 1.85 (5H, m), 2.20 - 2.26 (2H, d), 2.28 - 2.40 (1H, m), 3.61 (3H, s), 4.90 - 5.05 (2H, br s), 6.45 - 6.50 (1H, d), 6.65 (1H, s), 7.00 - 7.05 (1H, d), 7.18 - 7.60 (1H, m), 8.15 - 8.25 (1H, m),10.22 (1H, s), 11.00 (1H, s); MS m/e MH⁺ 504.

### ix) trans-2-[4-[2-[5-[(2,4,5-Trifluorophenyl)aminol-1,3,4-oxadiazol-2-yl]-1H-benzoimidazol-5-yl]cyclohexyl]acetic acid methyl ester

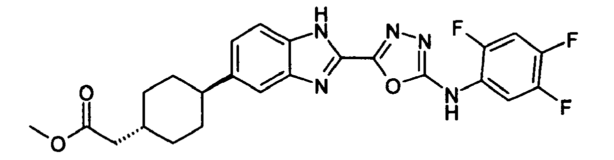

A solution of *trans-*methyl 2-[4-(3-amino-4-[[5-[(2,4,5-trifluorophenyl)amino]1,3,4-oxadiazole-2-carbonyl]amino]phenyl]cyclohexyl]acetate (1.01 g, 2.08 mmol) and acetic acid (3.2 mL) in acetonitrile (50 mL) was heated in a microwave at 150°C for 15 mins. The reaction mixture was cooled to ambient temperature and the resulting mixture was filtered and washed with acetonitrile (15 mL) to leave a solid. The solid was triturated with a hot mixture of MeOH and EtOH (200 mL), cooled to ambient temperature and filtered to leave the title compound (Intermediate 1) as a tan solid (640 mg, 67%).
¹H NMR δ 1.12 - 1.21 (2H, m), 1.48 - 1.55 (2H, m), 1.84 (5H, m), 2.26 - 2.27 (2H, d), 2.60 (1H, m), 3.60 - 3.61 (3H, m), 7.17 - 7.20 (0.5H, m), 7.25 (0.5H, d), 7.36 (0.5H, s), 7.46 (0.5H, d), 7.57 (0.5H, s), 7.64 (0.5H, d), 7.70 - 7.77 (1H, m), 8.20 - 8.27 (1H, m), 11.07 (1H, s), 13.58 (1H, s); MS m/e MH⁺ 486.

### Intermediate 2 : trans-Methyl2-[4-[2-[5-[(3,4-difluorophenyl)aminol-1,3,4-oxadiazol-2-yl-]1H-benzoimidazol-5-yl]cyclohexyl]acetate

### i) trans-Methyl 2-[4-[3-nitro-4-[[5-[(3,4-difluorophenyl)amino]1,3,4-oxadiazole-2-carbonyl]amino]phenyl]cyclohexyl]acetate

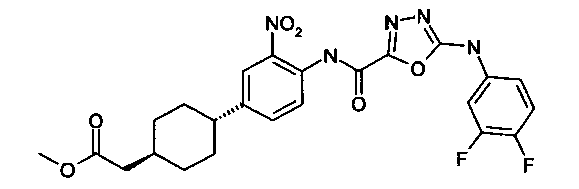

3,4-Difluorophenylisothiocyanate (161 µL, 1.27 mmol) was added in one portion to a stirred solution of methyl [*trans-*4-(4-{[hydrazino(oxo)acetyl]amino}-3-nitrophenyl)cyclohexyl]-acetate (Intermediate 1vi, 480 mg, 1.27 mmol) in DMA (25mL) at ambient temperature. The reaction mixture was stirred at ambient temperature for 20 mins and then EDAC (268 mg, 1.4 mmol) was added in one portion. The reaction was warmed to 80°C and stirred for 20 mins. The reaction mixture was concentrated *in vacuo* and the resulting residue was triturated with hot water (60 mL), cooled and filtered to leave the title compound as a yellow solid (660 mg, 100%).
¹HNMR δ 1.14- 1.21 (2H, m), 1.48 - 1.56 (2H, m), 1.76 - 1.87 (5H, m), 2.26 - 2.27 (2H, d), 2.60 - 2.68 (1H, m), 3.61 (3H, s), 7.35 - 7.38 (1H, m), 7.45 - 7.53 (1H, m), 7.67 - 7.73 (2H, m), 7.95 - 7.97 (2H, m), 11.29 (1H, br s), 11.39 (1H, s); MS m/e MH⁺ 516.

### ii) trans-Methyl 2-[4-[3-amino-4-[[5-[(3,4-difluorophenyl)amino]1,3,4-oxadiazole-2-carbonyl]amino]phenyl]cyclohexyl]acetate

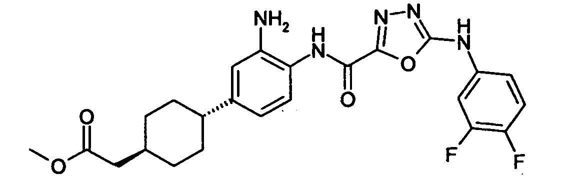

A suspension of *trans-*methyl 2-[4-[3-nitro-4-[[5-[(3,4-difluorophenyl)amino]1,3,4-oxadiazole-2-carbonyl]amino]phenyl]cyclohexyl]acetate (660 mg, 1.28 mmol) and 10% palladium on carbon (75 mg) in a mixture of MeOH (30 mL), THF (30 mL) and dioxane (30 mL) was stirred under a hydrogen atmosphere for 5 h. The reaction mixture was filtered and concentrated *in vacuo* to leave the title compound as a bright yellow solid (620 mg, 100%); MS m/e MH⁺ 486.

### iii) trans-Methyl 2-[4-[2-[5-[(3,4-difluorophenyl)aminol]-1,3,4-oxadiazol-2-yl]-1H-benzoimidazol-5-yl]cyclohexyl]acetate

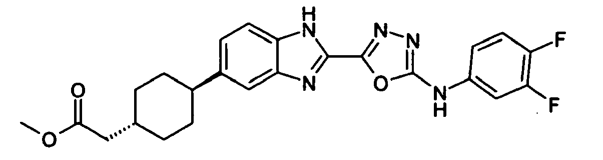

Acetic acid (2 mL) was added in one portion to a suspension of *trans*-methyl 2-[4-[3-amino-4-[[5-[(3,4-difluorophenyl)amino] 1,3,4-oxadiazole-2-carbonyl]amino]phenyl]cyclohexyl]-acetate (595 mg, 1.23 mmol) in acetonitrile (20 mL) and the mixture was heated in a microwave at 150°C for 15 mins. The reaction mixture was cooled to ambient temperature and filtered to leave a solid, which was washed with acetonitrile (15 mL) to leave the title compound (Intermediate 2) as a yellow solid (258 mg, 45%).
¹H NMR δ 1.13 - 1.22 (2H, m), 1.47 - 1.55 (2H, m), 1.82 - 1.92 (5H, m), 2.27 (2H, d), 2.53 - 2.62 (1H, m), 3.59 - 3.62 (3H, s), 7.18 - 7.65 (5H, m), 7.71 - 7.77 (1H, m), 11.15 - 11.68 (1H, m), 13.51 (1H, s); MS m/e MH⁺ 468.

### Intermediate 3: Methyl (trans-4-[2-(5-{[3-(benzyloxy)phenyl]amino}-1,3,4-oxadiazol-2-yl)-1H-benzimidazol-5-yl]cyclohexyl}acetate

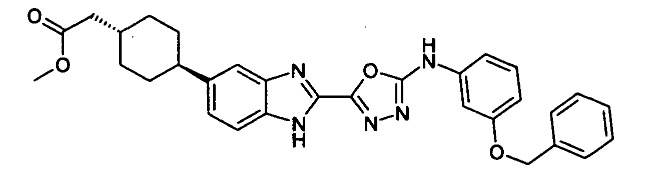

A suspension of methyl [*trans-*4-(3-amino-4-{[(5-{[3-(benzyloxy)phenyl]amino}-1,3,4-oxadiazol-2-yl)carbonyl]amino}phenyl)cyclohexyl]acetate (Intermediate 12, 400 mg, 0.72 mmol) in a 1:2 mixture of acetonitrile and glacial acetic acid (15 mL) was heated at 120°C in a microwave for 15 mins. The reaction mixture was cooled to ambient temperature, filtered to leave a solid and then washed with acetonitrile to give the title compound (200 mg, 50%) as a beige solid; MS m/e MH⁺ 538.

### Intermediates 4-11

The following intermediates were prepared in an analogous manner to Intermediate 3 using Intermediates 13-20 as starting material.

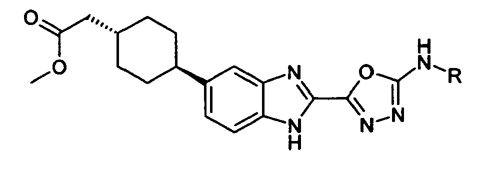

| **Int** | **R** | **MS m/e MH⁺** |
|---|---|---|
| **4** | 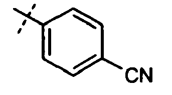 | 457 |
| **5** | 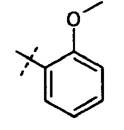 | 462 |
| **6** | 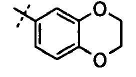 | 490 |
| **7** | 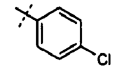 | 466 |
| **8** | 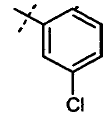 | 466 |
| **9** | 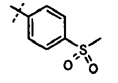 | 510 |
| **10** | 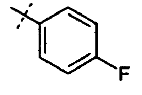 | 450 |
| **11** | 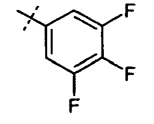 | 486 |

### Intermediate 12: Methyl [trans-4-(3-amino-4-{[(5-{[3-(benzyloxy)phenyl]amino}-1.3.4-oxadiazol-2-yl)carbonyl]amino}phenyl)cyclohexyl]acetate

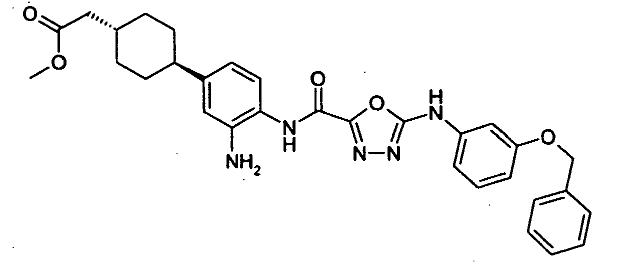

10% Platinum on carbon (100 mg) was added in one portion to a stirred solution of methyl [*trans-*4-(4-{[(5-{[3-(benzyloxy)phenyl]amino}-1,3,4-oxadiazol-2-yl)carbonyl]amino }-3-nitrophenyl)cyclohexyl]acetate (Intermediate 21, 470 mg, 0.80 mmol) in THF (50 mL) at ambient temperature and the reaction mixture was stirred under a hydrogen atmosphere. The reaction mixture was filtered and concentrated *in vacuo* to leave the title compound (400 mg, 90%) as a beige foam.
¹H NMR δ 1.1-1.25 (2H, m), 1.4-1.57 (2H, m), 1.74-1.92 (5H, m), 2.3 (2H, d), 2.34-2.45 (1H, m), 3.66 (3H, s), 4.95 (2H, s), 5.27 (2H, s), 6.52 (1H, dd), 6.7 (1H, d), 6.3 (1H, dd), 7.1-7.58 (9H, m), 10.15 (1H, s), 11.0 (1H, s); MS m/e MH⁺ 556.

### Intermediates 13-20

The following intermediates were prepared in an analogous manner to Intermediate 12 using Intermediates 22-29 as starting material.

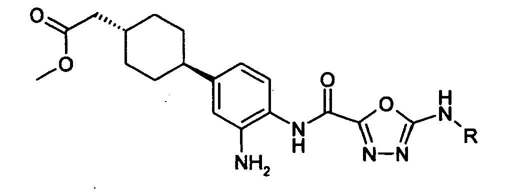

| **Int** | **R** | **¹H NMR** | **MS m/e MH⁺** |
|---|---|---|---|
| **13** | 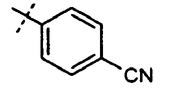 | δ1.1-1.25 (2H, m), 1.4-1.57 (2H, m), 1.74-1.92 (5H, m), 2.3 (2H, d), 2.34-2.45 (1H, m), 3.66 (3H, s), 4.95 (2H, s), 6.55 (1H, dd), 6.7 (1H, d), 7.12 (1H, dd), 7.82 (2H, d), 7.94 (2H, d), 10.22 (1H, s), 11.6 (1H, s). | 475 |
| **14*** | 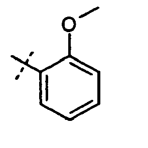 | δ1.1-1.25 (2H, m), 1.4-1.57 (2H, m), 1.74-1.92 (5H, m), 2.3 (2H, d), 2.34-2.45 (1H, m), 3.66 (3H, s), 3.9 (3H, s), 4.95 (2H, s), 6.55 (1H, dd), 6.7 (1H, d), 7.02-7.2 (4H, m), 8.0 (1H, d), 10.05 (1H, s), 10.1 (1H, s). | 480 |
| **15*** | 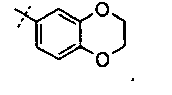 | δ1.1-1.25 (2H, m), 1.4-1.57 (2H, m), 1.74-1.92 (5H, m), 2.3 (2H, d), 2.34-2.45 (1H, m), 3.66 (3H, s), 4.2-4.37 (4H, m), 4.95 (2H, s), 6.54 (1H, dd), 6.7 (1H, d), 6.92 (1H, s), 7.05 (1H, dd), 7.13 (1H, d), 7.28 (1H,d), 10.1 (1H, s), 10.75 (1H, s). | 508 |
| **16** | 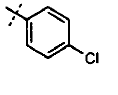 | δ1.1-1.25 (2H, m), 1.4-1.57 (2H, m), 1.74-1.92 (5H, m), 2.3 (2H, d), 2.34-2.45 (1H, m), 3.66 (3H, s), 4.95 (2H, s), 6.53 (1H, dd), 6.7 (1H, d), 7.12 (1H, d), 7.52 (2H, d), 7.7 (2H, d), 10.2 (1H, s), 11.1 (1H, s). | 484 |
| **17** | 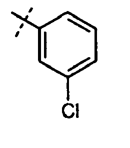 | δ1.1-1.25 (2H, m), 1.4-1.57 (2H, m), 1.74-1.92 (5H, m), 2.3 (2H, d), 2.34-2.45 (1H, m), 3.66 (3H, s), 4.95 (2H, s), 6.53 (1H, dd), 6.7 (1H, d), 7.1 (1H, d), 7.19 (1H, dd), 7.48 (1H, dd), 7.58 (1H, dd), 7.8 (1H, dd), 10.18 (1H, s), 11.2 (1H, s). | 484 |
| **18*** | 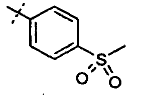 | δ1.1-1.25 (2H, m), 1.4-1.57 (2H, m), 1.74-1.92 (5H, m), 2.3 (2H, d), 2.34-2.45 (1H, m), 3.24 (3H, s), 3.66 (3H, s), 4.95 (2H, s), 6.53 (1H, dd), 6.7 (1H, d), 7.11 (1H, d), 7.88 (2H, d), 8.01 (2H, d), 10.2 (1H, s), 11.0 (1H, s). | 528 |
| **19*** | 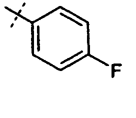 | δ1.1-1.25 (2H, m), 1.4-1.57 (2H, m), 1.74-1.92 (5H, m), 2.3 (2H, d), 2.34-2.45 (1H, m), 3.66 (3H, s), 4.95 (2H, s), 6.53 (1H, dd), 6.7 (1H, d), 7.12 (1H, d), 7.22-7.33 (2H, m), 7.63-7.7 (2H, m), 10.14 (1H, s), 11.0 (1H, s). | 468 |
| **20*** | 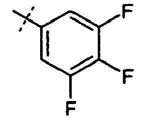 | δ1.1-1.25 (2H, m), 1.4-1.57 (2H, m), 1.74-1.92 (5H, m), 2.3 (2H, d), 2.34-2.45 (1H, m), 3.66 (3H, s), 4.95 (2H, s), 6.52 (1H, dd), 6.7 (1H, d), 7.1 (1H, d), 7.46-7.59 (2H, m), 10.2 (1H, s), 11.45 (1H, s). | 504 |

| | | | |
|---|---|---|---|
| * Intermediates 14, 15, 18-20 were synthesised using 10% palladium on carbon instead of 10% platinum on carbon. | | | |

### Intermediate 21 : Methyl[trans-4-(4-{[(5-{[3-(benzyloxy)phenyl]amino}-1,3,4-oxadiazol-2-yl)carbonyl]amino}-3-nitrophenyl)cyclohexyl]acetate

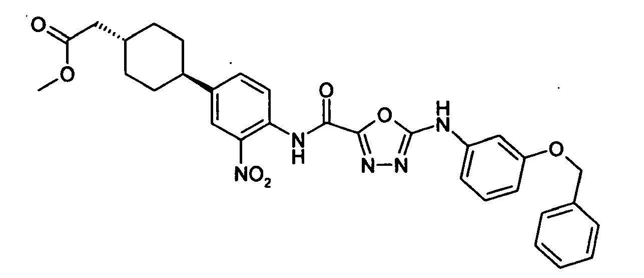

1-(Benzyloxy)-3-isothiocyanatobenzene (290 mg, 1.2 mmol) was added in one portion to a stirred solution of methyl [*trans-*4-(4-{[hydrazino(oxo)acetyl]amino}-3-nitrophenyl)cyclohexyl]acetate (Intermediate 1vi, 378 mg, 1.0 mmol) in DMA (5 mL) and the reaction mixture was stirred at ambient temperature for 4 h. EDCI (288 mg, 1.5 mmol) was added in one portion and the reaction mixture was heated in a microwave at 80°C for 10 mins. Water (15 mL) was added and the mixture was filtered to leave a solid, which was washed with water to give the title compound as a solid (470mg, 80%).
¹HNMR δ1.08-1.21 (2H, m), 1.46-1.6 (2H, m), 1.73-1.9 (5H, m), 2.27 (2H, d), 2.6-2.7 (1H, m), 3.61 (3H, s), 5.12 (2H, s), 6.76 (1H, dd), 7.17 (1H, dd), 7.25-7.5 (7H, m), 7.7 (1H, dd), 7.92-8.05 (2H, m), 11.05 (1H, s), 11.4 (1H, s); MS m/e MW 586.

### Intermediates 22-29

The following intermediates were prepared in an analogous manner to Intermediate 21 using Intermediate 1vi and the required isothiocyanate as starting material.

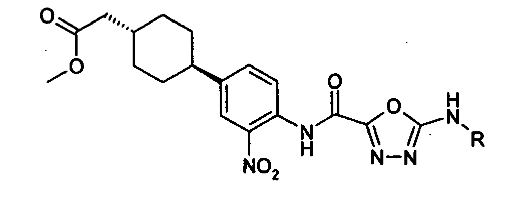

| **Int** | **R** | **¹NMR** | **MS m/e** **MH⁺** |
|---|---|---|---|
| **22** | 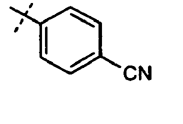 | δ1.08-1.21 (2H, m), 1.46-1.6 (2H, m), 1.73-1.9 (5H, m), 2.27 (2H, d), 2.6-2.7 (1H, m), 3.61 (3H, s), 7.7 (1H, dd), 7.78 (2H, d), 7.88 (2H, d), 7.91-8.0 (2H, m), 11.4 (1H, s), 11.6 (1H, s). | 505 |
| **23** | 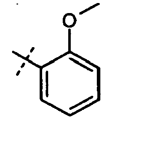 | δ1.08-1.21 (2H, m), 1.46-1.6 (2H, m), 1.73-1.9 (5H, m), 2.27 (2H, d), 2.6-2.7 (1H, m), 3.61 (3H, s), 3.85 (3H, s), 7.03 (1H, ddd), 7.19-7.2 (2H, m), 7.7 (1H, dd), 7.91 (1H, d), 7.95-8.05 (2H, m), 10.2 (1H, s), 11.45 (1H, s). | 510 |
| **24** | 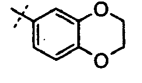 | δ1.08-1.21 (2H, m), 1.46-1.6 (2H, m), 1.73-1.9 (5H, m), 2.27 (2H, d), 2.6-2.7 (1H, m), 3.61 (3H, s), 4.15-4.3 (4H, m), 6.8 (1H, d), 7.0 (1H, dd), 7.2 (1H, d), 7.7 (1H, dd), 7.92-8.05 (2H, m), 10.8 (1H, s), 11.36 (1H, s). | 538 |
| **25** | 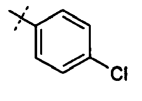 | δ1.08-1.21 (2H, m), 1.46-1.6 (2H, m), 1.73-1.9 (5H, m), 2.27 (2H, d), 2.6-2.7 (1H, m), 3.61 (3H, s), 7.47 (2H, d), 7.63 (2H, d), 7.72 (1H, dd), 7.91-8.05 (2H, m), 11.2 (1H, s), 11.4 (1H, s). | 514 |
| **26** | 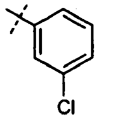 | δ1.08-1.21 (2H, m), 1.46-1.6 (2H, m), 1.73-1.9 (5H, m), 2.27 (2H, d), 2.6-2.7 (1H, m), 3.61 (3H, s), 7.14 (1H, dd), 7.43 (1H, dd), 7.52 (1H, dd), 7.71 (1H, dd), 7.75 (1H, dd), 7.92-8.05 (2H, m), 11.3 (1H, s), 11.4 (1H, s). | 514 |
| **27** | 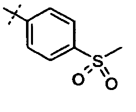 | δ1.08-1.21 (2H, m), 1.46-1.6 (2H, m), 1.73-1.9 (5H, m), 2.27 (2H, d), 2.6-2.7 (1H, m), 3.2 (3H, s), 3.61 (3H, s), 7.71 (1H, dd), 7.82 (2H, d), 7.93-8.05 (2H, m), 7.97 (2H, d), 11.4 (1H, s), 11.6 (1H, s). | 558 |
| **28** | 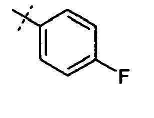 | δ1.08-1.21 (2H, m), 1.46-1.6 (2H, m), 1.73-1.9 (5H, m), 2.27 (2H, d), 2.6-2.7 (1H, m), 3.61 (3H, s), 7.23-7.31 (2H, m), 7.59-7.65 (2H, m), 7.7 (1H, dd), 7.93-8.05 (2H, m), 11.1 (1H, s), 11.4 (1H, s). | 498 |
| **29** | 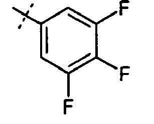 | δ1.08-1.21 (2H, m), 1.46-1.6 (2H, m), 1.73-1.9 (5H, m), 2.27 (2H, d), 2.6-2.7 (1H, m), 3.61 (3H, s), 7.42-7.54 (2H, m), 7.71 (1H, dd), 7.91-8.02 (2H, m), 11.0 (1H, s), 11.4 (1H, s). | 534 |

| | | | |
|---|---|---|---|
| All isothiocyanates are commercially available except for that required for Intermediate 27 which can be prepared as described in Bioorg Med Chem Lett 2006, 14, 918. | | | |

### Intermediate 30: cis-Ethyl 4-[[2-[5-[(2,4,5-trifluorophenyl)aminol-13,4-oxadiazol-2-yl]-3H-benzoimidazol-5-yl]oxy]cyclohexane-1-carboxylate

### i) Ethyl 4-(4-amino-3-nitro-phenoxy)cvclohexane-1-carboxylate

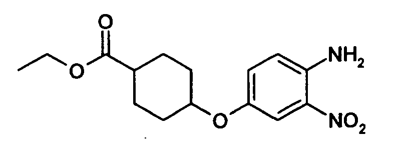

Diisopropyl azodicarboxylate (1.72 mL, 8.71 mmol) was added in one portion to a stirred solution of ethyl 4-hydroxycyclohexanecarboxylate (1.0 g, 5.8 mmol), 4-amino-3-nitrophenol (2.69 g, 17.4 mmol) and triphenylphoshine (2.29 g, 8.71 mmol) in THF (40 mL) under an argon atmosphere. The reaction mixture was stirred at ambient temperature for 16 h and then EtOAc (150 mL) and water (150 mL) were added. The layers were separated and the organic layer was washed with a 1M aqueous solution of HCl (150 mL), then a 1M aqueous solution of sodium hydroxide (150 mL) and then brine (150 mL). The organic layer was dried (MgSO₄), filtered and concentrated *in vacuo* to leave a dark brown solid. This solid was purified by column chromatography, eluting with a gradient of 30 to 40% EtOAc in isohexane, to give the title compound as an impure orange oil (1.15 g, 64%), that was used with no further purification; MS m/e MH⁺ 474.

### ii) cis-Ethyl 4-[4-[(methoxycarbonylformyl)amino]-3-nitro-phenoxy]cyclohexane-1-carboxylate

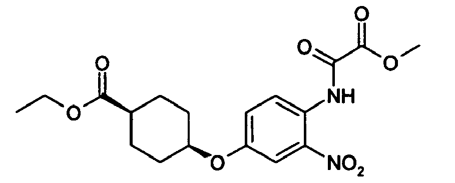

Methylchlorooxoacetate (0.89 mL, 9.66 mmol) was added in one portion to a stirred solution of ethyl 4-(4-amino-3-nitro-phenoxy)cyclohexane-1-carboxylate (2.98 g, 9.66 mmol) and pyridine (939 µL, 11.6mmol) in DCM (50 mL) at ambient temperature. The reaction mixture was stirred at ambient temperature for 30 mins and then concentrated *in vacuo* to leave a residue. The residue was partitioned between a 1M aqueous solution of HCl (150 mL) and EtOAc (200 mL). The layers were separated and the organic layer was washed with water (100 mL) and brine (100 mL) and then dried (MgSO₄), filtered and concentrated *in vacuo* to leave a yellow oil. The oil was purified by column chromatography, eluting with a gradient of 30 to 40% EtOAc in isohexane, to give the title compound as a yellow solid (666 mg, 18%).
¹H NMR δ 1.27 (3H, t), 1.63 - 1.82 (4H, m), 1.91 - 2.05 (4H, m), 2.39 - 2.46 (1H, m), 4.00 (3H, s), 4.16 (2H, q), 4.53 (1H, m), 7.28 (1H, m), 7.75 (1H, d), 8.69 (1H, d), 11.59 (1H, s); MS m/e MH⁺ 395.

### iii) cis-Ethyl4-[4-[(hydrazinecarbonylformyl)amino]-3-nitro-phenoxy]cyclohexane-1-carboxylate

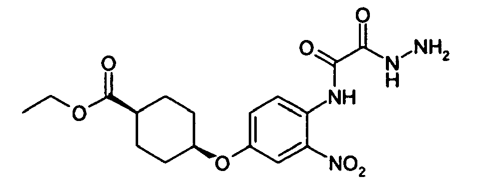

Hydrazine monohydrate (49 µL, 1.0mmol) was added to a stirred solution of *cis-*ethyl 4-[4-[(methoxycarbonylformyl)amino]-3-nitro-phenoxy]cyclohexane-1-carboxylate (330mg, 0.84mmol) in EtOH (50 mL). The reaction mixture was stirred at ambient temperature for 30 mins and then filtered to leave a solid. The solid was washed with EtOH (10 mL) and ether (10 mL) to leave the title compound as a bright yellow solid (228 mg, 69%).
¹H NMR δ 1.20 (3H, t), 1.68 - 1.87 (8H, m), 2.50 (1H, m), 4.08 (2H, q), 4.67 (3H, br s), 7.42 - 7.45 (1H, m), 7.63 (1H, m), 8.05 - 8.10 (1H, m), 10.40 (1H, s), 11.20 (1H, s); MS m/e (M-H)⁻ 393.

### iv) cis-Ethyl 4-[3-nitro-4-[[5-[(2,4,5-trifluorophenyl)amino]1,3,4-oxadiazole-2-carbonyl]amino]phenoxy]cyclohexane-1-carboxylate

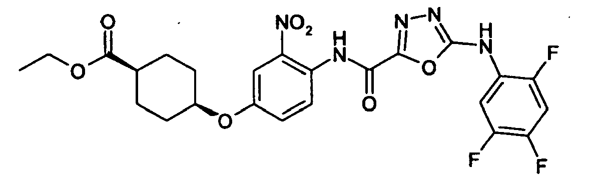

2,4,5-Trifluorophenylisothiocyanate (103 mg, 0.54 mmol) was added in one portion to a stirred solution of *cis-*ethyl 4-[4-[(hydrazinecarbonylformyl)amino]-3-nitrophenoxy]cyclohexane-1-carboxylate (213 mg, 0.54 mmol) in DMA (5 mL) at ambient temperature and the reaction mixture was stirred at ambient temperature for 30 mins. EDAC (119mg, 0.62mmol) was added and the reaction mixture was warmed to 80°C and stirred for 40 mins. The mixture was concentrated *in vacuo* to leave an orange residue and then EtOAc (200mL) and water (50mL) were added. The layers were separated and the aqueous layer was re-extracted with EtOAc (100mL). The combined organic layers were washed with brine (30mL), dried (MgSO₄ and concentrated *in vacuo* to leave a yellow solid. The solid was recrystallised using MeOH to give the title compound as a bright yellow solid (243mg, 82%).
¹H NMR δ 1.20 (3 H, t), 1.69 - 1.87 (8H, m), 2.50 (1H, m), 4.09 (2H, q), 4.69 (1H, s), 7.41 - 7.45 (1H, m), 7.61 (1H, m), 7.67 - 7.75 (1H, m), 7.82 - 7.87 (1H, m), 8.12 - 8.19 (1H, m), 11.10 (1H, s), 11.25 (1H, s); MS m/e MH⁺ 550.

### v) cis-Ethyl 4-[3-amino-4-[[5-[(2,4,5-trifluorophenyl)amino]1,3,4-oxadiazole-2-carbonyl]amino]phenoxy]cyclohexane-1-carboxylate

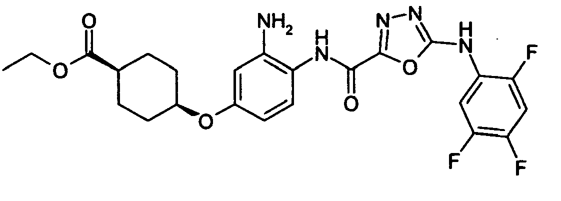

A solution of *cis-*ethyl 4-[3-nitro-4-[[5-[(2,4,5-trifluorophenyl)amino]1,3,4-oxadiazole-2-carbonyl]amino]phenoxy]cyclohexane-1-carboxylate (100 mg, 0.18 mmol) and 10% palladium on carbon (10 mg) in a mixture of EtOH (5 mL), THF (5 mL) and dioxane (5 mL) was stirred under a hydrogen atmosphere for 4 h. The reaction mixture was filtered and concentrated *in vacuo* to give the title compound as a yellow solid (100 mg, 100%); MS m/e MH⁺ 520.

### vi) cis-Ethyl 4-112-[5-[(2,4,5-trifluorophenyl)aminol-1,3,4-oxadiazol-2-yl]-3H-benzoimidazol-5-yl]oxyl]cyclohexane-1-carboxylate

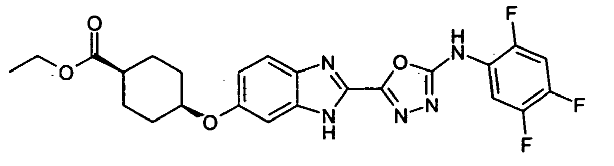

Acetic acid (1 mL) was added in one portion to a solution of *cis-*ethyl 4-[3-amino-4-[[5-[(2,4,5-trifluorophenyl)amino] 1,3,4-oxadiazole-2-carbonyl]amino]phenoxy]-cyclohexane-1-carboxylate (88 mg, 0.17mmol) in acetonitrile (8 mL) and the reaction mixture was heated at 150°C in a microwave for 15 mins. The reaction mixture was cooled to ambient temperature and filtered to leave a solid. The solid was washed with acetonitrile (20 mL) to leave the title compound (Intermediate 30) as a grey solid (55 mg, 65%).
¹H NMR δ 1.19 - 1.22 (3H, t), 1.62 -1.90 (8H, m), 2.50 (1H, m), 4.09 (2H, q), 4.58 (1H, br s), 6.93 - 6.96 (0.65H, m), 7.01 - 7.02 (1H, m), 7.28 (0.35H, m), 7.45 (0.35H, m), 7.63 (0.65H, m), 7.67 - 7.74 (1H, m), 8.21 (1H, m), 10.00 (0.65H, s), 11.90 (0.35H, s), 13.50 (¹H, m); MS m/e MH⁺ 502.

### Intermediate 31: cis-Ethyl 4-[[2-[5-[(4-fluorophenyl)amino]-1,3,4-oxadiazol-2-yl]-3H-benzoimidazol-5-yl]oxy]cyclohexane-1-carboxylate

### i) cis-Ethyl 4-[4-[[5-[(4-fluorophenyl)amino]1,3,4-oxadiazole-2-carbonyl]amino]-3-nitrophenoxy]cyclohexane-1-carboxylate

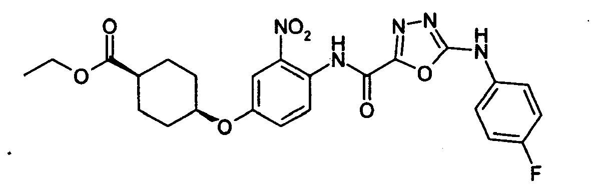

4-Fluorophenylisothiocyanate (89 mg, 0.58mmol) was added in one portion to a stirred solution of *cis-*ethyl4-[4-[(hydrazinecarbonylformyl)amino]-3-nitrophenoxy]cyclohexane-1-carboxylate (Intermediate 30iii, 227 mg, 0.58 mmol) in DMA (7 mL) and the reaction mixture was stirred at ambient temperature for 30 mins. EDAC (133 mg, 0.73 mmol) was added in one portion and the reaction mixture was stirred at 80 °C for 40 mins. The mixture was concentrated *in vacuo* to leave an orange residue that was partitioned between EtOAc (200 mL) and water (50 mL). The layers were separated and the aqueous layer was re-extracted with EtOAc (100 mL). The combined organic layers were washed with brine (30 mL), dried (MgSO₄) and concentrated *in vacuo* to leave a yellow solid. The solid was triturated with hot methanol, cooled to ambient temperature and filtered to leave the title compound as a yellow solid (236 mg, 79%).
¹H NMR δ 1.27 (3H, t), 1.67 - 1.82 (4H, m), 1.92 - 2.05 (4H, m), 2.41 - 2.46 (1H, m), 4.16 (2H, q), 4.55 (1H, s), 7.09 - 7.13 (2H, t), 7.27 - 7.30 (1H, m), 7.53 - 7.56 (2H, m), 7.59 (1H, s), 7.77 (1H, d), 8.68 (1H, d), 11.54 (1H, s); MS m/e MH⁺ 514.

### ii) cis-Ethyl 4-[3-amino-4-[[5-[(4-fluorophenyl)amino]1,3,4-oxadiazole-2-carbonyl]amino]phenoxy]cyclohexane-1-carboxytate

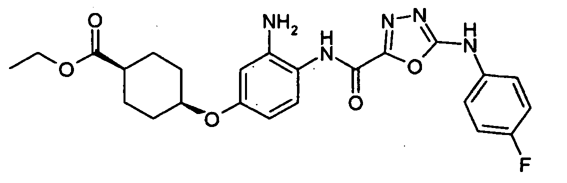

A solution of *cis-*ethyl 4-[4-[[5-[(4-fluorophenyl)amino]1,3,4-oxadiazole-2-carbonyl]amino]-3-nitro-phenoxy]cyclohexane-1-carboxylate (235 mg, 0.46 mmol) and 10% palladium on carbon (20 mg) in a mixture of EtOH (8 mL), THF (8 mL) and dioxane (8 mL) was stirred under a hydrogen atmosphere for 4 h. The reaction mixture was filtered and concentrated *in vacuo* to leave the title compound as a yellow solid (203 mg, 92%); MS m/e MH⁺ 484.

### iii) cis-Ethyl 4-[[2-[5-[(4-fluorophenyl)aminol-1,3,4-oxadiazol-2-yl]-3H-benzoimidazol-5-yl]oxy]clohexane-1-carboxylate

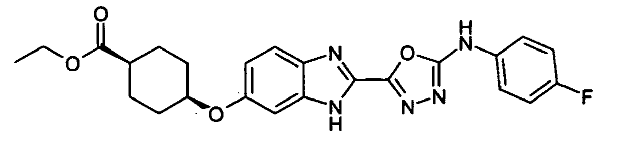

Acetic acid (2 mL) was added in one portion to a solution of 4-(3-amino-4-{[5-(4-fluorophenylamino)-[1,3,4]oxadiazole-2-carbonyl]-amino}-phenoxy)-cyclohexanecarboxylic acid ethyl ester (200 mg, 0.41mmol) in acetonitrile (10 mL) and the reaction mixture was heated in a microwave at 150°C for 15 mins. The reaction mixture was concentrated *in vacuo* to leave a solid that was recrystallised from EtOAc to give the title compound (Intermediate 31) as a white solid (30 mg, 16%); MS m/e MH⁺ 466.

### Intermediate 32: trans-Ethyl 4-[[2-[5-[(2,4,5-tritluorouhenyl)amino]-1,3,4-oxadiazol-2-yl]-3H-benzoimidazol-5-yl]oxy]cyclohexane-1-carboxylate

### i) trans-Ethyl 4-14-[(methoxycarbonylformyl)amino]-3-nitro-phenoxy]cyclohexane-1-carboxylate

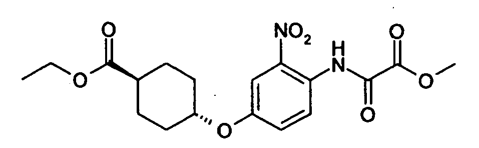

Methylchlorooxoacetate (66 µL, 0.71mmol) was added in one portion to a stirred solution of ethyl 4-(4-amino-3-nitro-phenoxy)cyclohexane-1-carboxylate (Intermediate 30i, 219 mg, 0.71 mmol) and pyridine (69 µL, 0.85 mmol) in DCM (5 mL) and the reaction mixture was stirred at ambient temperature for 30 mins. The mixture was then concentrated *in vacuo* to leave a residue. A 1M aqueous solution of HCl (50 mL) and EtOAc (50 mL) were added to the residue and the layers were separated. The organic layer was washed with water (50 mL) and brine (50 mL), dried (MgSO₄) and concentrated *in vacuo* to leave a yellow oil. The oil was purified by column chromatography, eluting with a gradient of 30 to 40% EtOAc in isohexane, to give the title compound as a yellow solid (58 mg, 12%); ¹H NMR δ 1.20 (3H, t), 1.40 - 1.61 (4H, m), 2.00 - 2.18 (4H, m), 2.26 - 2.33 (1H, m), 3.93 (3H, s), 4.08 (2H, q), 4.17 - 4.24 (1H, m), 7.17 - 7.19 (1H, m), 7.67 (1H, d), 8.62 (1H, d), 11.52 (1H, s); MS m/e MH⁺ 395.

### ii) trans-Ethyl 4-[4-[(hydrazinecarbonylformyl)amino]-3-nitro-phenoxy]cyclohexane-1-carboxylate

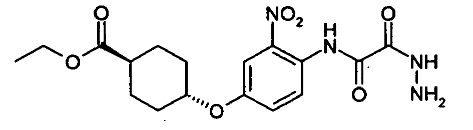

Hydrazine monohydrate (47 µL, 0.97 mmol) was added in one portion to a stirred solution of *trans-*ethyl 4-[4-[(methoxycarbonylformyl)amino]-3-nitro-phenoxy]cyclohexane-1-carboxylate (318 mg, 0.81 mmol) in EtOH (50 mL) and the reaction mixture was stirred at ambient temperature for 1 h. The mixture was filtered to leave a solid, which was washed with EtOH (10 mL) and ether (10 mL) to give the title compound as a yellow solid (156 mg, 49%); MS m/e MH⁺ 395.

### iii) trans-Ethyl 4-[3-nitro-4-[[5-[(2,4,5-trifluoronphenyl)aminol1,3,4-oxadiazole-2-carbonyl]amino]phenoxylcyclohexane-1-carboxylate

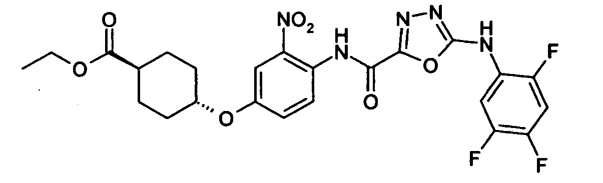

2,4,5-Trifluorophenylisothiocyanate (118 mg, 0.62 mmol) was added in one portion to a stirred solution of *trans-*ethyl 4-[4-[(hydrazinecarbonylformyl)amino]-3-nitrophenoxy]cyclohexane-1-carboxylate (245 mg, 0.62mmol) in DMA (5 mL) and the reaction mixture was stirred at ambient temperature for 30 mins. EDAC (143 mg, 0.75 mmol) was added in one portion and the mixture was stirred at 80°C for 30 mins. The reaction mixture was concentrated *in vacuo* to leave a yellow solid that was triturated with water (50 mL). The mixture was filtered to leave a solid that was recrystallised from MeOH to yield the title compound as a yellow solid (273 mg, 80%).
¹H NMR δ 1.20 (3H, t), 1.46 (2H, m), 1.53 - 1.63 (2H, m), 1.94 - 1.98 (2H, m), 2.07 - 2.11 (2H, m), 2.33 - 2.41 (1H, m), 4.08 (2H, q), 4.46 - 4.51 (1H, m), 7.40 - 7.43 (1H, m), 7.62 (1H, m), 7.67 - 7.75 (1H, m), 7.80 - 7.85 (1H, m), 8.12 - 8.19 (1H, m), 11.10 (1H, s), 11.25 (1H, s); MS m/e MH⁺ 550.

### iv) trans-Ethyl 4-[[2-[5-[(2,4,5-tritluorophenyl)aminol-1,3,4-oxadiazol-2-yl]-3H-benzoimidazol-5-yl]oxy]cyclohexane-1-carboxylate

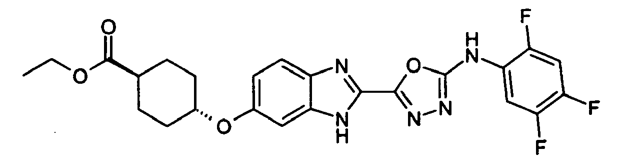

A suspension of *trans-*ethyl 4-[3-nitro-4-[[5-[(2,4,5-trifluorophenyl)amino] 1,3,4-oxadiazole-2-carbonyl]amino]phenoxy]cyclohexane-1-carboxylate (270 mg, 0.58mmol), ammonium formate (630 mg, 10mmol) and 10% palladium on carbon (3mg) in a mixture of EtOH (8 mL), DMF (8 mL) and formic acid (7 mL) was heated in a microwave at 150 °C for 20 mins. EtOH (40 mL) was added and the reaction mixture was filtered and concentrated *in vacuo* to leave a residue. The residue was recrystallised from MeOH to give the title compound (Intermediate 32) as a tan solid (75 mg, 30%); MS m/e MH⁺ 502.

### Intermediate 33: cis-Ethyl 4-[[4-fluoro-2-[5-[(4-fluorophenyl)aminol-1,3,4-oxadiazol-2-yl]-3H-benzoimidazol-5-yl]oxy]cyclohexane-1-carboxylate

### i) 3-Amino-2-fluoro-4-nitrophenol

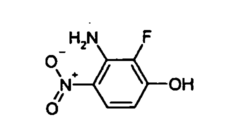

To a solution of 2,3-difluoro-6-nitroaniline (20.9 g, 120.05 mmol) in 1,4-dioxane (200 mL) was added a solution of potassium hydroxide (26.94 g, 480.18 mmol) in water (75 mL) and tetrabutylammonium hydrogen sulphate (10 g, 29.5 mmol) and the mixture stirred at 100°C for 18hr. 1M Citric acid (100 mL) added and the mixture extracted with ethyl acetate (2x100 mL). The organic extracts were washed with water (50 mL) and satd brine (50 mL), dried (MgSO₄), filtered and concentrated in vacuo. The crude residue was purified by silica flash chromatography with 20-65 % EtOAc in iso-hexane as eluent to give the title compound 14.1g, 68 %) as a yellow powder.
¹H NMR: δ 6.25 (1H, dd), 7.18 (2H, s), 7.75 (1H, dd); MS m/e MH⁻ 171.

### ii) Ethyl trans-4-(3-amino-2-fluoro-4-nitrophenoxy)cyclohexane-1-carboxylate and ethyl cis-4-(3-amino-2-fluoro-4-nitrophenoy)cyclohexane-1-carboxylate.

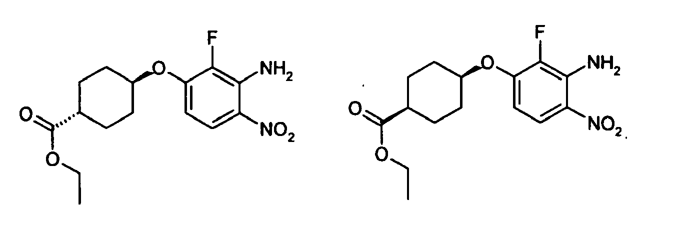

To a mixture of 3-amino-2-fluoro-4-nitrophenol (14.1 g, 81.93mmol), ethyl-4-hydroxycyclohexanecarboxylate (14.11 g, 81.93mmol) and triphenylphosphine (26.87 g, 102.41 mmol) in anhydrous THF (200 mL) cooled to 10°C was added a solution of diethylazodicarboxylate (17.83 g, 102.41 mmol) in anhydrous THF (50 mL) and the reaction stirred at ambient temperature for 72 hr. The mixture was concentrated in vacuo and the residue purified by silica flash chromatography with 20-50% EtOAc in iso-hexane as eluent to give:
ethyl *trans-*4-(3-amino-2-fluoro-4-nitro-phenoxy)cyclohexane-1-carboxylate (3.6g, 27%) as a yellow powder.
¹H NMR: δ 1.18 (3H, t), 1.4-1.64 (4H, m), 1.9-2.12 (4H, m), 2.31-2.44 (1H, m), 4.08 (2H, q), 4.43-4.59 (1H, m), 6.65 (1H, m), 7.14 (2H, s), 7.82 (1H, m) and ethyl *cis-*4-(3-amino-2-fluoro-4-nitro-phenoxy)cyclohexane-1-carboxylate (6.25g, 47%) as a yellow powder.
¹H NMR: δ 1.19 (3H, t), 1.62-1.92 (8H, m), 2.4-2.55 (1H, m), 4.08 (2H, q), 4.68-4.8 (1H, m), 6.62 (1H, m), 7.17 (2H, s), 7.84 (1H, m).

### iii) Ethyl cis-4-[2-fluoro-3-[(methoxycarbonylformyl)aminol-4-nitrophenoxy]cyclohexane-1-carboxylate

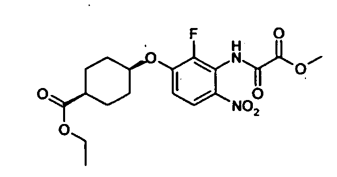

To a solution of ethyl *cis-*4-(3-amino-2-fluoro-4-nitrophenoxy)cyclohexane-1-carboxylate (1.1 g, 3.37 mmol) and pyridine (814 µL, 10.1 mmol) in anhydrous DCM (40 mL) cooled to 10 °C was added a solution of methyl 2-chloro-2-oxoacetate (620 mg, 5.06 mmol) in anhydrous DCM (10 mL) and the reaction stirred for 30 mins. The mixture was concentrated in vacuo, the residue diluted with EtOAc (50 mL), washed with 1M HCl (20 mL), satd NaHCO₃ (20 mL), water (25 mL) and brine (25 mL). The organic phase was dried (MgSO₄), filtered and concentrated in vacuo to give the title compound (1.39 g, 100 %) as a yellow oil.
MS m/e MH⁻ 411.

### iv) Ethyl cis-4-[2-fluoro-3-[(hydrazinecarbonylformyl)amino]-4-nitrophenoxylcyclohexane-1-carboxylate

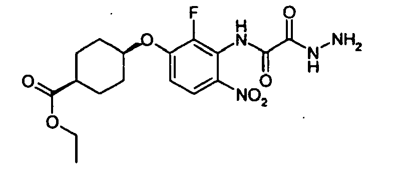

Ethyl *cis-*4-[2-fluoro-3-[(methoxycarbonylformyl)amino]-4-nitro-phenoxy]cyclohexane-1-carboxylate (1.66 g, 4.03 mmol) dissolved in absolute ethanol (25 mL), hydrazine hydrate (202 mg, 4.03 mmol) added and the mixture stirred at ambient temperature for 1 hr. Evaporated and dried in vacuo to give the title compound (1.6 g, 94%) as a yellow oil. MS m/e MH⁻ 411.

### v) Ethyl cis-4-[2-fluoro-3-[[5-[(4-fluorophenyl)amino]1,3,4-oxadiazole-2-carbonylamino]-4-nitro-phenoxy]cyclohexane-1-carboxylate

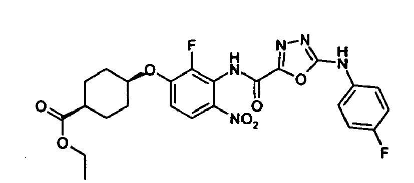

*cis-*Ethyl 4-[2-fluoro-3-[(hydrazinecarbonylformyl)amino]-4-nitro-phenoxy]cyclohexane-1-carboxylate (1.6 g, 3.88 mmol) was dissolved in anhydrous DMA (20 mL), 4-fluorophenyl isothiocyanate (714 mg, 4.66 mmol) added and the mixture stirred at ambient temperature for 1hr. EDC (1.12 g, 5.82 mmol) was added and the mixture heated to 90 °C for 1hr. Diluted with ethyl acetate (75 mL), washed with water and brine, dried (MgSO₄), filtered and evaporated in vacuo. The residue was purified by silica flash chromatography with 20-50% EtOAc in iso-hexane as eluent to give the title compound (1.5 g, 73%) as a pale yellow solid.
¹H NMR: δ 1.6-1.95(8H, m), 2.43-2.55(1H, m), 4.04(2H, q), 4.8-4.9(1H, m), 7.2-7.3(1H, m), 7.36-7.44(1H, m), 7.57-7.64(1H, m), 7.9-8.0(1H, m), 11.04(1H, s), 11.16(1H, s); MS m/e MH⁻ 530.

### vi) Ethyl cis-4-[4-amino-2-fluoro-3-[[5-[(4-fluorophenyl)amino]1,3,4-oxadiazole-2-carbonyl]amino]phenoxy]cyclohexane-1-carboxylate

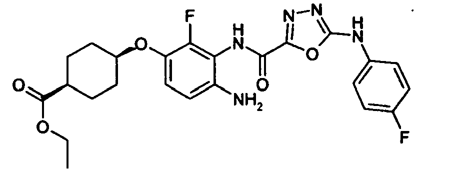

Ethyl *cis-*4-[2-fluoro-3-[[5-[(4-fluorophenyl)amino]-1,3,4-oxadiazole-2-carbonyl]amino]-4-nitrophenoxy]cyclohexane-1-carboxylate (1.5 g, 2.82 mmol) dissolved in THF (50 mL), 10% palladium on carbon (500 mg) added and the mixture stirred under a hydrogen atmosphere for 18 hr. Filtered and evaporated to give the title compound (760 mg, 54%) as a pale yellow solid.
MS m/e MH⁻ 500.

### vii Ethyl cis-4-[[4-fluoro-2-[5-[(4-fluorophenyl)amino]-1,3,4-oxadiazol-2-yl]-3H-benzoimidazol-5-yl]oxy]cyclohexane-1-carboxylate

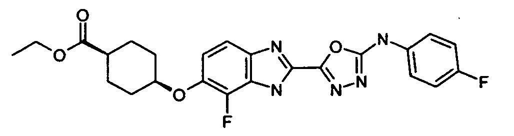

Ethyl *cis-*4-[4-amino-2-fluoro-3-[[5-[(4-fluorophenyl)amino]1,3,4-oxadiazole-2-carbonyl]amino]phenoxy]cyclohexane-1-carboxylate (760 mg, 1.51 mmol) was dissolved in 2:1 acetonitrile:glacial acetic acid (15 mL) and heated in a microwave reactor at 120°C for 15mins. The mixture was cooled, the solids filtered, washed with acetonitrile (10 mL) and dried in vacuo to give the title compound (300 mg, 41%) as a white powder.
¹H NMR: δ 1.27(3H, t), 1.66-1.8(4H, m), 1.85-2.0(4H, m), 2.45-2.59(1H, m), 4.15(2H, q), 4.49-4.6(1H, m), 7.23-7.44(4H, m), 7.68-7.77(2H, m), 11.1(1H, s), 14.0(1H, s); MS m/e MH⁺ 484.

The following intermediates were prepared in an analogous manner.

### Intermediate 34: Ethyl trans-4-[[4-fluoro-2-[5-[(4-fluorophenyl)aminol-1,3,4-oxadiazol-2-yl]-3H-benzoimidazol-5-yl]oxy]cyclohexane-1-carboxylate

### i) Ethyl trans-4-[2-fluoro-3-[(methoxycarbonylformyl)amino]-4-nitrophenoxy]cyclohexane-1-carboxylate

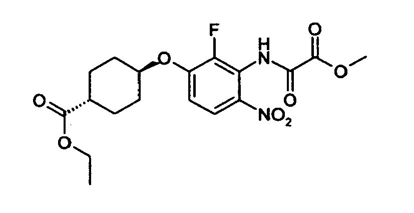

Prepared from ethyl *trans-*4-(3-amino-2-fluoro-4-nitrophenoxy)cyclohexane-1-carboxylate in an analogous manner to that described for intermediate 33 part iii)
MS m/e MH⁻ 411.

### ii) Ethyl trans-4-[2-fluoro-3-[(hydrazinecarbonylformyl)amino]-4-nitrophenoxy]cyclohexane-1-carboxylate

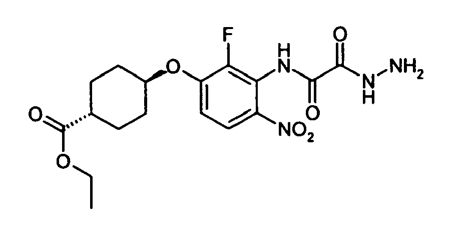

Prepared in an analogous manner to that described for intermediate 33 part iv) MS m/eMH⁻ 411.

### iii) Ethyl trans-4-[2-fluoro-3-[[5-[(4-fluorophenyl)amino]1,3,4-oxadiazole-2-carbonyl]amino]-4-nitrophenoxy]cyclohexane-1-carboxylate

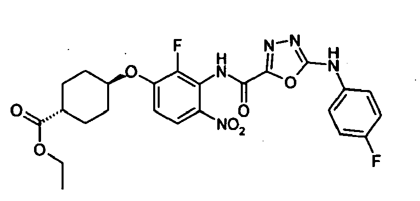

Prepared in an analogous manner to that described for intermediate 33 part v)
¹H NMR: δ 1.15(3H, t), 1.42-1.6(4H, m), 1.84-2.01(2H, m), 2.05-2.17(2H, m), 2.34-2.45(1H, m), 4.05(2H, q), 4.56-4.66(1H, m), 7.18-7.31(1H, m), 7.41-7.5(1H, m), 7.58-7.68(1H, m), 7.9-8.0(1H, m), 11.02(1H, s), 11.15 (1H, s).
MS m/e MH⁻ 530

### iv) Ethyl trans-4-[4-amino-2-fluoro-3-[[5-[(4-fluorophenyl)amino]1,3,4-oxadiazole-2-carbonyl]amino]phenoxy]cyclohexane-1-carboxylate

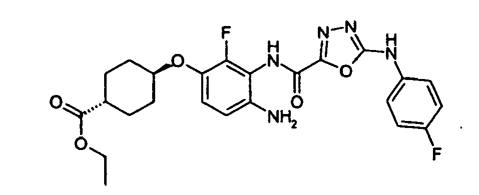

Prepared in an analogous manner to that described for intermediate 33 part vi)
MS m/e MH⁻ 500

### v) Ethyl trans4-[[4-fluoro-2-[5-[(4-fluorophenyl)amino]-1,3,4-oxadiazol-2-yl]-3H-benzoimidazol-5-yl]oxy]cyclohexane-1-carboxylate

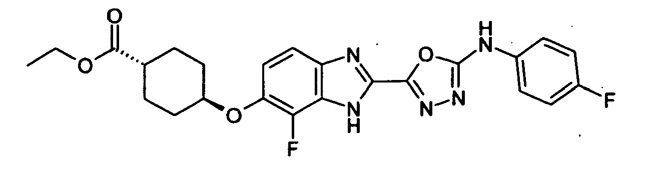

Prepared in an analogous manner to that described for intermediate 33 part vii)
¹H NMR: δ 1.25(3H, t), 1.45-1.63(4H, m), 1.95-2.2(4H, m), 2.38-2.5(1H, m), 4.12(2H, q), 4.2-4.33(1H, m), 7.23-7.45(4H, m), 7.67-7.7792H, m), 11.1(1H, s), 14.0(1H, s).
MS m/e MH⁺ 484.

### Intermediate 35: Ethyl cis-4-[[4-fluoro-2-[5-[(2,4,5-trifluorophenyl)amino]-1,3,4-oxadiazol-2-yl]-3H-benzoimidazol-5-yl]oxy]clohexane-1-carboxylate

### i) Ethyl cis-4-[2-fluoro-4-nitro-3-[[5-[(2,4,5-trifluorophenyl)amino]1,3,4-oxadiazole-2-carbonyl]amino]phenoxy]cyclohexane-1-carboxylate

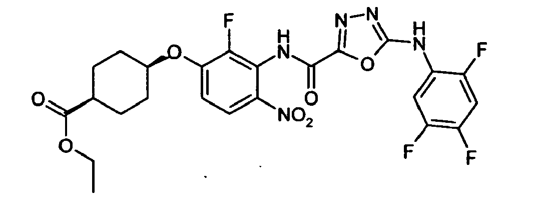

Prepared in an analogous manner to that described for intermediate 33 part v)
¹H NMR: 1.18(3H, t), 1.6-1.95(8H, m), 2.43-2.55(1H, m), 4.04(2H, q), 4.8-4.9(1H, m), 7.2-7.3(1H, m), 7.36-7.44(1H, m), 7.57-7.64 (1H,m), 7.9-8.0(1H, m), 11.04(1H, s), 11.16(1H, s).
MS m/e MH⁻ 566

### ii) Ethyl cis-4-[4-amino-2-fluoro-3-[[5-[(2,4,5-trifluorophenyl)amino]1,3,4-oxadiazole-2-carbonyl]amino]phenoxy]cyclobexane-1-carboxylate

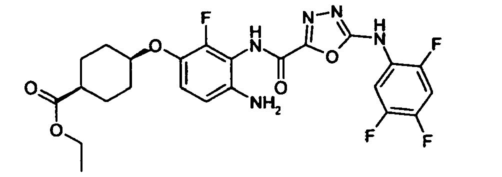

Prepared in an analogous manner to that described for intermediate 33 part vi) MS m/e MH⁻ 536

### iii) Ethyl cis-4-[[4-fluoro-2-[5-[(2,4,5-trifluoronhenyl)aminol-1,3,4-oxadiazol-2-yl]-3H-benzoimidazol-5-yl]oxy]cyclohexane-1-carboxylate

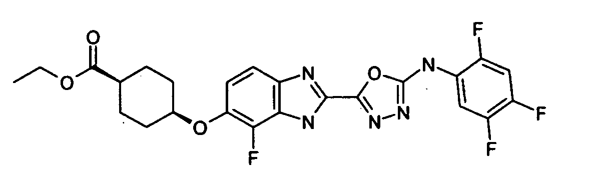

Prepared in an analogous manner to that described for intermediate 33 part vii)
¹NMR: 1.21 (3H, t), 1.61-1.73 (4H, m), 1.79-1.91 (4H, m), 2.42-2.53 (1H, m), 4.1 (2H, q), 4.43-4.54 (1H, m), 7.2-7.39 (2H, m), 7.69-7.8 (1H, m), 8.16-8.29 (1H, m), 11. (1H, s), 14.0 (1H, s).
MS m/e MH⁺ 520

### Intermediate 36: Ethyl trans-4-[[4-fluoro-2-[5-[(2,4,5-trifluorophenyl)aminol-1-3-4-oxadiazol-2-yl]-3H-benzoimidazol-5-yl]oxy]cyclohexane-1-carboxylate

### i) Ethyl trans-4-[2-fluoro-4-nitro-3-[[5-[(2,4,5-trifluorophenyl)amino]1,3,4-oxadiazole-2-carbonyl]amino]phenoxy]cyclohexane-1-carboxylate

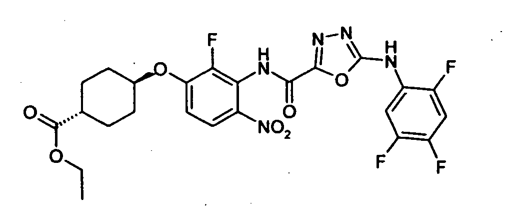

Prepared in an analogous manner to that described for intermediate 33 part v)
¹H NMR: 1.1(3H, t), 1.42-1.5(4H, m), 1.8-2.0(2H, m), 2.04-2.2(2H, m), 2.35-2.5(1H, m), 4.06(2H, q), 4.58-4.71(1H, m), 7.41-7.5(1H, m), 7.6-7.75(1H, m), 7.9-8.0(1H, m), 8.1-8.22(1H,m), 11.16(2H, s). MS m/e MH⁻ 566 '

### ii) Ethyl trans-4-[4-amino-2-fluoro-3-[[5-[(2,4,5-trifluorophenyl)amino]1,3,4-oxadiazole-2-carbonyl]amino]phenoxy]cyclohexane-1-carboxylate

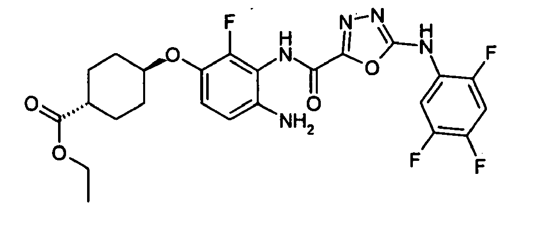

Prepared in an analogous manner to that described for intermediate 33 part vi) MS m/e MH⁻ 536.

### iii) Ethyl trans-4-[[4-fluoro-2-[5-[(2,4,5-trifluorophenyl)amino]-1,3,4-oxadiazol-2-yl]-3H-benzoimidazol-5-yl]oxy]cyclohexane-1-carboxylate

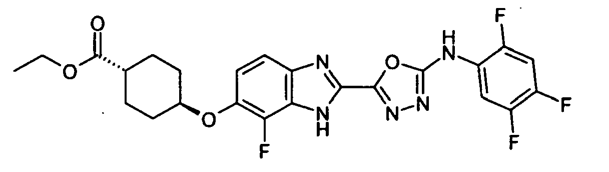

Prepared in an analogous manner to that described for intermediate 33 part vii)
¹H NMR: 1.25(3H, t), 1.45-1.62(4H, m), 1.96-2.2(4H, m), 2.38-2.48(1H, m), 4.11(2H, q), 4.22-4.35(1H, m), 7.26-7.44(2H, m), 7.74-7.86(1H, m), 8.23-8.35(1H, m), 11.1(1H, s), 14.0(1H, s). MS m/e MH⁺ 520.

### Intermediate 37: Methyl 3-[4-[2-[5-[(4-fluorophenyl)aminol-1,3,4-oxadiazol-2-yl]-1H-benzoimidazol-5-yl]cyclohexyl]oxypropanoate

### i) Benzyl N-[4-(1,4-dioxaspiro[4.5]dec-7-en-8-yl)phenyl]carbamate

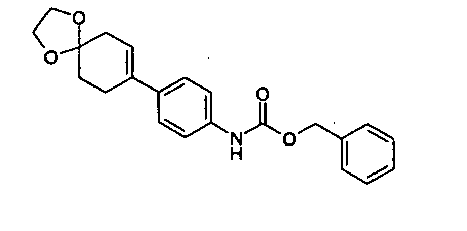

Tetrakistriphenylphosphine palladium (565 mg, 0.49 mmol) was added to a solution of 2-(1,4-dioxaspiro[4.5]dec-8-en-8-yl)-4,4,5,5-tetramethyl-1,3,2-dioxaborolane (1.3 g, 4.88 mmol) and benzyl *N-*(4-bromophenyl)carbamate (1.50 g, 4.88 mmol) in degassed DME (20 mL) and degassed 2M aqueous potassium carbonate solution (6.25 mL) and stirred under a nitrogen atmosphere at 80 °C for 24 h. The reaction mixture was cooled to ambient temperature and EtOAc (50 mL) was added before washing with water (2 x 20 mL) and brine (20 mL), dried (MgSO₄) and concentrated *in vacuo* to leave a residue. The crude residue was purified by column chromatography, using a gradient of 20-70% EtOAc in isohexane as eluent, to give the title compound as a cream coloured solid (1.30 g, 72 %). ¹H NMR δ 1.87-1.97 (2H, m), 2.42-2.5 (2H, m), 2.6-2.73 (2H, m), 4.03 (4H, s), 5.2 (2H, s), 5.9-5.99 (1H, m), 6.65 (1H, s), 7.5-7.7 (9H, m); MS m/e MH⁺ 266.

### ii) 4-(1,4-dioxaspiro[4.5]dec-8-yl)aniline

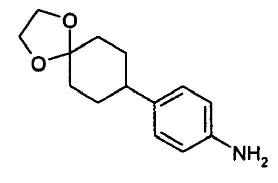

Palladium (10 wt. %) on carbon (1.0 g) was added in one portion to a solution of benzyl *N-*[4-(1,4-dioxaspiro[4.5]dec-7-en-8-yl)phenyl]carbamate (7.01 g, 19.20 mmol) in THF (500 mL) and the reaction mixture was stirred under a hydrogen atmosphere at ambient temperature for 16 h. The reaction mixture was filtered and concentrated *in vacuo* to leave the title compound as a white solid (4.03 g, 90%).
¹H NMR δ 1.51 - 1.76 (m, 8H), 2.32 - 2.43 (m, 1H), 3.87 (s, 4H), 4.77 (s, 2H), 6.48 (d, 2H), 6.86 (d, 2H).

### iii) N,N-Dibenzyl-4-(1,4-dioxaspiro[4.5]dec-8-yl)aniline

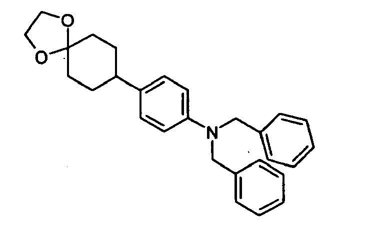

Benzyl bromide (5.95 g, 34.80 mmol) was added to a solution of 4-(1,4-dioxaspiro[4.5]dec-8-yl)aniline (3.87 g, 16.57 mmol) and potassium carbonate (6.86 g, 49.71 mmol) in DMA (60 mL) and stirred under a nitrogen atmosphere at 60°C for 16 h. The reaction mixture was allowed to cool to ambient temperature and water (30 mL) was added. The resulting precipitate was removed by filtration and dried under high vacuum to give the title compound as a white solid (5.46 g, 85%).
¹H NMR δ 1.46 - 1.77 (m, 8H), 2.32 - 2.46 (m, 1H), 3.84 (s, 4H), 4.63 (s, 4H), 6.58 (d, 2H), 6.92 (d, 2H), 7.16 - 7.36 (m, 10H); MS m/e MH⁺ 414.

### iv) 4-[4-(dibenzylamino)phenyl]cyclohexan-1-one

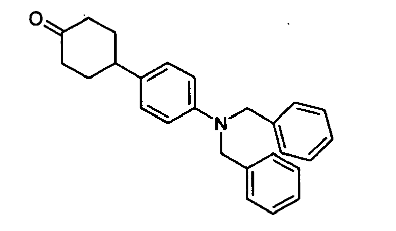

Water (1 mL) was added to a solution of N,N-dibenzyl-4-(1,4-dioxaspiro[4.5]dec-8-yl)aniline (3.09 g, 13.22 mmol) in TFA (20 mL) and stirred at ambient temperature for 72 h. The reaction mixture was concentrated *in vacuo* and the residue was dissolved in EtOAc (50 mL). This was washed with saturated aqueous sodium hydrogen carbonate (50 mL), dried (MgSO₄ and concentrated *in vacuo.* The crude residue was purified by column chromatography, using a gradient of 10-30% EtOAc in isohexane as eluent, to give the title compound as a colourless oil (3.16 g, 65%).
¹H NMR δ 1.48 - 1.84 (m, 4H), 1.91 - 2.04 (m, 2H), 2.14 - 2.26 (m, 2H), 2.80 - 2.93 (m, 1H), 4.65 (s, 4H), 6.59 (d, 2H), 7.00 (d, 2H), 7.17 - 7.36 (m, 10H); MS m/e MH⁺ 370.

### v) trans-4-[4-(Dibenzylamino)phenyl]cyclohexan-1-ol

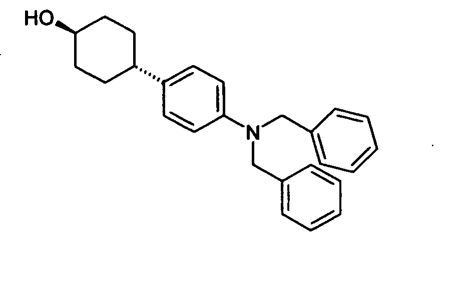

Sodium borohydride (563 mg, 14.88 mmol) was added to a stirred solution of 4-[4-(dibenzylamino)phenyl]cyclohexan-1-one (2.75 g, 7.44 mmol) in EtOH (50 mL) and acetonitrile (50 mL). The reaction mixture was stirred at ambient temperature for 1 h. Acetic acid (5 mL) was added and after stirring at ambient temperature for 15 min the solvent was removed *in vacuo.* The residue was dissolved in EtOAc and washed with a saturated aqueous solution of sodium hydrogen carbonate (10 mL), dried (MgSO₄) and concentrated *in vacuo* to leave a residue. The crude residue was purified by column chromatography, using a gradient of 20-50% EtOAc in isohexane as eluent, to give the title compound as a white crystalline solid (2.20 g, 79%).
¹H NMR (CDCl₃) δ1.33-1.53 (4H, m), 1.57 (1H, s), 1.85-1.96 (2H, m), 2.04-2.14 (2H, m), 2.33-2.45 (1H, m), 3.59-3.73 (1H, m), 4.63 (4H, s), 6.7 (2H, s), 7.01 (2H, d), 7.2-7.39 (10H, m).

### vi) tert-Butyl trans-3-[4-[4-(dibenzylamino)phenyl]cyclohexyl]oxypropanoate

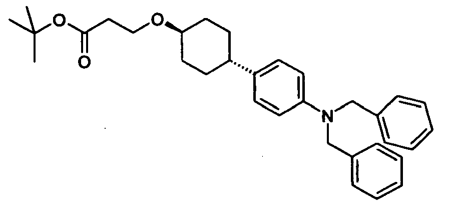

*tert*-Butyl prop-2-enoate (158 uL, 1.08 mmol) in DCM (5 mL) was added via syringe pump over 16 h to a stirred solution of *trans*-4-[4-(dibenzylamino)phenyl]cyclohexan-1-ol (201 mg, 0.54 mmol) and tetrabutylammonium hydrogen sulfate (46 mg, 0.14 mmol) in DCM (5 mL) and 50% wt. aqueous sodium hydroxide (500 uL) at ambient temperature. Water (10 mL) was added and the layers were separated. The aqueous layer was extracted with DCM (20 mL) and the combined organic layers were dried (MgSO₄) and concentrated *in vacuo* to leave a residue. The crude residue was purified by column chromatography, using a gradient of 0-20% EtOAc in isohexane as eluent, to give the title compound as a colourless oil (270 mg, 100%).
¹H NMR: δ 1.12- 1.39 (m, 4H), 1.40 (s, 9H), 1.68 - 1.77 (m, 2H), 1.95 - 2.04 (m, 2H), 2.24 - 2.34 (m, 1H), 2.39 (t, 2H), 3.17 - 3.28 (m, 1H), 3.56 (t, 2H), 4.65 (s, 4H), 6.57 (d, 2H), 6.92 (d, 2H), 7.19 - 7.35 (m, 10H); MS m/e MH⁺ 500.

### vii) Methyl trans-3-[4-[4-(dibenzylamino)phenyl]cyclohexyl]oxypropanoate

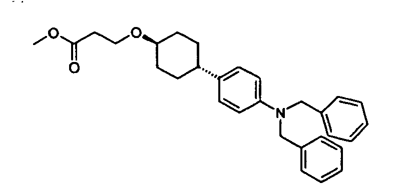

Concentrated HCl (0.5 mL) was added to a solution of tert-butyl *trans*-3-[4-[4-(dibenzylamino)phenyl]cyclohexyl]oxypropanoate (1.2 g, 2.40 mmol) in MeOH (20 mL) and the reaction mixture was stirred at 70°C for 4 h. An aqueous solution of sodium hydrogen carbonate (10 mL) was added and the resulting solution was extracted with EtOAc (50 mL). The organic layer was dried (MgSO₄) and concentrated *in vacuo* to leave a residue. The crude residue was purified by column chromatography, using a gradient of 0-50% EtOAc in isohexane as eluent, to give the title compound as a colourless oil (1.07 g, 98%).
¹H NMR: δ 1.07 - 1.43 (m, 4H), 1.65 - 1.78 (m, 2H), 1.92 - 2.05 (m, 2H), 2.21 - 2.34 (m, 1H), 2.47 - 2.55 (m, 2H), 3.15 - 3.27 (m, 1H), 3.58 (s, 3H), 3.64 (t, 2H), 4.63 (s, 4H), 6.57 (d, 2H), 6.92 (d, 2H), 7.17 - 7.36 (m, 10H); MS m/e MH⁺ 458.

### viii) Methyl trans-3-[4-(4-aminophenyl)cyclohexyl]oxypropanoate

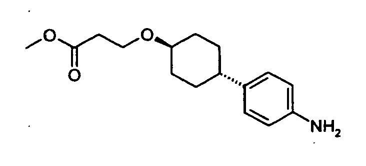

Palladium (10 wt. %) on carbon (500 mg) was added in one portion to a solution of methyl *trans*-3-[4-[4-(dibenzylamino)phenyl]cyclohexyl]oxypropanoate (1.21 g, 2.63 mmol) in THF (50 mL) and the reaction mixture was stirred under a hydrogen atmosphere at ambient temperature for 16 h. The reaction mixture was filtered and concentrated *in vacuo* to leave the title compound as a white solid (702 mg, 96%).
¹H NMR: δ 1.10 - 1.45 (m, 4H), 1.65 - 1.79 (m, 2H), 1.93 - 2.05 (m, 2H), 2.19 - 2.34 (m, 1H), 2.47 - 2.55 (m, 2H), 3.18 - 3.27 (m, 1H), 3.60 (s, 3H), 3.66 (t, 2H), 4.76 (s, 2H), 6.46 (d, 2H), 6.83 (d, 2H); MS m/e MH⁺ 278.

### ix) Methyl trans-3-[4-[4-[(methoxycarbonylformyl)amino]phenyl]-cyclohexyl]oxypropanoate

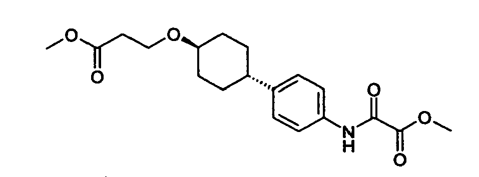

Methyl chlorooxoacetate (257 uL, 2.78 mmol) was added dropwise to a stirred solution of methyl *trans*-3-[4-(4-aminophenyl)cyclohexyl]oxypropanoate (701 mg, 2.53 mmol) and diisopropylethylamine (880 uL, 5.06 mmol) in DCM (20 mL) and the reaction mixture was stirred at ambient temperature for 16 h. Water (15 mL) was added and the layers were separated. The organic layer was washed with an aqueous solution of hydrochloric acid (1M, 10 mL) and then a saturated aqueous solution of sodium hydrogen carbonate (10 mL), dried (MgSO₄) and concentrated *in vacuo* to provide the title compound as a white solid (848 mg, 92%).
¹H NMR: δ 1.16 - 1.30 (m, 2H), 1.38 - 1.52 (m, 2H), 1.74 - 1.83 (m, 2H), 2.00 - 2.09 (m, 2H), 2.41 - 2.48 (m, 1H), 2.51 - 2.56 (m, 2H), 3.24 - 3.32 (m, 1H), 3.61 (s, 3H), 3.67 (t, 2H), 3.85 (s, 3H), 7.21 (d, 2H), 7.63 (d, 2H), 10.74 (s, 1H); MS m/e M-H⁺ 362.

### x) Methyl trans-3-[4-[4-[(methoxycarbonylformyl)amino]-3-nitro-phenyl]-cyclohexyl]oxypropanoate

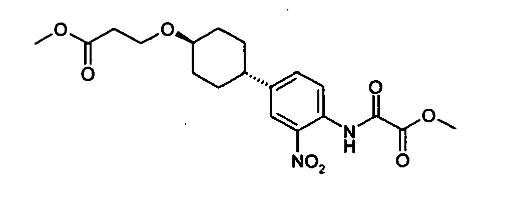

Trifluoroacetic anhydride (326 uL, 2.34 mmol) was added in one portion to a solution of ammonium nitrate (188 mg, 2.34 mmol) in DCM (12 mL) stirring under a nitrogen atmosphere at 0°C. After 1 min methyl *trans*-3-[4-[4-[(methoxycarbonylformyl)amino]-phenyl]cyclohexyl]oxypropanoate dissolved in DCM (4 mL) was added dropwise before stirring at 0°C for 30 min. The reaction mixture was allowed to warm to ambient temperature and stirred for a further 2 h. An aqueous solution of sodium hydrogen carbonate (10 mL) was added and the layers were separated. The organic layer was dried (MgSO₄) and concentrated *in vacuo* to to leave a residue. The crude residue was purified by column chromatography, using a gradient of 0-50% EtOAc in isohexane as eluent, to give the title compound as a yellow solid (707 mg, 1.73 mmol, 74%).
¹H NMR: δ 1.15 - 1.33 (m, 2H), 1.40 - 1.58 (m, 2H), 1.76 - 1.89 (m, 2H), 1.98 - 2.11 (m, 2H), 2.49 - 2.56 (m, 2H), 2.57 - 2.70 (m, 1H), 3.24 - 3.35 (m, 1H), 3.59 (s, 3H), 3.67 (t, 2H), 3.87 (s, 3H), 7.64 - 7.70 (m, 1H), 7.89 - 7.96 (m, 2H), 11.27 (s, 1H); MS m/e M-H⁺ 407.

### xi) Methyl trans-3-[4-[4-[(hydrazinecarbonylformyl)amino]-3-nitro-phenyl]cyclohexyl]oxypropanoate

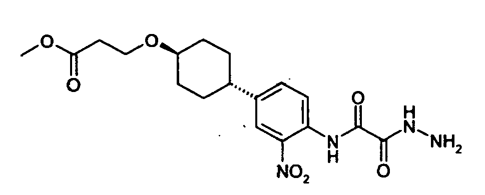

Hydrazine monohydrate (2.29 mL, 47.1 mmol) was added in one portion to a stirred solution of methyl *trans*-3-[4-[4-[(methoxycarbonylformyl)amino]-3-nitrophenyl]cyclohexyl]oxypropanoate (702 mg, 1.72 mmol) in EtOH (20 mL) and the reaction mixture was stirred at 60°C for 1 h. The solvent was removed *in vacuo* and the resulting solid was triturated with ether to give the title compound as a yellow solid (560 mg, 80%).
¹H NMR: δ 1.16 - 1.34 (m, 2H), 1.39 - 1.60 (m, 2H), 1.76 - 1.89 (m, 2H), 1.99 - 2.11 (m, 2H), 2.46 - 2.52 (m, 2H), 2.53 - 2.74 (m, 1H), 3.24 - 3.36 (m, 1H), 3.61 (s, 3H), 3.67 (t, 2H), 4.69 (s, 2H), 7.68 (d, 1H), 7.96 (s, 1H), 8.14 (d, 1H), 10.47 (s, 1H), 11.39 (s, 1H); MS m/e MH⁺ 409.

### xii) Methyl trans-3-[4-[4-[[5-f(4-fluorophenyl)amino]1,3,4-oxadiazole-2-carbonyl]amino]-3-nitro-phenyl]cyclohexyl]oxypropanoate

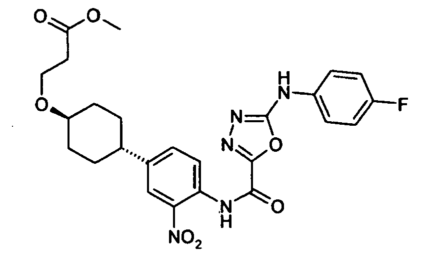

Methyl *trans*-3-[4-[4-[(hydrazinecarbonylformyl)amino]-3-nitrophenyl]cyclohexyl]oxypropanoate (559 mg, 1.37 mmol) was added in one portion to a stirred solution of 4-difluorophenyl isothiocyanate (252 mg, 1.64 mmol) in DMF (10 mL) and the reaction mixture was stirred at 60°C for 1 h. EDCI (316 mg, 1.64 mmol) was added in one portion and the reaction mixture was heated to 85°C for 4 h. The mixture was cooled to ambient temperature and water (15 mL) was added and the resulting suspension was filtered to leave a cream solid. This was washed with water (10 mL) and ether (10 mL) to give the title compound as a yellow solid (438 mg, 61%).
1H NMR: δ 1.16 - 1.34 (m, 2H), 1.42 - 1.59 (m, 2H), 1.78 - 1.91 (m, 2H), 2.00 - 2.11 (m, 2H), 2.53 (t, 2H), 2.58 - 2.74 (m, 1H), 3.26 - 3.38 (m, 1H), 3.60 (s, 3H), 3.68 (t, 2H), 7.18 - 7.31 (m, 2H), 7.56 - 7.73 (m, 3H), 7.89 - 7.98 (m, 2H), 10.89 - 11.59 (m, 2H); MS m/e mass ion not seen.

### xiii) Methyl trans-3-[4-[3-amino-4-[[5-[(4-fluorophenyl)amino]1,3,4-oxadiazole-2-carbonyl]amino]phenyl]cyclohexyl]oxypropanoate

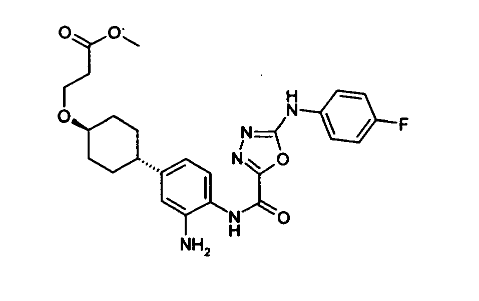

Palladium (10 wt. %) on carbon (100 mg) was added in one portion to a solution of methyl *trans*-3-[4-[4-[[5-[(4-fluorophenyl)amino] 1,3,4-oxadiazole-2-carbonyl]amino]-3-nitrophenyl]cyclohexyl]oxypropanoate (438 mg, 0.83 mmol) in THF (20 mL) and the reaction mixture was stirred under a hydrogen atmosphere at ambient temperature for 16 h. The reaction mixture was filtered and concentrated *in vacuo* to leave the title compound as a yellow solid (413 mg, 100%).
¹H NMR (499.803 MHz): δ 1.19 - 1.50 (m, 4H), 1.71-1.88 (m, 2H), 1.97 - 2.09 (m, 2H), 2.32 - 2.43 (m, 1H), 2.47 - 2.56 (m, 2H), 3.21 - 3.35 (m, 1H), 3.60 - 3.63 (m, 3H), 3.66 - 3.73 (m, 2H), 4.66 (s, 2H), 6.44 - 6.71 (m, 2H), 7.09 - 7.64 (m, 5H), 10.54 (s, 1H), 10.73 (s, 1H); MS m/e MH⁺ 499.

### xiv) Methyl trans-3-[4-[2-[5-[(4-fluorophenyl)amino]-1,3,4-oxadiazol-2-yl]-1H-benzoimidazol-5-yl]cyclohexyl]oxyprovanoate

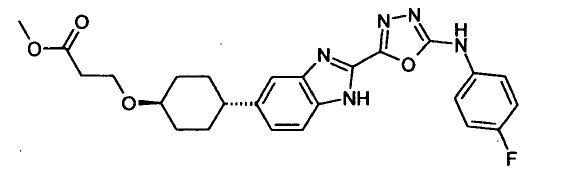

Methyl *trans*-3-[4-[3-amino-4-[[5-[(4-fluorophenyl)amino] 1,3,4-oxadiazole-2-carbonyl]amino]phenyl]cyclohexyl]oxypropanoate (408 mg, 0.82 mmol) in acetic acid (10 mL) was heated in the microwave at 100°C for 15 mins. The precipitate formed was filtered and dried under high vacuum to give the title compound as a yellow solid (190 mg, 48%).
¹H NMR: δ 1.26 - 1.43 (m, 2H), 1.48 - 1.69 (m, 2H), 1.87-1.99 (m, 2H), 2.07 - 2.18 (m, 2H), 2.61 (t, 2H), 2.64 - 2.75 (m, 1H), 3.36 - 3.45 (m, 1H), 3.68 (s, 3H), 3.75 (t, 2H), 7.21 - 7.36 (m, 4H), 7.59 - 7.77 (m, 3H), 11.04 (s, 1H), 13.63 (s, 1H); MS m/e MH⁺ 480.

### Intermediate 38 Methyl trans-2-[4-[2-[5-[(4-fluorohenyl)amino]-1,3,4-oxadiazol-2-yl]-1H-benzoimidazol-5-yl]cyclohexyl]oxyacetate

### i) tert-Butyl trans-2-[4-[4-(dibenzylamino)phenyl]cyclohexyl]oxyacetate

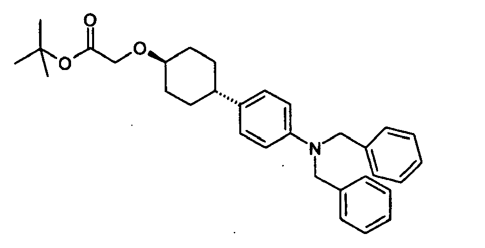

Prepared in an analogous manner to intermediate 37 part vi)
¹H NMR: δ 1.15 - 1.50 (m, 13H), 1.65 - 1.78 (m, 2H), 1.94 - 2.09 (m, 2H), 2.23 - 2.35 (m, 1H), 3.23 - 3.30 (m, 1H), 3.97 (s, 2H), 4.64 (s, 4H), 6.55 (d, 2H), 6.91 (d, 2H), 7.16 - 7.36 (m, 10H); MS m/e MH⁺ 486.

### ii) Methyl trans-2-[4-[4-(dibenzylamino)phenyl]cyclohexyl]oxyacetate

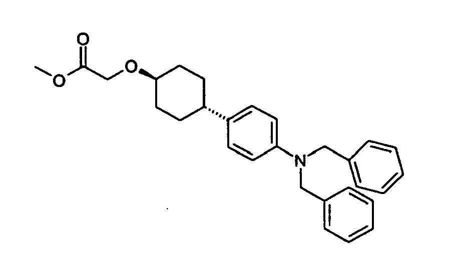

Prepared in an analogous manner to intermediate 37 part vii)
¹H NMR: δ 1.10 - 1.44 (m, 4H), 1.65 - 1.79 (m, 2H), 1.95 - 2.09 (m, 2H), 2.21 - 2.40 (m, 1H), 3.24 - 3.38 (m, 1H), 3.62 (s, 3H), 4.11 (s, 2H), 4.63 (s, 4H), 6.56 (d, 2H), 6.91 (d, 2H), 7.16 - 7.37 (m, 10H); MS m/e MH⁺ 444.

### iii) Methyl trans-2-[4-(4-aminophenyl)cyclohexyl]oxyacetate

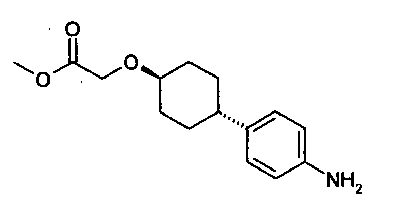

Prepared in an analogous manner to intermediate 37 part viii)
1H NMR: δ 1.18 - 1.42 (m, 4H), 1.68 - 1.78 (m, 2H), 2.02 - 2.10 (m, 2H), 2.24 - 2.35 (m, 1H), 3.29 - 3.34 (m, 1H), 3.65 (s, 3H), 4.15 (s, 2H), 4.81 (s, 2H), 6.46 (d, 2H), 6.85 (d, 2H); MS m/e MH⁺ 264.

### iv) Methyl trans-2-[4-[4-[(methoxycarbonylformyl)amino]-phenyl]cyclohexyl]oxyacetate

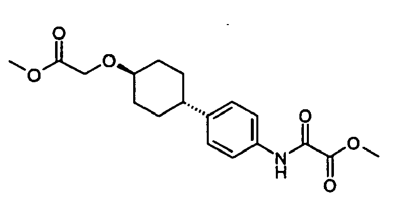

Prepared in an analogous manner to intermediate 37 part ix)
¹H NMR: δ 1.21 - 1.51 (m, 4H), 1.75 - 1.84 (m, 2H), 2.05 - 2.13 (m, 2H), 2.42 - 2.49 (m, 1H), 3.34 - 3.39 (m, 1H), 3.66 (s, 3H), 3.85 (s, 3H), 4.16 (s, 2H), 7.20 (d, 2H), 7.63 (d, 2H), 10.74 (s, 1H); MS m/e MH⁺ 350.

### v) Methyl trans-2-[4-[4-[(methoxycarbonylformyl)amino]-3-nitrophenyl]cyclohexyl]oxyacetate

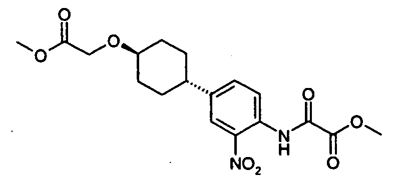

Prepared in an analogous manner to intermediate 37 part x)
¹H NMR: δ 1.23 - 1.37 (m, 2H), 1.43 - 1.57 (m, 2H), 1.78 - 1.89 (m, 2H), 2.05 - 2.17 (m, 2H), 2.59 - 2.72 (m, 1H), 3.36 - 3.44 (m, 1H), 3.66 (s, 3H), 3.88 (s, 3H), 4.17 (s, 2H), 7.66 - 7.72 (m, 1H), 7.89 - 7.97 (m, 2H), 11.30 (s, 1H); MS m/e M-H⁺ 393.

### vi) Methyl trans-2-[4-[4-[(hydrazinecarbonylformyl)amino]-3-nitrophenyl]cyclohexyl]oxyacetate

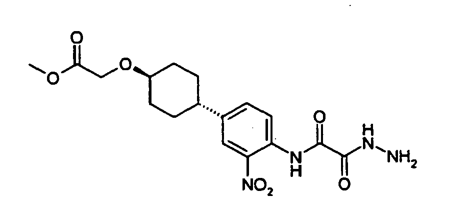

Prepared in an analogous manner to intermediate 37 part xi)
¹H NMR: δ 1.01 - 1.18 (m, 2H), 1.22 - 1.37 (m, 2H), 1.58 - 1.69 (m, 2H), 1.85 - 1.97 (m, 2H), 2.38 - 2.50 (m, 1H), 3.17 - 3.24 (m, 1H), 3.45 (s, 3H), 3.96 (s, 2H), 4.48 - 4.54 (m, 2H), 7.46 - 7.51 (m, 1H), 7.77 (d, 1H), 7.92 (d, 1H), 10.31 (s, 1H), 11.20 (s, 1H); MS m/e M-H⁺ 393.

### vii) Methyl trans-2-[4-[4-[[5-[(4-fluorophenyl)amino]1,3,4-oxadiazole-2-carbonyl]amino]-3-nitro-phenyl]cyclohexyl]oxyacetate

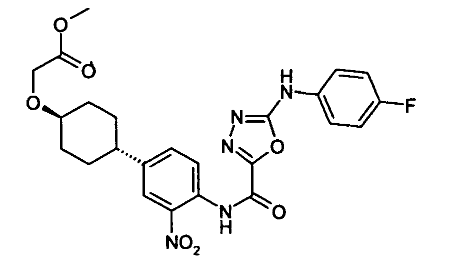

Prepared in an analogous manner to intermediate 37 part xii)
¹H NMR: δ 1.24 - 1.38 (m, 2H), 1.44 - 1.59 (m, 2H), 1.80 - 1.91 (m, 2H), 2.06 - 2.18 (m, 2H), 2.58 - 2.72 (m, 1H), 3.39 - 3.46 (m, 1H), 3.66 (s, 3H), 4.17 (s, 2H), 7.23 - 7.31 (m, 2H), 7.59 - 7.66 (m, 2H), 7.67 - 7.72 (m, 1H), 7.89 - 7.97 (m, 2H), 11.11 (s, 1H), 11.43 (s, 1H); MS m/e MH⁺ 515.

### viii) Methyl trans-2-[4-[3-amino-4-[[5-[(4-fluorophenyl)amino]1,3,4-oxadiazole-2-carbonyl]amino]phenyl]cyclohexyl]oxyacetate

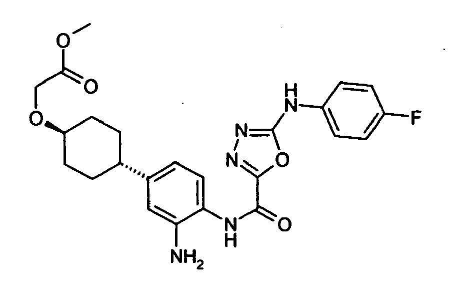

Prepared in an analogous manner to intermediate 37 part xiii)
¹H NMR: δ 1.22 - 1.53 (m, 4H), 1.74 - 1.85 (m, 2H), 2.04 - 2.15 (m, 2H), 2.31 - 2.41 (m, 1H), 3.36 - 3.42 (m, 1H), 3.66 (s, 3H), 4.16 (s, 2H), 4.93 (s, 2H), 6.45 (d, 1H), 6.61 (s, 1H), 7.02 (d, 1H), 7.20 - 7.29 (m, 2H), 7.57 - 7.65 (m, 2H), 10.18 (s, 1H), 11.00 (s, 1H); MS m/e M-H⁺ 483.

### ix) Methyl trans-2-[4-[2-[5-[(4-fluorophenyl)aminol-1,3,4-oxadiazol-2-yl]-1H-benzoimidazol-5-yl]cyclohexyl]oxyacetate

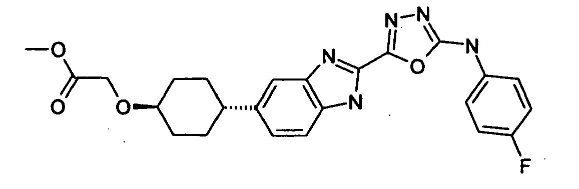

Prepared in an analogous manner to intermediate 37 part xiv)
¹H NMR δ: 1.21 - 1.60 (m, 4H), 1.75 - 1.93 (m, 2H), 2.04 - 2.18 (m, 2H), 2.58 - 2.70 (m, 1H), 3.38 - 3.47 (m, 1H), 3.64 - 3.69 (m, 3H), 4.14 - 4.20 (m, 2H), 7.15 - 7.30 (m, 4H), 7.58 - 7.71 (m, 3H), 10.91 - 11.05 (m, 1H), 13.56 (s, 1H); MS m/e MH⁺ 466.

## Claims

1. A compound of formula (I) 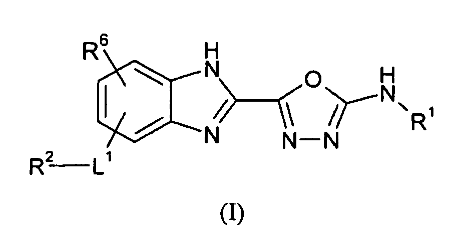 or a salt thereof, wherein:
R¹ is selected from phenyl, cyclopentyl, cyclohexyl, and HET-1, wherein R¹ is optionally substituted with either:
i) a substituent selected from group a) and optionally a substituent selected from either group b) or group c); or
ii) 1 or 2 substituents independently selected from group b) and optionally a substituent selected from group c); or
iii) up to 4 substituents independently selected from group c);
wherein groups a) to c) are as follows:
group a) nitro, -C(O)ₙR²⁰, a carboxylic acid mimic or bioisostere thereof, -NR²¹R²², - C(O)NR²¹R²², -OC(O)NR²¹R²², -NR²¹C(O)ₙR²⁰, -NR²⁰CONR²¹R²², -S(O)₂NR²¹R²² or-NR²¹S(O)₂R²² where R²⁰, R²¹ and R²² are independently selected from hydrogen and (1-6C)alkyl, or R²¹ and R²² together with the nitrogen atom to which they are attached form an optionally substituted ring having 3 to 10 atoms, which optionally contains further heteroatoms such as S(O)ₘ, oxygen and nitrogen;
group b) R¹ is optionally substituted by 1 or 2 substituents independently selected from halo(1-6C)alkyl, cyano, (1-6C)alkyl, hydroxy, (1-6C)alkoxy, benzyloxy, -SOₘ(1-6C)alkyl and -OSO₂(1-6C)alkyl;
group c) halo;
and when R¹ is substituted by two (1-6C)alkoxy groups, these may be joined together to form a 5- or 6-membered ring fused to R¹;
n is (independently at each occurrence) 1 or 2;
m is (independently at each occurrence) 0, 1 or 2;
L¹ is a direct bond or is a linker selected from -O-, -OCH₂-, -CH₂O-, -S(O)ₘ-, -S(O)ₘCH₂-, -CH₂S(O)ₘ- and -(CR⁷R⁸)₁₋₂-;
R² is selected from (3-6C)cycloalkyl, (5-12C)bicycloalkyl, phenyl, HET-2 and (2-6C)alkyl; wherein R² is optionally substituted by -L²-R³;
L² is a direct bond or is a linker selected from -(CR⁴R⁵)₁₋₂-, -O-(CR⁴R⁵)₁₋₂- and - CH₂(CR⁴R⁵)₁₋₂- (wherein for each value of L², the CR⁴R⁵ group is directly attached to R³); each R⁴ is independently selected from hydrogen, hydroxy, (1-3C)alkoxy, (1-4C)alkyl, hydroxy(1-3C)alkyl and (1-2C)alkoxy(1-2C)alkyl; provided that when L² is -O-(CR⁴R⁵)₁₋₂-then the R⁴ on the carbon atom directly attached to the oxygen atom is not hydroxy or (1-3C)alkoxy;
each R⁵ is independently selected from hydrogen and methyl;
R³ is selected from hydroxy, carboxy, (1-6C)alkoxycarbonyl, and a carboxylic acid mimic or bioisostere;
R⁶ is selected from hydrogen, fluoro, chloro, hydroxy, methoxy, halo(1-2C)alkyl, methyl, ethyl, cyano and methylsulfonyl;
each R⁷ is independently selected from hydrogen, (1-4C)alkyl and (1-4C)alkoxy;
each R⁸ is independently selected from hydrogen and methyl;
HET-1 is a 5- or 6-membered heteroaryl ring containing I or 2 ring heteroatoms independently selected from O, N and S (provided there are no O-O, S-S or O-S bonds within the ring);
HET-2 is a 4, 5- or 6-membered saturated, partially or fully unsaturated heterocyclyl ring containing 1, 2 or 3 ring heteroatoms independently selected from O, N and S (provided there are no O-O, S-S or O-S bonds within the ring), wherein a ring carbon atom may be oxidised to C(O) and/or a ring sulfur atom may be oxidised to S(O) or S(O)₂.

2. A compound as claimed in Claim 1 wherein R¹ is phenyl.

3. A compound as claimed in Claim 1 or Claim 2 wherein R¹ is substituted by 1, 2 or 3 fluoro.

4. A compound as claimed in any one of the preceding claims wherein L¹ is a direct bond or -O-.

5. A compound as claimed in any one of the preceding claims wherein R² is (3-6C)cycloalkyl.

6. A compound as claimed in any one of the preceding claims wherein L² is a direct bond, -CH₂- or -O-(CR⁴R⁵)₁₋₂-.

7. A compound as claimed in any one of the preceding claims wherein R³ is carboxy.

8. A compound as claimed in any one of the preceding claims wherein R⁶ is hydrogen or fluoro.

9. A compound of formula (IA) as claimed in Claim 1, 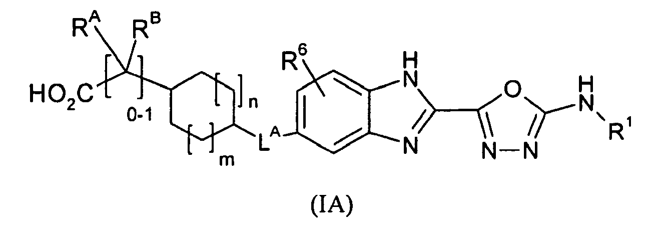 or a salt thereof, wherein:
R¹ is selected from phenyl, optionally substituted with 1 or 2 substituents independently selected from halo, halo(1-6C)alkyl, cyano, (1-6C)alkyl, hydroxy, (1-6C)alkoxy, -SOₘ(1-6C)alkyl and -OSO₂(1-6C)alkyl; or R¹ is optionally substituted with 1, 2, 3, or 4 fluoro;
R^{A} and R^{B} are each independently hydrogen or methyl;
R⁶ is hydrogen, fluoro, chloro or methyl;
L^{A} is a direct bond, -CH₂- or -O-;
m is 0, 1 or 2;
n is 0 or 1;
provided that m + n is 0, 1 or 2.

10. A compound as claimed in Claim 1 which is selected from
trans-2-[4-[2-[5-[(2,4,5-trifluorophenyl)amino]-1,3,4-oxadiazol-2-yl]-1H-benzoimidazol-5-yl]cyclohexyl]acetic acid;
trans-2-[4-[2-[5[(3,4-difluorophenyl)amino]-1,3,4-oxadiazol-2-yl]-1H-benzoimidazol-5-yl]cyclohexyl]acetic acid;
{trans-4-[2-(5-{[3-(benzyloxy)phenyl]amino}-1,3,4-oxadiazol-2-yl)-1H-benzimidazol-5-yl]cyclohexyl}acetic acid;
[trans-4-(2-{5-[(4-cyanophenyl)amino]-1,3,4-oxadiazol-2-yl}-1H-benzimidazol-5-yl)cyclohexyl]acetic acid;
trans-2-[4-[2-[5-[(2-methoxyphenyl)amino]-1,3,4-oxadiazol-2-yl]-1H-benzoimidazol-5-yl]cyclohexyl]acetic acid;
trans-2-[4-[2-[5-(7,10-dioxabicyclo[4.4.0]deca-1,3,5-trien-3-ylamino)-1,3,4-oxadiazol-2-yl]-1H-benzoimidazol-5-yl]cyclohexyl]acetic acid;
trans-2-[4-[2-[5-[(4-chlorophenyl)amino]-1,3,4-oxadiazol-2-yl]- H-benzoimidazol-5-yl]cyclohexyl]acetic acid;
trans-2-[4-[2-[5-[(3-chlorophenyl)amino]-1,3,4-oxadiazol-2-yl]-1H-benzoimidazol-5-yl]cyclohexyl]acetic acid;
trans-2-[4-[2-[5-[(4-methylsulfonylphenyl)amino]-1,3,4-oxadiazol-2-yl]-1H-benzoimidazol-5-yl]cyclohexyl]acetic acid;
trans-2-[4-[2-[5-[(4-fluorophenyl)amino]- 1,3,4-oxadiazol-2-yl]-1H-benzoimidazol-5-yl]cyclohexyl]acetic acid;
trans-2-[4-[2-[S-[(3,4,5-trifluorophenyl)amino]-1,3,4-oxadiazol-2-yl]-1H-benzoimidazol-5-yl]cyclohexyl]acetic acid;
cis-4-[[2-[5-[(2,4,5-trifluorophenyl)amino]-1,3,4-oxadiazol-2-yl]-3H-benzoimidazol-5-yl]oxy]cyclohexane-1-carboxylic acid;
cis-4-[[2-[5-[(4-fluorophenyl)amino]-1,3,4-oxadiazol-2-yl]-3H-benzoimidazol-5-yl]oxy]cyclohexane-1-carboxylic acid;
trans-4-[[2-[5-[(2,4,5-trifluorophenyl)amino]-1,3,4-oxadiazol-2-yl]-3H-benzoimidazol-5-yl]oxy]cyclohexane-1-carboxylic acid;
*cis*-4-{4-Fluoro-2-[5-(4-fluorophenylamino)-1,3,4-oxadiazol-2-yl]-1H-benzimidazol-5-yloxy}cyclohexanecarboxylic acid;
*trans*-4-{4-Fluoro-2-[5-(4-fluorophenylamino)-1,3,4-oxadiazol-2-yl]-1H-benzimidazol-5-yloxy}cyclohexanecarboxylic acid;
*cis*-4-[[4-fluoro-2-[5-[(2,4,5-trifluorophenyl)amino]-1,3,4-oxadiazol-2-yl]-3H-benzoimidazol-5-yl]oxy]cyclohexane-1-carboxylic acid;
*trans*-4-[[4-fluoro-2-[5-[(2,4,5-trifluorophenyl)amino]-1,3,4-oxadiazol-2-yl]-3H-benzoimidazol-5-yl]oxy]cyclohexane-1-carboxylic acid;
*trans*-3-[4-[2-[5-[(4-fluorophenyl)amino]-1,3,4-oxadiazol-2-yl]-1H-benzoimidazol-5-yl]cyclohexyl]oxypropanoic acid;
*trans*-2-[4-[2-[5-[(4-fluorophenyl)amino]-1,3,4-oxadiazol-2-yl]-1H-benzoimidazol-5-yl]cyclohexyl]oxyacetic acid; or a pharmaceutically-acceptable salt of any of these.

11. A compound according to any one of the preceding claims or a pharmaceutically-acceptable salt thereof for use as a medicament.

12. The use of a compound according to any one of claims 1 to 10 or a pharmaceutically-acceptable salt thereof in the manufacture of a medicament for use in the production of an inhibition of DGAT1 activity in a warm-blooded animal such as a human being.

13. The use as claimed in Claim 12 wherein the medicament is for use in the treatment of diabetes mellitus and/or obesity in a warm-blooded animal such as a human being.

14. A pharmaceutical composition which comprises a compound of formula (I) or formula (IA) as claimed in any one of claims 1 to 10 or a pharmaceutically-acceptable salt thereof, in association with a pharmaceutically-acceptable excipient or carrier.

15. A process for preparing a compound according to any one of claims 1 to 10 which comprises one of the following steps (wherein all variables are as hereinbefore defined for a compound of formula (I) unless otherwise stated):
a) reaction of a compound of formula (I) to form another compound of formula (I);
b) cyclisation of a compound of formula (2); 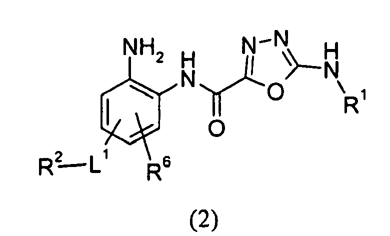
c) when L¹ is -O- or -O-CH₂-, by reaction of a compound of formula (3) with a compound of formula R²-L¹-X¹, wherein X¹ is a suitable leaving group; 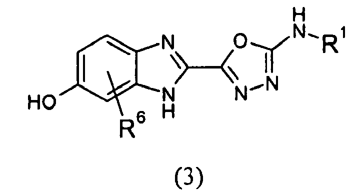
d) by reaction of a compound of formula (4) with a compound of formula R²-L¹-X², wherein X² is for example a boronic acid, stannane or a sulfide, L¹ is a direct bond and X is suitably halo; 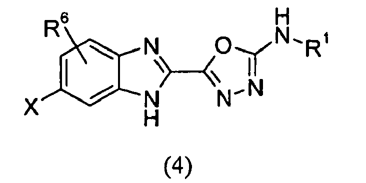
and thereafter if necessary or desirable:
i) removing any protecting groups; and/or
ii) forming a salt thereof.

## Patentansprüche

1. Verbindungen der Formel (I): 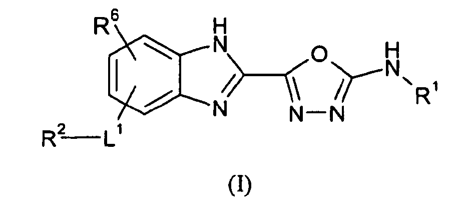 und deren Salze, wobei:
R¹ aus Phenyl, Cyclopentyl, Cyclohexyl und HET-1 ausgewählt ist, wobei R¹ gegebenenfalls entweder mit:
i) einem Substituenten ausgewählt aus Gruppe a) und gegebenenfalls einem Substituenten ausgewählt aus entweder Gruppe b) oder Gruppe c); oder
ii) 1 oder 2 Substituenten unabhängig voneinander ausgewählt aus Gruppe b) und gegebenenfalls einem Substituenten ausgewählt aus Gruppe c); oder
iii) bis zu 4 Substituenten unabhängig voneinander ausgewählt aus Gruppe c) substituiert ist;
wobei die Gruppen a) bis c) wie folgt sind:
Gruppe a) Nitro, -C(O)ₙR²⁰, ein Carbonsäuremimetikum oder ein Bioisoster davon, -NR²¹R²², -C (O) NR²¹R²² , -OC(O) NR²¹R²², -NR²¹C (O) ₙR²⁰, -NR²⁰CONR²¹R²², -S (O) ₂NR²¹R²² oder -NR²¹S (O) ₂R²², wobei R²⁰, R²¹ und R²² unabhängig voneinander aus Wasserstoff und (1-6C)-Alkyl ausgewählt sind oder R²¹ und R²² zusammen mit dem Stickstoffatom, an das sie gebunden sind, einen gegebenenfalls substituierten Ring mit 3 bis 10 Atomen bilden, welcher gegebenenfalls weitere Heteroatome wie S(O)ₘ, Sauerstoff und Stickstoff enthält;
Gruppe b) R¹ ist gegebenenfalls durch 1 oder 2 Substituenten unabhängig voneinander ausgewählt aus Halogen-(1-6C)-alkyl, Cyano, (1-6C)-Alkyl, Hydroxy, (1-6C)-Alkoxy, Benzyloxy, -SOₘ- (1-6C) -Alkyl und -OSO₂- (1-6C)-Alkyl substituiert;
Gruppe c) Halogen;
und, wenn R¹ durch zwei (1-6C) -Alkoxygruppen substituiert ist, diese unter Bildung eines an R¹ kondensierten 5- oder 6gliedrigen Rings miteinander verbunden sein können;
n (unabhängig bei jedem Vorkommen) für 1 oder 2 steht:
m (unabhängig bei jedem Vorkommen) für 0, 1 oder 2 steht;
L¹ für eine direkte Bindung oder einen Linker ausgewählt aus -O-, -OCH₂-, -CH₂O-, -S (O) ₘ-, -S (O) ₘCH₂-, -CH₂S (O) mund - -(CR⁷R⁸)₁₋₂- steht;
R² aus (3-6C) -Cycloalkyl, (5-12C)-Bicycloalkyl, Phenyl, HET-2 und (2-6C)-Alkyl ausgewählt ist; wobei R² gegebenenfalls durch -L²-R³ substituiert ist;
L² für eine direkte Bindung oder einen Linker ausgewählt aus - (CR⁴R⁵) ₁₋₂-, -O- (CR⁴R⁵) ₁₋₂- und -CH₂ (CR⁴R⁵) ₁₋₂- steht (wobei für jeden Wert von L² die CR⁴R⁵-Gruppe direkt an R³ gebunden ist);
R⁴ jeweils unabhängig aus Wasserstoff, Hydroxy, (1-3C)-Alkoxy, (1-4C)-Alkyl, Hydroxy- (1-3C) -alkyl und (1-2C)-Alkoxy(1-2C)-alkyl ausgewählt ist; mit der Maßgabe, dass, wenn L² für -O- (CR⁴R⁵) ₁₋₂- steht, der R⁴-Rest an dem Kohlenstoffatom, das direkt an das Sauerstoffatom gebunden ist, nicht für Hydroxy oder (1-3C)-Alkoxy steht;
R⁵ jeweils unabhängig aus Wasserstoff und Methyl ausgewählt ist;
R³ aus Hydroxy, Carboxyl, (1-6C) -Alkoxycarbonyl und einem Carbonsäuremimetikum oder -bioisoster ausgewählt ist;
R⁶ aus Wasserstoff, Fluor, Chlor, Hydroxy, Methoxy, Halogen- (1-2C) -alkyl, Methyl, Ethyl, Cyano und Methylsulfonyl ausgewählt ist;
R⁷ jeweils unabhängig aus Wasserstoff, (1-4C)-Alkyl und (1-4C)-Alkoxy ausgewählt ist;
R⁸ jeweils unabhängig aus Wasserstoff und Methyl ausgewählt ist;
HET-1 für einen 5- oder 6gliedrigen Heteroarylring mit 1 oder 2 Ringheteroatomen unabhängig voneinander ausgewählt aus O, N und S steht (mit der Maßgabe, dass im Ring keine O-O-, S-S- oder O-S-Bindungen vorhanden sind);
HET-2 für einen 4-, 5- oder 6gliedrigen gesättigten, teilweise ungesättigten oder vollständig ungesättigten Heterocyclylring mit 1, 2 oder 3 Ringheteroatomen unabhängig voneinander ausgewählt aus O, N und S steht (mit der Maßgabe, dass im Ring keine O-O-, S-S- oder O-S-Bindungen vorhanden sind), wobei ein Ringkohlenstoffatom zu C(O) oxidiert sein kann und/oder ein Ringschwefelatom zu S(O) oder S(O)₂ oxidiert sein kann.

2. Verbindung nach Anspruch 1, wobei R¹ für Phenyl steht.

3. Verbindungen nach Anspruch 1 oder 2, wobei R¹ durch 1, 2 oder 3 Fluor substituiert ist.

4. Verbindungen nach einem der vorhergehenden Ansprüche, wobei L¹ für eine direkte Bindung oder -O-steht.

5. Verbindungen nach einem der vorhergehenden Ansprüche, wobei R² für (3-6C)-Cycloalkyl steht.

6. Verbindungen nach einem der vorhergehenden Ansprüche, wobei L² für eine direkte Bindung, -CH₂- oder -O- (CR⁴R⁵) ₁₋₂- steht.

7. Verbindung nach einem der vorhergehenden Ansprüche, wobei R³ für Carboxyl steht.

8. Verbindung nach einem der vorhergehenden Ansprüche, wobei R⁶ für Wasserstoff oder Fluor steht.

9. Verbindungen der Formel (IA) nach Anspruch 1 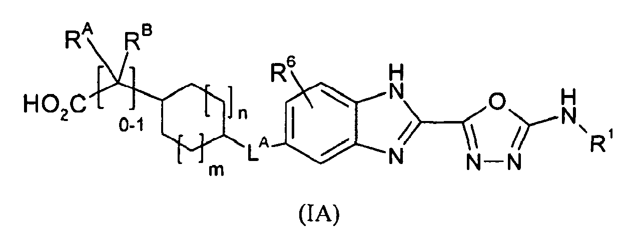 und deren Salze, wobei
R¹ aus Phenyl ausgewählt ist, welches gegebenenfalls durch 1 oder 2 Substituenten unabhängig voneinander ausgewählt aus Halogen, Halogen-(1-6C)-alkyl, Cyano, (1-6C)-Alkyl, Hydroxy, (1-6C)-Alkoxy, -SOₘ- (1-6C) -alkyl und -OSO₂- (1-6C) -alkyl substituiert ist; oder R¹ gegebenenfalls durch 1, 2, 3 oder 4 Fluor substituiert ist;
R^{A} und R^{B} jeweils unabhängig voneinander für Wasserstoff oder Methyl stehen;
R⁶ für Wasserstoff, Fluor, Chlor oder Methyl steht; L^{A} für eine direkte Bindung, -CH₂- oder -O- steht;
m für 0, 1 oder 2 steht;
n für 0 oder 1 steht;
mit der Maßgabe, dass m + n für 0, 1 oder 2 steht.

10. Verbindungen nach Anspruch 1, ausgewählt aus
trans-2-[4-[2-[5-[(2,4,5-Trifluorphenyl)amino]-1,3,4-oxadiazol-2-yl]-1H-benzimidazol-5-yl]cyclohexyl]essigsäure;
trans-2-[4-[2-[5-[(3,4-Difluorphenyl)amino]-1,3,4-oxadiazol-2-yl]-1H-benzimidazol-5-yl]cyclohexyl]essigsäure;
{trans-4-[2-(5-{[3-(Benzyloxy)phenyl]amino}-1,3,4-oxadiazol-2-yl)-1H-benzimidazol-5-yl]cyclohexyl}essigsäure;
[trans-4-(2-{5-[(4-Cyanophenyl)amino]-1,3,4-oxadiazol-2-yl}-1H-benzimidazol-5-yl)cyclohexyl]essigsäure;
trans-2-[4-[2-[5-[(2-Methoxyphenyl)amino]-1,3,4-oxadiazol-2-yl]-1H-benzimidazol-5-yl]cyclohexyl]essigsäure;
trans-2-[4-[2-[5-(7,10-Dioxabicyclo[4.4.0]deca-1,3,5-trien-3-ylamino)-1,3,4-oxadiazol-2-yl]-1H-benzimidazol-5-yl]cyclohexyl]essigsäure;
trans-2-[4-[2-[5-[(4-Chlorphenyl)amino]-1,3,4-oxadiazol-2-yl]-1H-benzimidazol-5-yl]cyclohexyl]essigsäure;
trans-2-[4-[2-[5-[(3-Chlorphenyl)amino]-1,3,4-oxadiazol-2-yl]-1H-benzimidazol-5-yl]cyclohexyl]essigsäure;
trans-2-[4-[2-[5-[(4-Methylsulfonylphenyl)amino]-1,3,4-oxadiazol-2-yl]-1H-benzimidazol-5-yl]cyclohexyl]essigsäure;
trans-2-[4-[2-[5-[(4-Fluorphenyl)amino]-1,3,4-oxadiazol-2-yl]-1H-benzimidazol-5-yl]cyclohexyl]essigsäure;
trans-2-[4-[2-[5-[(3,4,5-Trifluorphenyl)amino]-1,3,4-oxadiazol-2-yl]-1H-benzimidazol-5-yl]cyclohexyl]essigsäure;
cis-4-[[2-[5-[(2,4,5-Trifluorphenyl)amino]-1,3,4-oxadiazol-2-yl]-3H-benzimidazol-5-yl]oxy]cyclohexan-1-carbonsäure;
cis-4-[[2-[5-[(4-Fluorphenyl)amino]-1,3,4-oxadiazol-2-yl]-3H-benzimidazol-5-yl]oxy]cyclohexan-1-carbonsäure;
trans-4-[[2-[5-[(2,4,5-Trifluorphenyl)amino]-1,3,4-oxadiazol-2-yl]-3H-benzimidazol-5-yl]oxy]cyclohexan-1-carbonsäure;
*cis*-4-{4-Fluor-2-[5-(4-fluorphenylamino)-1,3,4-oxadiazol-2-yl]-1H-benzimidazol-5-yloxy}cyclohexancarbonsäure;
*trans*-4-{4-Fluor-2-[5-(4-fluorphenylamino)-1,3,4-oxadiazol-2-yl]-1H-benzimidazol-5-yloxy}cyclohexancarbonsäure;
*cis*-4-[[4-Fluor-2-[5-[(2,4,5-trifluorphenyl)amino]-1,3,4-oxadiazol-2-yl]-3H-benzimidazol-5-yl]oxy]cyclohexan-1-carbonsäure;
*trans*-4[[4-Fluor-2-[5-[(2,4,5-trifluorphenyl)amino]-1,3,4-oxadiazol-2-yl]-3H-benzimidazol-5-yl]oxy]cyclohexan-1-carbonsäure;
*trans*-3-[4-[2-[5-[(4-Fluorphenyl)amino]-1,3,4-oxadiazol-2-yl]-1H-benzimidazol-5-yl]cyclohexyl]oxypropansäure;
*trans*-2-[4-[2-[5-[(4-Fluorphenyl)amino]-1,3,4-oxadiazol-2-yl]-1H-benzimidazol-5-yl]cyclohexyl]oxyessigsäure; und deren pharmazeutisch annehmbare Salze.

11. Verbindung nach einem der vorhergehenden Ansprüche oder ein pharmazeutisch annehmbares Salz davon zur Verwendung als Medikament.

12. Verwendung einer Verbindung nach einem der Ansprüche 1 bis 10 oder eines pharmazeutisch annehmbaren Salzes davon bei der Herstellung eines Medikaments zur Verwendung beim Hervorrufen einer Inhibierung der DGAT1-Aktivität in einem Warmblüter wie einem Menschen.

13. Verwendung nach Anspruch 12, wobei das Medikament für die Behandlung von Diabetes mellitus und/oder Obesitas in einem Warmblüter wie einem Menschen bestimmt ist.

14. Pharmazeutische Zusammensetzung, enthaltend eine Verbindung der Formel (I) oder der Formel (IA) nach einem der Ansprüche 1 bis 10 oder eines pharmazeutisch annehmbaren Salzes davon zusammen mit einem pharmazeutisch annehmbaren Exzipienten oder Träger.

15. Verfahren zur Herstellung einer Verbindung nach einem der Ansprüche 1 bis 10, welches einen der folgenden Schritte umfasst (wobei, wenn nicht anders angegeben, alle Variablen wie oben für eine Verbindung der Formel (I) definiert sind):
a) Umsetzung einer Verbindung der Formel (I) unter Bildung einer anderen Verbindung der Formel (I);
b) Cyclisieren einer Verbindung der Formel (2) 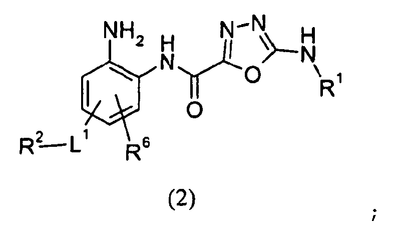
c) wenn L¹ für -O- oder -O-CH2- steht, Umsetzung einer Verbindung der Formel (3) mit einer Verbindung der Formel R²-L¹-X¹, wobei X¹ für eine geeignete Abgangsgruppe steht 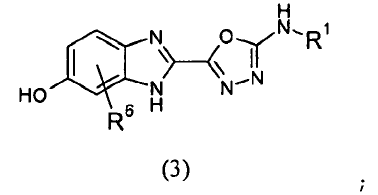
d) Umsetzung einer Verbindung der Formel (4) mit einer Verbindung der Formel R²-L¹-X², wobei X² zum Beispiel für eine Boronsäure, ein Stannan oder ein Sulfid steht, L¹ für eine direkte Bindung steht und X für ein geeignetes Halogen steht 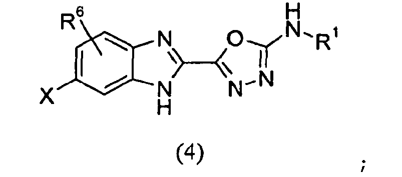
und anschließend, falls erforderlich oder wünschenswert:
i) Entfernen gegebenenfalls vorhandener Schutzgruppen; und/oder
ii) Bildung eines Salzes davon.

## Revendications

1. Composé de formule (I) : 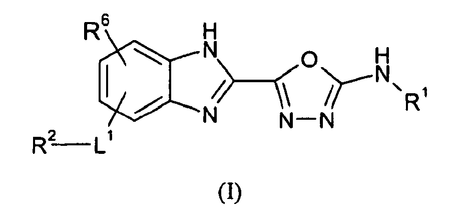 ou de l'un de ses sels, où :
R¹ est choisi parmi les groupements phényle, cyclopentyle, cyclohexyle et HET-1, R¹ étant éventuellement substitué par :
i) un substituant choisi dans le groupe a) et éventuellement un substituant choisi dans le groupe b) ou dans le groupe c) ; ou
ii) 1 ou 2 substituants indépendamment choisis dans le groupe b) et éventuellement un substituant choisi dans le groupe c) ; ou
iii) jusqu'à 4 substituants indépendamment choisis le groupe c) ;
les groupes a) à c) étant les suivants :
groupe a) : groupements nitro, -C (O) ₙR²⁰, substituant imitant un acide carboxylique ou l'un de ses bioisostères, -NR²¹R²², -C(O)NR²¹R²², -OC (O) NR²¹R²², - NR²¹C (O) ₙR²⁰, -NR²⁰CONR²¹R²², -S(O) ₂NR²¹R²² OU -NR²¹S (O) ₂R²², R²⁰, R²¹ et R²² étant indépendamment choisis parmi l'atome d'hydrogène et les groupements alkyle en C1-6, ou R²¹ et R²² forment ensemble et avec l'atome d'azote auquel ils sont liés un cycle éventuellement substitué comportant entre 3 et 10 atomes, et incluant éventuellement des hétéroatomes supplémentaires tels que S(O)ₘ, l'oxygène et l'azote ;
groupe b) : R¹ est éventuellement substitué par 1 ou 2 substituants indépendamment choisis parmi les groupements halogénoalkyle en C1-6, cyano, alkyle en C1-6, hydroxy, alkoxy en C1-6, benzyloxy, -SOₘ-alkyle en C1-6 et -OSO₂-alkyle en C1-6 ;
groupe c) : atomes d'halogène ;
et lorsque R¹ est substitué par deux groupements alkoxy en C1-6, ils peuvent être joints pour former un cycle à 5 ou 6 chaînons fusionné à R¹ ;
n est égal à (indépendamment à chaque apparition) 1 ou à 2 ;
m est égal à (indépendamment à chaque apparition) 0, 1 ou 2 ;
L¹ représente une liaison directe ou un pont choisi parmi -O-, -OCH₂-, -CH₂O-, -S(O)ₘ-, -S (O) ₘ CH₂-, -CH₂S (O) ₘ- et - (CR⁷R⁸) ₁₋₂- ;
R² est choisi parmi les groupements cycloalkyle en C3-6, bicycloalkyle en C5-12, phényle, HET-2 et alkyle en C2-6 ;
R² étant éventuellement substitué par -L²-R³ ;
L² représente une liaison directe ou un pont choisi parmi - (CR⁴R⁵) ₁₋₂-, -O- (CR⁴R⁵) ₁₋₂- et -CH₂ (CR⁴R⁵) ₁₋₂- (où pour chaque valeur de L², le groupement CR⁴R⁵ est directement lié à R³) ;
chacun des radicaux R⁴ est indépendamment choisi parmi l'atome d'hydrogène et les groupements hydroxy, alkoxy en C1-3, alkyle en C1-4, hydroxyalkyle en C1-3 et (alkoxy en C1-2)-alkyle en C1-2 ; à la condition que lorsque L² représente -O- (CR⁴R⁵) ₁₋₂-,
le radical R⁴ de l'atome de carbone directement lié à l'atome d'oxygène ne représente pas un groupement hydroxy ni alkoxy en C1-3 ;
chacun des radicaux R⁵ est indépendamment choisi parmi l'atome d'hydrogène et le groupement méthyle ;
R³ est choisi parmi les groupements hydroxy, carboxy, (alkoxy en C1-6)-carbonyle, et un substituant imitant un acide carboxylique ou l'un de ses bioisostères ;
R⁶ est choisi parmi l'atome d'hydrogène et les groupements fluoro, chloro, hydroxy, méthoxy, halogénoalkyle en C1-2, méthyle, éthyle, cyano et méthylsulfonyle ;
chacun des radicaux R⁷ est indépendamment choisi parmi les atomes d'hydrogène et les groupements alkyle en C1-4 et alkoxy en C1-4 ;
chacun des radicaux R⁸ est indépendamment choisi parmi l'atome d'hydrogène et le groupement méthyle ;
HET-1 représente un cycle hétéroaryle à 5 ou 6 chaînons comportant 1 ou 2 hétéroatomes de cycle indépendamment choisis parmi O, N et S (à la condition qu'il n'y ait aucune liaison O-O, S-S ou O-S dans le cycle) ;
HET-2 représente un cycle hétérocyclyle à 4, 5 ou 6 chaînons, saturé, partiellement insaturé ou entièrement insaturé contenant 1, 2 ou 3 hétéroatomes de cycle indépendamment choisis parmi O, N et S (à la condition qu'il n'y ait aucune liaison O-O, S-S ou O-S dans le cycle), un atome de carbone de cycle pouvant être oxydé en C(O) et/ou un atome de soufre de cycle pouvant être oxydé en S(O) ou S(O)₂.

2. Composé conforme à la Revendication 1, où R¹ représente un groupement phényle.

3. Composé conforme à la Revendication 1 ou à la Revendication 2, où R¹ est substitué par 1, 2 ou 3 atomes de fluore.

4. Composé conforme à l'une quelconque des revendications précédentes, où L¹ représente une liaison directe ou -O-.

5. Composé conforme à l'une quelconque des revendications précédentes, où R² représente un groupement cycloalkyle en C3-6.

6. Composé conforme à l'une quelconque des revendications précédentes, où L² représente une liaison directe, -CH₂- ou -O- (CR⁴R⁵) ₁₋₂-.

7. Composé conforme à l'une quelconque des revendications précédentes, où R³ représente un groupement carboxy.

8. Composé conforme à l'une quelconque des revendications précédentes, où R⁶ représente un atome d'hydrogène ou de fluor.

9. Composé de formule (IA) conforme à la Revendication 1, 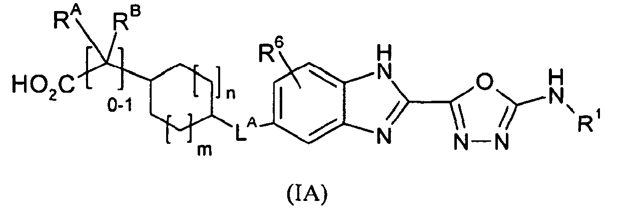 ou de l'un de ses sels, où :
R¹ est choisi parmi les groupements phényle, éventuellement substitué par 1 ou 2 substituants indépendamment choisi parmi les atomes d'halogène et les groupements halogénoalkyle en C1-6, cyano, alkyle en C1-6, hydroxy, alkoxy en C1-6, -SOₘ-alkyle en C1-6 et -OSO₂-alkyle en C1-6 ; ou R¹ est éventuellement substitué par 1, 2, 3, ou 4 atomes de fluor ;
chacun des radicaux R^{A} et R^{B} représente indépendamment un atome d'hydrogène ou un groupement méthyle ;
R⁶ représente un atome d'hydrogène, de fluor ou de chlore ou un groupement méthyle ;
L^{A} représente une liaison directe, -CH₂- ou -O- ;
m est égal à 0, 1 ou 2 ;
n est égal à 0 ou à 1 ;
à la condition que m + n soit égal à 0, 1 ou 2.

10. Composé conforme à la Revendication 1, choisi parmi les suivants :
acide trans-2-[4-[2-[5-[(2,4,5-trifluorophényl)amino]-1,3,4-oxadiazol-2-yl]-1H-benzimidazol-5-yl]cyclohexyl]acétique ;
acide trans-2-[4-[2-[5-[(3,4-difluorophényl)amino]-1,3,4-oxadiazol-2-yl]-1H-benzimidazol-5-yl]cyclohexyl]acétique ;
acide {trans-4-[2-(5-{[3-(benzyloxy)phényl]amino}-1,3,4-oxadiazol-2-yl)-1H-benzimidazol-5-yl]cyclohexyl}acétique ;
acide [trans-4-(2-{5-[(4-cyanophényl)amino]-1,3,4-oxadiazol-2-yl}-1H-benzimidazol-5-yl)cyclohexyl]acétique ;
acide trans-2-[4-[2-[5-[(2-méthoxyphényl)amino]-1,3,4-oxadiazol-2-yl]-1H-benzimidazol-5-yl]cyclohexyl]acétique ;
acide trans-2-[4-[2-[5-(7,10-dioxabicyclo[4.4.0]déca-1,3,5-trién-3-ylamino)-1,3,4-oxadiazol-2-yl]-1H-benzimidazol-5-yl]cyclohexyl]acétique ;
acide trans-2-[4-[2-[5-[(4-chlorophényl)amino]-1,3,4-oxadiazol-2-yl]-1H-benzimidazol-5-yl]cyclohexyl]acétique ;
acide trans-2-[4-[2-[5-[(3-chlorophényl)amino]-1,3,4-oxadiazol-2-yl]-1H-benzimidazol-5-yl]cyclohexyl]acétique ;
acide trans-2-[4-[2-[5-[(4-méthylsulfonylphényl)amino]-1,3,4-oxadiazol-2-yl]-1H-benzimidazol-5-yl]cyclohexyl]acétique ;
acide trans-2-[4-[2-[5-[(4-fluorophényl)amino]-1,3,4-oxadiazol-2-yl]-1H-benzimidazol-5-yl]cyclohexyl]acétique ;
acide trans-2-[4-[2-[5-[(3,4,5-trifluorophényl)amino]-1,3,4-oxadiazol-2-yl]-1H-benzimidazol-5-yl]cyclohexyl]acétique ;
acide cis-4-[[2-[5-[(2,4,5-trifluorophényl)amino]-1,3,4-oxadiazol-2-yl]-3H-benzimidazol-5-yl]oxy]cyclohexane-1-carboxylique ;
acide cis-4-[[2-[5-[(4-fluorophényl)amino]-1,3,4-oxadiazol-2-yl]-3H-benzoimidazol-5-yl]oxy]cyclohexane-1-carboxylique ;
acide trans-4-[[2-[5-[(2,4,5-trifluorophényl)amino]-1,3,4-oxadiazol-2-yl]-3H-benzoimidazol-5-yl]oxy]cyclohexane-1-carboxylique ;
acide *cis*-4-{4-fluoro-2-[5-(4-fluorophénylamino)-1,3,4-oxadiazol-2-yl]-1H-benzimidazol-5-yloxy}cyclohexanecarboxylique ;
acide *trans*-4-{4-fluoro-2-[5-(4-fluorophénylamino)-1,3,4-oxadiazol-2-yl]-1H-benzimidazol-5-yloxy}cyclohexanecarboxylique ;
acide *cis*-4-[[4-fluoro-2-[5-[(2,4,5-trifluorophényl)amino]-1,3,4-oxadiazol-2-yl]-3H-benzimidazol-5-yl]oxy]cyclohexane-1-carboxylique ;
acide *trans*-4[[4-fluoro-2-[5-[(2,4,5-trifluorophényl)amino]-1,3,4-oxadiazol-2-yl]-3H-benzimidazol-5-yl]oxy]cyclohexane-1-carboxylique ;
acide *trans*-3-[4-[2-[5-[(4-fluorophényl)amino]-1,3,4-oxadiazol-2-yl]-1H-benzoimidazol-5-yl]cyclohexyl]oxypropanoïque ;
acide *trans*-2-[4-[2-[5-[(4-fluorophényl)amino]-1,3,4-oxadiazol-2-yl]-1H-benzoimidazol-5-yl]cyclohexyl]oxyacétique ;
ou l'un de leurs sels de qualité pharmaceutique.

11. Composé conforme à l'une quelconque des revendications précédentes, ou l'un de ses sels de qualité pharmaceutique, pour emploi en tant que médicament.

12. Emploi d'un composé conforme à l'une quelconque des revendications 1 à 10 ou de l'un de ses sels de qualité pharmaceutique dans la fabrication d'un médicament pouvant être employé dans l'obtention d'une inhibition de l'activité de DGAT1 chez un animal à sang chaud tel qu'un être humain.

13. Emploi conforme à la Revendication 12, où le médicament est employé dans le traitement du diabète sucré et/ou de l'obésité chez un animal à sang chaud tel qu'un être humain.

14. Composition pharmaceutique qui comprend un composé de formule (I) ou de formule (IA) conforme à l'une quelconque des revendications 1 à 10 ou l'un de ses sels de qualité pharmaceutique, associé à un excipient ou un vecteur de qualité pharmaceutique.

15. Procédé de synthèse d'un composé conforme à l'une quelconque des revendications 1 à 10 qui comprend l'une des étapes suivantes (toutes les variables étant telles que définies ci-avant pour un composé de formule (I), à moins que le contraire ne soit précisé) :
a) réaction d'un composé de formule (I) pour former un autre composé de formule (I) ;
b) cyclisation d'un composé de formule (2) ; 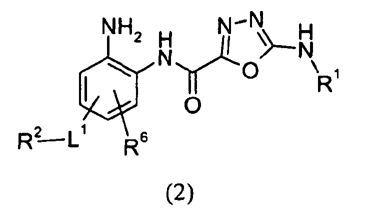
c) lorsque L¹ représente -O- ou -O-CH2-, par réaction d'un composé de formule (3) avec un composé de formule R²-L¹X¹, où X¹ représente un groupement partant adapté ; 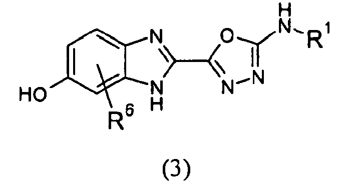
d) par réaction d'un composé de formule (4) avec un composé de formule R²-L¹-X², où X² représente par exemple un acide boronique, un stannane ou un sulfure, L¹ représente une liaison directe et X représente de façon adaptée un atome d'halogène ; 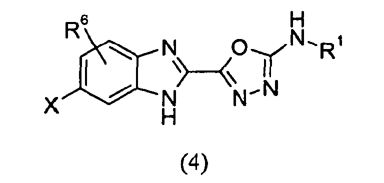
puis, si cela est nécessaire ou recherché : i) clivage des éventuels groupements partants ; et/ou ii) formation d'un sel du composé.
